Europäisches Patentamt

(19) European Patent Office

Office européen des brevets

(11) Publication number : **0 463 848 A2**

# EUROPEAN PATENT APPLICATION

(21) Application number : **91305717.0**

(22) Date of filing : **25.06.91**

(51) Int. Cl.$^5$ : **C12N 15/40**, C12Q 1/70, A61K 39/29, C07K 15/00, G01N 33/569

(30) Priority : 25.06.90 JP 167466/90
31.08.90 JP 230921/90
09.11.90 JP 305605/90
28.12.90 US 635451
08.05.91 JP 132090/91
14.05.91 JP 138493/91

(43) Date of publication of application :
02.01.92 Bulletin 92/01

(84) Designated Contracting States :
AT BE CH DE DK ES FR GB GR IT LI LU NL SE

(71) Applicant : **The Research Foundation for
Microbial Diseases of Osaka University
3-1 Yamadaoka
Suita-shi Osaka (JP)**

(72) Inventor : **Okayama, Hiroto
16-ban 23-310, Onoharahigashi 6-chome
Minoo-shi, Osaka-fu (JP)**
Inventor : **Fuke, Isao
No. 701 Tasankotsu Buldg., 6-2, Torimachi
Takamatsu-shi, Kagawa-ken (JP)**
Inventor : **Mori, Chisato
1152-3, Muromoto-cho
Kanonji-shi, Kagawa-ken (JP)**
Inventor : **Takamizawa, Akihisa
729-2, Yoshioka-cho
Kanonji-shi, Kagawa-ken (JP)**
Inventor : **Yoshida, Iwao
1247-2, Nagareoka-cho
Kanonji-shi, Kagawa-ken (JP)**

(74) Representative : **Blake, John Henry Francis et
al
BROOKES AND MARTIN High Holborn House
52/54 High Holborn
London WC1V 6SE (GB)**

(54) Non-A, non-B hepatitis virus particles.

(57) Disclosed are an isolated non-A, non-B hepatitis virus particle comprising at least one antigen selected from the group consisting of a core antigen, a matrix antigen and an envelope antigen of the non-A, non-B hepatitis virus and a method for efficiently producing the same by genetic engineering. The non-A, non-B hepatitis virus particle can advantageously be used not only for the production of an NANBV hepatitis vaccine exhibiting an extremely high immunogenicity and a diagnostic agent which is extremely high in the antibody detection ratio and in the degree of accuracy of the detection, but is also useful for researches on liver diseases, such as liver cancer.

EP 0 463 848 A2

## Background of the Invention

### Field of the Invention

The present invention relates to non-A, non-B hepatitis virus particles and a method for producing the same. More particularly, the present invention is concerned with non-A, non-B hepatitis virus particles which are obtained by expressing the nucleotide sequence of a region selected from the entire region of non-A, non-B hepatitis virus genome, the entire region of the ORF thereof and a region of the ORF which is obtained by cutting off NS4 and/or NS5 from the ORF, and also concerned with an effective method for producing the same. The non-A, non-B hepatitis virus particles of the present invention are useful for providing a vaccine for non-A, non-B hepatitis, a diagnostic reagent for non-A, non-B hepatitis and an agent for screening blood for transfusion for preventing post-transfusion hepatitis each of which comprises the non-A, non-B hepatitis virus particles as an active ingredient, and for providing a polyclonal or monoclonal antibody which is prepared by using the non-A, non-B hepatitis virus particles. Thus, the non-A, non-B hepatitis virus particles of the present invention are useful for producing a vaccine, an immunoglobulin, an immunological diagnostic reagent, an agent for use in affinity column chromatography for removing non-A, non-B hepatitis virus from blood for transfusion.

### Discussion of Related Art

Definition of non-A, non-B hepatitis virus:

The viral hepatitis is a liver disease caused by the infection of a hepatitis virus. Heretofore, hepatitis A virus, hepatitis B virus and hepatitis D (delta) virus have been isolated and identified. The hepatitis D virus (delta-hepatitis virus) is a deficient virus which cannot multiply by itself and requires for its multiplication the co-presence of hepatitis B virus as a helper virus. Therefore, the hepatitis D virus is present only in a patient having hepatitis B. In 1974, it was reported that there were many patients having hepatitis caused by a factor other than the infection with either hepatitis A virus or hepatitis B virus. Such a hepatitis was named "non-A, non-B hepatitis", and researches on the non-A, non-B hepatitis virus have been made extensively and intensively throughout the world. Heretofore, it has been found that a plurality of types of non-A, non-B hepatitis viruses exist. Results of the researches up to now show that the non-A, non-B hepatitis virus is classified into two types according to the infection route: an epidemic hepatitis virus, namely an enterically-transmitted non-A, non-B hepatitis virus, which is spread through water and food; and a blood transmitted non-A, non-B hepatitis virus which is spread through blood by transfusion, etc. Of the non-A, non-B hepatitis viruses, only an enterically-transmitted non-A, non-B hepatitis virus which spreads over the areas of Africa, India and Southeast Asia has been virologically identified, but the blood-transmitted non-A, non-B hepatitis virus has not yet been identified.

Hereinbelow, the blood-transmitted non-A, non-B hepatitis is often referred to simply as "NANB hepatitis", and the blood-transmitted non-A, non-B hepatitis virus is often referred to simply as "NANBV". Current situation of the studies on NANB hepatitis and problems:

With respect to the epidemiology, clinical examination, diagnosis, treatment and prevention of the NANB hepatitis, virological studies have been made in the world by the comparison of NANBV with the other hepatitis viruses, based on the knowledge of diagnostics, histopathology, immunology, molecular biology and the like ["Japan Medical Journal", No. 3320, pp.3-10, 1987; "Igaku-no Ayumi (Progress of medicine)", 151(13), pp.735-923, 1989; "Kan Tan Sui (Liver, Gallbladder, Pancreas)", 21(1), pp.5-113, 1990; "Jikken Igaku (Experimental Medicine)", 8(3), pp.201-233, 1990]. With respect to the NANB hepatitis, the following findings have been reported.

(1) Epidemiology: In Japan, according to the estimation by the Ministry of Health and Welfare, about 60 % of chronic hepatitis patients (namely about 720 thousand patients), about 40 % of hepatocirrhosis patients (namely about 100 thousand patients) and about 40 % of liver cancer patients (namely about 7 thousand patients) are patients having NANB hepatitis. Further, the mortality attributed to the above-mentioned NANB hepatitis reaches 16 thousand per year. In U.S.A., the number of post-transfusion hepatitis patients reaches 150 to 300 thousand per year and 90 % of the post-transfusion hepatitis patients are patients having NANB hepatitis. Further, it is considered that 1 to 6 % of the blood donors are NANBV carriers. Further, it is estimated that in the other countries also, the incidence of NANB hepatitis and the ratio of the NANBV carrier are equal to or higher than those in U.S.A. and Japan. Therefore, prevention, early diagnosis and early treatment of the NANB hepatitis are of global importance.

(2) Virology: The NANBV heretofore reported comprises an envelope and assumes a viral particle having a spherical shape of about 50 nm in diameter. The taxonomic observations suggest that the known NANBV is a virus similar to a togavirus or a flavivirus, or a virus of new type different from the togavirus or flavivirus. Further, the results of pathological observations of the cytoplasm of hepatocytes of a plurality of chimpanzees injected with serum of a patient having NANBV hepatitis show that the formation of a tubular structure occurs in the cytoplasm of a hepatocyte of some of the chimpanzees, but does not occur in the cytoplasm of a hepatocyte of the other chimpanzees, and that an intranuclear particle is formed in the cytoplasm of a hepatocyte of some of the chimpanzees. These results and the results of the epidemiological observations, tests on the presence or absence of the chloroform sensitivity and immunological diagnosis suggest that a plurality of types of NANBVs exist (see, for example, "Science", 205, 197-200, 1979, "Journal of Infectious Disease", 148, 254-265, 1983, and "Biseibutsu" (Microorganism), 5(5) 463-475, 1989). The amount of the NANBV present in the blood of a patient having NANB hepatitis is extremely small as compared to either the amount of a hepatitis A virus present in the feces of a patient having hepatitis A or the amount of a hepatitis B virus present in the blood of a patient having hepatitis B. For example, the amount of hepatitis B virus in the blood of the patient is $10^8$ to $10^9$ per ml in terms of Chimpanzee Infectious dose (CID), whereas the amount of NANBV in the blood of the patient is only $10^4$ to $10^5$ per ml in terms of CID (Bradley, D.W.: Research perspectives in post-transfusion non-A, non-B hepatitis, in "Infection, Immunity and Blood Transfusion", edited by Dodd, R.Y. & Barker, L.F., published by Alan R. Liss, Inc., New York (1985) pp.81-97). Further, it is known that except for human, there are no animals except chimpanzee that are sensitive to NANBV and that in the cytoplasm of the hepatocyte, a typical tubular structure is occasionally formed by NANBV infection. Since only chimpanzee can be used as an animal for experiment of the NANBV infection, a large number of chimpanzees are required to be used for the study of NANBV. However, the chimpanzee is not easily available and expensive. Therefore, the study of NANBV by, for example, experimental infection by NANBV, identification of NANBV and search for a useful marker for NANBV, is necessarily restricted and delayed. In order to solve these problems, various attempts have been made for the study of NANBV. For example, in an attempt, an NANBV genomic cDNA [referred to as "hepatitis C virus (HCV)"] was cloned from blood plasma of chimpanzees suffering from NANB hepatitis (Science, 244, 359-362, 1989), and it was confirmed that the antigen (referred to as "C-100") obtained by expressing the cDNA exhibited an antigen-antibody reaction with the antibody in the blood of an NANB hepatitis patient (Science, 244, 362-364, 1989). Further, in another attempt, a chimpanzee was not used and an NANBV genomic cDNA was cloned from the blood plasma of NANB hepatitis patients, and it was confirmed that the antigen obtained by expressing the cDNA exhibited an antigen-antibody reaction with the antibody in the serum of an NANB hepatitis patient (Gastroenterologia Japonica, 24, 540-544 and 545-548, 1989). Furthermore, with respect to the cloning of an NANBV genomic cDNA and the determination of the nucleotide sequence thereof and the corresponding amino acid sequence, clones provided by the following institutions are known: Mitsubishi Kasei Corp., Japan (European Patent Application Publication No. 293274), Chiron Corporation, U.S.A. (European Patent Application Publication Nos. 318216, 388232 and 398748), the Research Foundation for Microbial Diseases of Osaka University, Japan (European Patent Application Publication No. 363025 and Journal of Virology, 65, 1105-1113, 1991), Sanwa Kagaku Kenkyusho Co., Ltd., Japan (Japanese Patent Application Laid-Open Specification No. 1-186990), National Cancer Center Research Institute, Japan [Proceedings of the National Academy of Sciences (U.S.A.), 87, 9524-9528, 1990], Jichi Medical School (Japanese Journal of Experimental Medicine, 60, 167-177, 1990), National Institute of Health, Japan [Nucleic Acid Research, 17(24), 10367-10372, 1989; the same literature, 18(15), 4626, 1990; Gene, 91, 287-291, 1990; and Journal of General Virology, 71, 3027-3033, 1990), and the like. Moreover, concerning the structure of NANBV gene, it has been reported: that the total length of NANBV genome is about 10 kb; that the genome is comprised of a non-coding region at the 5'-end, an open reading frame (ORF) region and a non-coding region at the 3'-end; that in the ORF region, genes which code for a virus core antigen (protein) (C antigen), a matrix antigen (protein) (M antigen), an envelope antigen (protein) (E antigen), and six types of non-structural proteins (NS proteins) are disposed in this order from the 5'-end to the 3'-end; and that the NS protein gene is comprised of NS1, NS2, NS3, NS4a, NS4b and NS5 which are disposed in this order from the 5'-end to 3'-end. With respect to the functions of these antigens (proteins) it is believed that C antigen is responsible for the protection of the gene, E antigen is responsible for infection, M antigen is responsible for the maintenance of the structure of E antigen, NS1 serves as a complement fixing antigen, NS3 serves as a protease, NS5 serves as a polymerase and the non-coding region is responsible for the maintenance of the structure of the genome and for the replication of the genome. The functions of NS2 and NS4 have not yet been known.

(3) Clinical observations: Hepatitis is generally classified either into epidemic hepatitis and sporadic hepatitis according to the number and frequency of the occurrences of hepatitis, or into acute hepatitis, fulminant hepatitis, subacute hepatitis, persistent hepatitis and chronic hepatitis according to the severeness and stage of the hepatitis patients. The latent period of the NANB hepatitis is 2 to 26 weeks. The symptom of NANB hepatitis in the early stage is mild as compared to that of hepatitis B. For example, a patient having NANB hepatitis only becomes feverish and complains of languor. Further, 70 % of the patients have anicteric symptom. Therefore, the NANB hepatitis is frequently overlooked. However, the NANB hepatitis is very dangerous because the NANB hepatitis is likely to become chronic and, then, to progress to liver cirrhosis. Illustratively stated, 40 to 50 % of the patients having NANB hepatitis whose serum exhibits an increased aminotransferase activity develop chronic hepatitis. 10 to 20 % of the cases of chronic hepatitis suffer from liver cirrhosis. Further, 0.5 to 1 % of blood recipients per year becomes liver cirrhosis patients without subjective symptoms. More seriously, the liver cirrhosis may further progress to liver cancer or hepatoma. Therefore, for preventing biohazard caused by blood transfusion and bleeding, eradication of the NANB hepatitis is a matter of global importance from the viewpoint of public health.

(4) Diagnosis: As mentioned above, the NANBV (blood-transmitted type) has not yet been identified and a viral marker, such as an NANBV antigen, which is useful for the diagnosis of NANB hepatitis has not been known. Therefore, diagnosis of NANB hepatitis has been conducted by examining the titer of the antibody in serum of a patient, which is specific for each of the known pathogenic viruses, such as hepatitis A virus, hepatitis B virus, cytomegalovirus, EB virus, varicella virus and herpes simplex virus, and diagnosing the patient whose serum is negative with respect to the antibody specific for any of the above-mentioned viruses, as having NANB hepatitis, or by performing a histopathological examination through a biopsy of the liver ("Disease of the Liver and biliary system", 8th edition, S. Shenlock, pp. 326-333, 1989, Blackwell Scientific Publications). At the same time, another diagnosis method has also been used. For example, there have been used a method in which the activity of an enzyme in serum, such as GPT [glutamic-pyruvic transaminase, also known as "ALT" (alanine aminotransaminase)], GOT [glutamic-oxalo-acetic transaminase, also known as "AST" (aspartate aminotransferase)], and guanine deaminase (also known as "guanase") is determined ("Kan Tan Sui (Liver, Gallbladder, Pancreas)", Vol. 14, pp. 519-522, 1987). With respect to the GPT or GOT in serum mentioned above, a standard for the diagnosis of NANB hepatitis in which lasting and abnormally high activities of GPT and GOT are utilized as a criterion for the diagnosis of NANB hepatitis, is employed in Japan ("Journal of Blood Transfusion Society in Japan", 31(4), 316-320, 1985; and "Nippon Rinsho", 46, 2635-2638, 1988). Regarding the immunological diagnosis, in the present situation in which the isolation and identification of NANBV are difficult as described above, an antigen-antibody reaction between an antigen obtained by expression of NANBV cDNA clone (which has been isolated using the techniques of genetic engineering and the knowledge of immunology) and the serum of an NANB hepatitis patient is used as a criterion. Examples of known antigens include an expression product of an NANBV cDNA prepared from the plasma of an NANB hepatitis patient (European Patent Application Publication No. 363025), an expression product of "HCV" cDNA prepared from the plasma of a chimpanzee having the symptoms of NANB hepatitis (European Patent Application Publication No. 318216 and Japanese Patent Application Laid-Open Specification No. 2-500880), an expression product of an NANBV cDNA derived from the liver of an NANBV-infected chimpanzee (European Patent Application Publication No. 293274, Japanese Patent Publication Specification No. 64-2576 and Japanese Patent Application Laid-Open Specification No. 1-124387). As a method for determining the antigen-antibody reaction, RIA (radioimmunoassay) and EIA (enzyme immunoassay) are generally used. However, these expression products are different in antigenicity. The antigen which is an expression product of HCV cDNA (that is, the C-100 antigen mentioned) can be some criterion or yardstick for the diagnosis of chronic hepatitis caused by the HCV infection. However, since the region in which the antigen (C-100) exhibits its antigenicity is limited and the detection ratio of the antibody is as disadvantageously low as about 70 % ["Biseibutsu (Microorganism)", 5, 463-475, 1989; "Kan Tan Sui (Liver, Gallbladder, Pancreas)", 20, 47-51, 1990; and "Igaku-no Ayumi (Progress of Medicine)", 151, 871, 1989], this antigen is unsatisfactory from the viewpoint of accurate diagnosis of NANB hepatitis and NANBV infection and from the viewpoint of accurate determination of the progress of a patient suffering from chronic hepatitis and acute hepatitis for treatment thereof. Therefore, it has been desired to obtain a reliable method for the diagnosis and prognosis of the NANB hepatitis.

(5) Therapy and Prevention: Recently, the usefulness of α- and β-interferons in the treatment of chronic NANB hepatitis have been reported ("Kan Tan Sui (Liver, Gallbladder, Panceras)" vol. 20, pp. 59-64, 1990; "Igaku-no Ayumi (Progress of Medicine)", vol. 151, pp. 871-876, 1989). However, a suitable dose of α- and β-interferons and a suitable period for administration thereof have not yet been established.

On the other hand, for prevention of NANB hepatitis, various vaccines are used in which the above-mentioned conventional expression products of NANBV cDNAs (European Patent Application Publication No. 363025) or HCV cDNAs (European Patent Application Publication No. 318216) are used as an antigen. However, as is apparent from the fact that the NANBV itself has not yet been isolated and identified before completion of the present invention, it has been impossible to specify an antigen useful for NANBV vaccines from the above-mentioned expression products each having a variety of antigenic determinants (epitopes) and determine the effectiveness and safety of such a specific antigen so that the antigen can be clinically used. Accordingly, there is no NANBV vaccine which can be advantageously put into practical use.

(6) Production of NANBV particles and significance thereof: although various NANBV cDNA clones have been known as described in item (2) above, no report has been made such that an NANBV particle has been successfully produced by using the known clones. This fact means that it is extremely difficult to construct a cDNA of about 10 kb which covers the entire region of NANBV genome, the cDNA being necessary for the production of an NANBV particle. That is, by using the prior art technique for the selection of materials to be used for the extraction of NANBV genomic RNA and the prior art technique for the extraction and the purification of the RNA, it is only possible to isolate a short RNA fragment of at most a few hundreds nucleotides and a cDNA clone thereof. When it is intended to construct a cDNA of the entire region of NANBV genome by using such a short-length cDNA fragment, it is necessary to select more than several tens of different types of cDNA fragments in a combination such that the ORFs of the cDNA fragments can form the entire region of NANBV genome, and ligate them in sequence accurately without any mistake. Needless to say, the operation for ligating cDNA fragments in sequence while satisfying such strict requirements is extremely cumbersome and difficult. It should be noted that the probability of the occurrence of a fatal mistake in the ligating operation for cDNA fragments is increased in proportion to the increase in the number of ligations. Therefore, in order to attain the accurate construction of the cDNA of the entire region of NANBV genome, it is necessary to reduce the number of ligation steps by using cDNA fragments which are as long as possible. It should further be noted that the realization of the reduction of the number of the ligations needs a high level of academic knowledge and experience and extraordinary skills with respect to the preparation by extraction of a long-length NANBV genomic RNA fragment of about 2 kb to about 5 kb and with respect to the cloning of a cDNA thereof. On the other hand, as described in item (4), since the antigen used in the commercially available diagnostic reagents for NANB hepatitis is an expression product of a part of NANBV genomic cDNA fragment and, therefore, is narrow in the antigen spectrum, the antigen reacts mainly with the serum of a chronic hepatitis patient and exhibits an antibody detection ratio as low as about unsatisfactory 70 %. Therefore, a diagnostic reagent is in a great demand which exhibits excellent specificity in the antigen-antibody reaction with the serum of not only a chronic NANB hepatitis patient but also an acute NANBV hepatitis patient and is high in the antibody detection ratio. To meet the demand, it has been earnestly desired to develop a diagnostic reagent employing, as an antigen, for example, an NANBV particles having a broad antigen spectrum and which exhibits a high detection ratio for antibody. Further, the production of NANBV particles is considered to contribute to solving the problem of item (5) so that a practically employable NANB hepatitis vaccine can be realized. From the foregoing it is apparent that the construction of a cDNA of the entire region of NANBV genome and the attainment of the mass production of NANBV particles by expressing the cDNA have been earnestly desired as a matter of global interest.

## Summary Of The Invention

The present inventors have made extensive and intensive studies with a view toward solving the above-mentioned problems of the prior art by developing novel isolated NANBV particles. As a result, the present inventors have succeeded in constructing an ORF (open reading frame) region from the C antigen gene through the NS3 gene of the NANBV genomic cDNA, an entire ORF region of the NANBV genomic cDNA which is longer than the above-mentioned ORF region, and the entire region of NANBV genome comprised of the above entire ORF region and, ligated at its 5'-end and 3'-end, non-coding regions for 5'-end and 3'-end, by skillfully ligating not more than ten different NANBV cDNA clones each comprising at least 1000 nucleotides so that a desired ORF having or not having non-coding regions at 3'- and 5'ends is constructed. Moreover, the present inventors have surprisingly succeeded in the mass production of NANBV particles by introducing or inserting each of the above-mentioned regions of the NANBV genome individually into an expression vector and expressing the regions. In the present invention, the terminology "non-A, non-B hepatitis virus particle" means an expression product of the above-mentioned regions of the NANBV genome and comprises at least one antigen selected from the group consisting of a core antigen, a matrix antigen and an envelope antigen of the non-A, non-B hepatitis virus. Examples of non-A, non-B hepatitis virus (NANBV) particles include those of the following structures: a complete NANBV particle which is an NANBV antigen assembly comprised mainly of C (core) antigen, M (matrix) antigen and E (envelope) antigen and which has a nucleic acid in the virus particle; an incomplete NANBV particle which is an NANBV antigen assembly comprised mainly of C antigen, M antigen and E antigen but which has no nucleic acid in the virus particle; an NANBV core which is an NANBV antigen assembly comprised mainly of C antigen and which has a nucleic acid in the core; an incomplete NANBV core which is an NANBV antigen assembly comprised mainly of C antigen but which has no nucleic acid in the core; and an NANBV surface antigen assembly comprised mainly of E antigen. This success is attributed to a unique technique of the present inventors such that in order to obtain an authentic NANBV genome, NANBV RNAs are extracted directly from NANBV particles contained in whole blood of a patient having NANB hepatitis or a resected liver of a patient having NANB hepatitis and liver cancer in combination, without multiplying the NANBV in a chimpanzee having unknown factors which are considered to have rendered difficult the isolation of NANBV, although the amount of NANBV in the blood or resected liver is extremely small, that is as small as about 1/10,000 that of a hepatitis A virus or a hepatitis B virus, but with paying minute care in the operating procedure so that the NANBV and its genome do not undergo cleavage and/or decomposition by the action of body fluids or blood enzymes during the storage of fresh materials for NANBV genome and that a complete NANBV genomic RNA or RNA fragments having a length of about 2 kb to 5 kb are obtained. RNAs thus prepared from fresh human materials are then converted to cDNA by means of a reverse transcriptase to obtain a cDNA library. In order to screen a NANB genomic cDNA of about 1000 to about 5000 nucleotides from the cDNA library, the cDNAs are individually inserted in lambda gt11 phage vectors and then expressed on the phage plaques at high concentration, followed by screening of NANBV genomic cDNAs by repeatedly conducting enzyme immunoassay (EIA) in which both serum from a convalescent patient having acute NANB hepatitis and serum from a patient having chronic NANB hepatitis are used. Thus, safe production of NANBV particles or NANBV antigen assemblies with high purity on a large scale at low cost without biohazard, has for the first time been realized by expressing by recombinant DNA techniques the entire region of NANBV genomic cDNA or the entire ORF region of NANBV genomic cDNA constructed by selecting cDNA clones covering the entire region or the entire ORF region of NANBV genomic cDNA, cutting-off any overlapping portion from the cDNA clones and ligating the cDNA clones in sequence such that the entire region or the entire ORF region of NANBV genomic cDNA is formed. Furthermore, it has been found that the expression product of the present invention has an extremely broad antigen spectrum as compared with a conventional expression product of NANBV genomic cDNA fragments of short length, and exhibits antigen-antibody reaction specifically with the serum of both a chronic patient and an acute patient of NANB hepatitis so that the detection ratio for the antibody is 95 % or more, solving the problem of item (6) above. That is, the expression product of the present invention has been found to make a great contribution to the prevention, diagnosis and treatment of NANB hepatitis by providing a vaccine having an enhanced immunogenicity, a diagnostic reagent exhibiting an improved detection ratio for antibody and an improvement in the preparation of antibodies. Based on the above, the present invention has been completed.

The foregoing features and advantages of the present invention will be apparent from the following detailed description and appended claims taken in connection with the accompanying drawings.

Brief Description Of The Drawings

In the Drawings:
Fig. 1(1) and Fig. 1(2) are diagrams showing the relationships between the cDNA clones of the NANBV gene to be used in the present invention, shown relative to the entire region of the NANBV genome;
Fig. 2(1) through Fig. 2(16) show the nucleotide sequence of the entire region of the NANBV genomic cDNA to be used in the present invention and the amino acid sequence coded for by the nucleotide sequence;
Fig. 3 is a diagram showing the hydrophobicity profiles of both of the NANBV of the present invention and the Japanese encephalitis virus (JEV), in which the hydrophobicity index of the NANBV is compared with that of the JEV, and wherein the abscissa indicates the amino acid number, the ordinate indicates the hydrophobicity index, the vacant triangle indicates the glycosylation site, the asterisk indicates the site of amino acid sequence (Gly-Asp-Asp) common to RNA polymerase, and C, M, E and NS represent core antigen, matrix antigen, envelope antigen and non-structural protein, respectively.

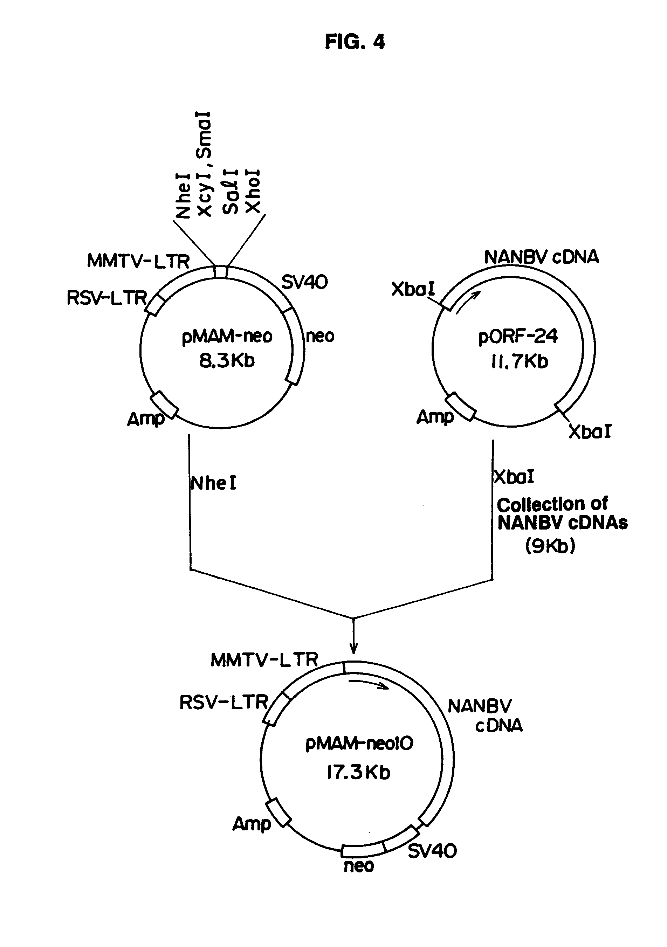

Fig. 4 is a diagram showing the steps for the construction of plasmid pMAM-neo10 for expressing the NANBV genomic cDNA in an animal cell.
Fig. 5 is a diagram showing the steps for the construction of plasmid pYHC5 for expressing the NANBV genomic cDNA in yeast.

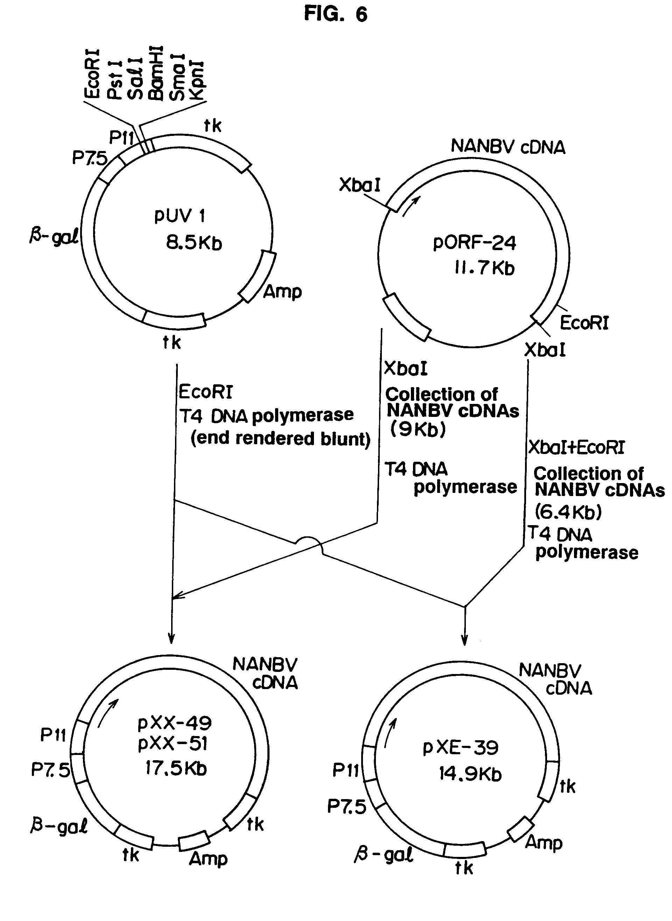

Fig. 6 is a diagram showing the steps for the construction of plasmid pXX-49, pXX-51 and pXE-39 for the preparation of a recombinant vaccinia virus.
Fig. 7 is a graph showing the sucrose concentration and the antigenic activity of each of the fractions obtained by sucrose density-gradient centrifugation of the supernatant of the culture of recombinant vaccinia virus vXX39.
Fig. 8 is an electron microscopic photomicrograph of NANBV particles produced by culturing cells infected with recombinant vaccinia virus vXX39.

Detailed Description Of The Invention

Essentially, according to the present invention, there is provided an isolated non-A, non-B hepatitis virus particle comprising at least one antigen selected from the group consisting of a core antigen, a matrix antigen and an envelope antigen of the non-A, non-B hepatitis virus.

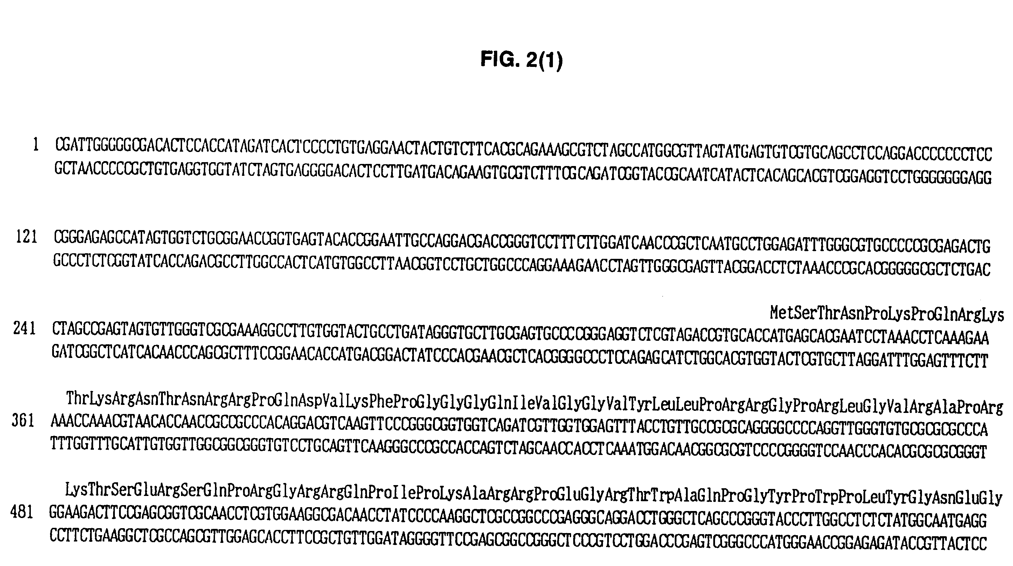

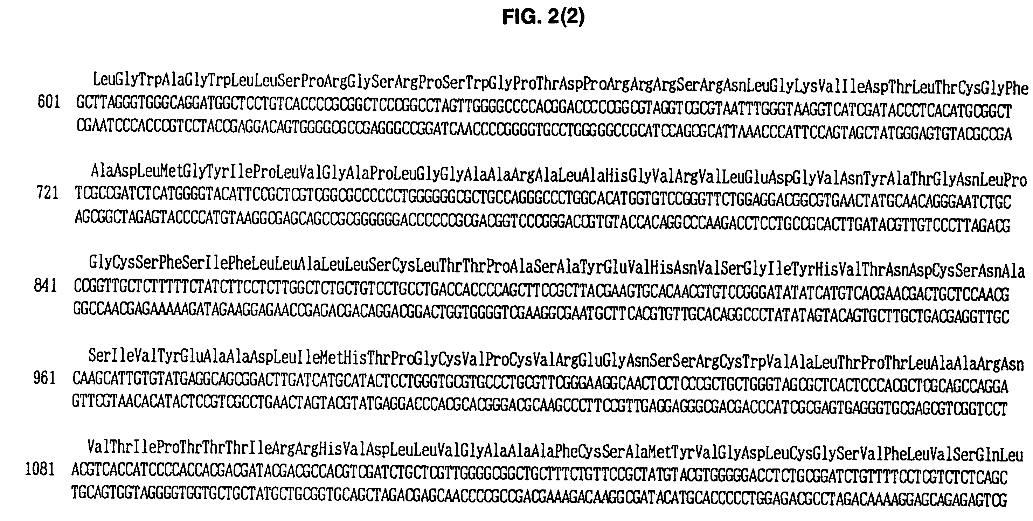

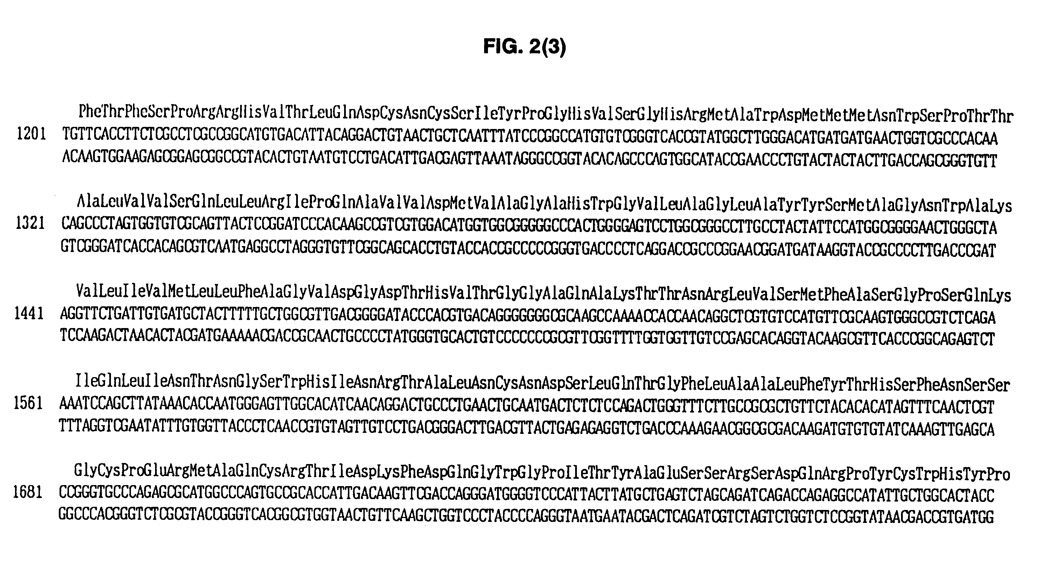

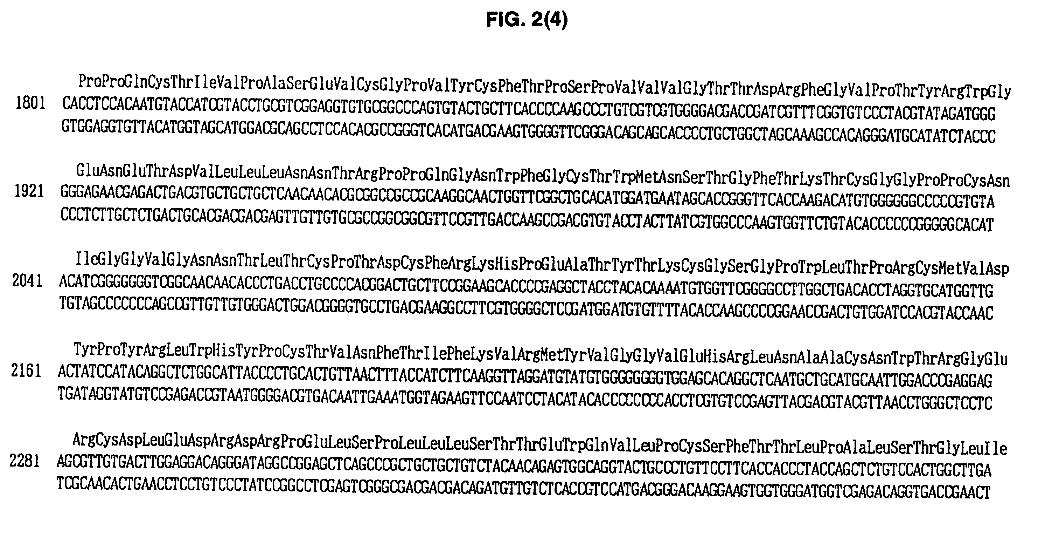

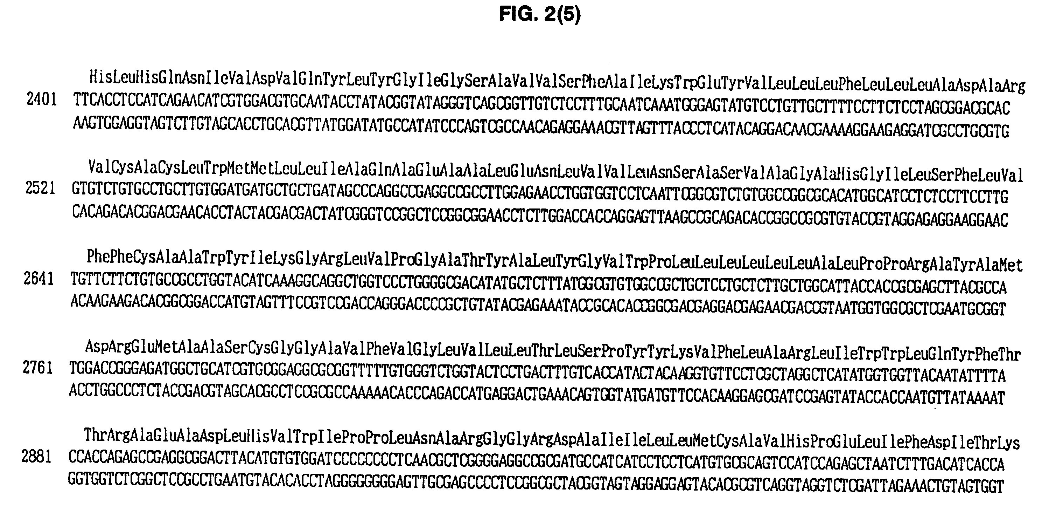

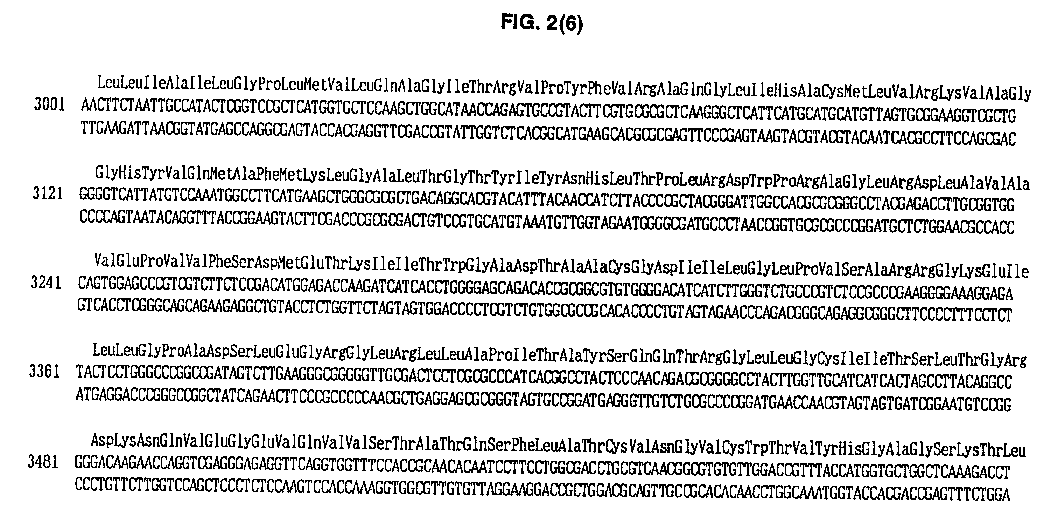

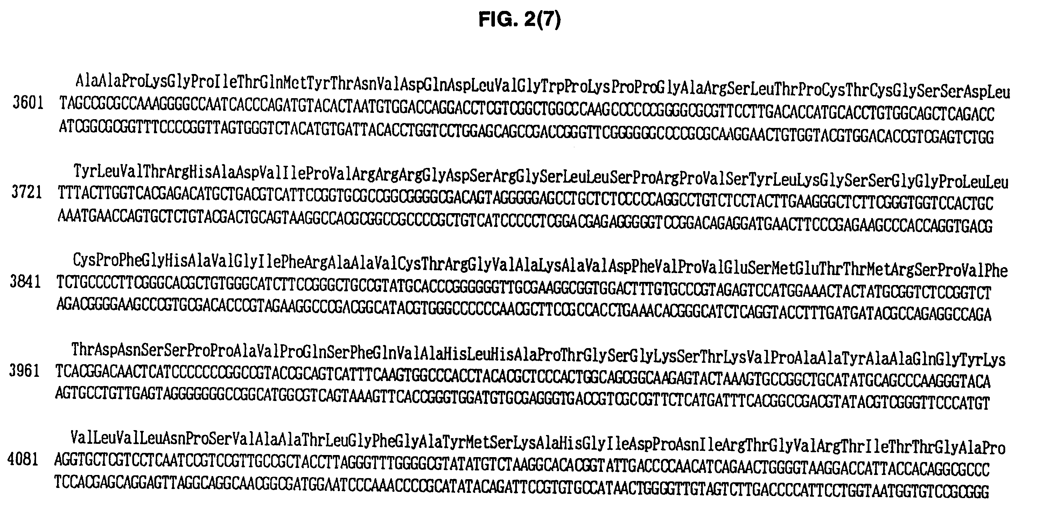

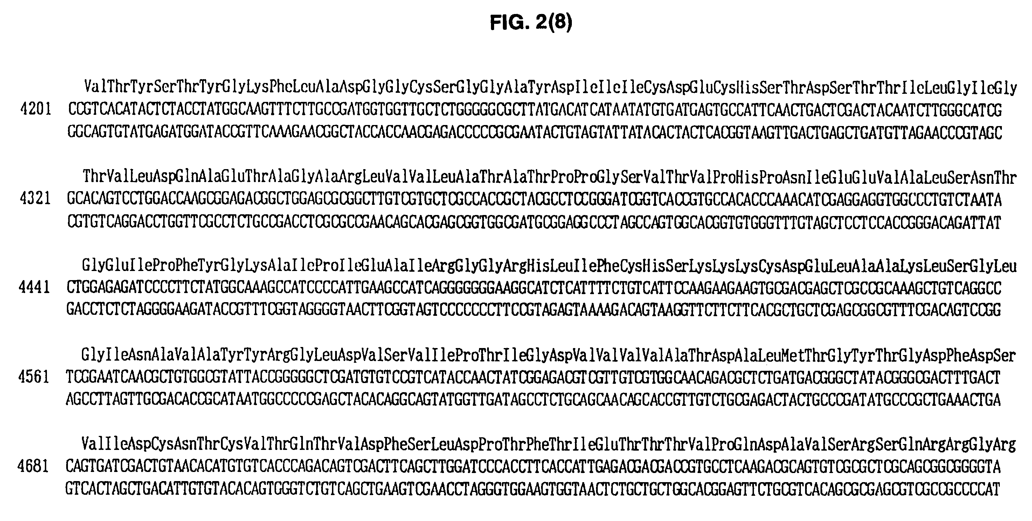

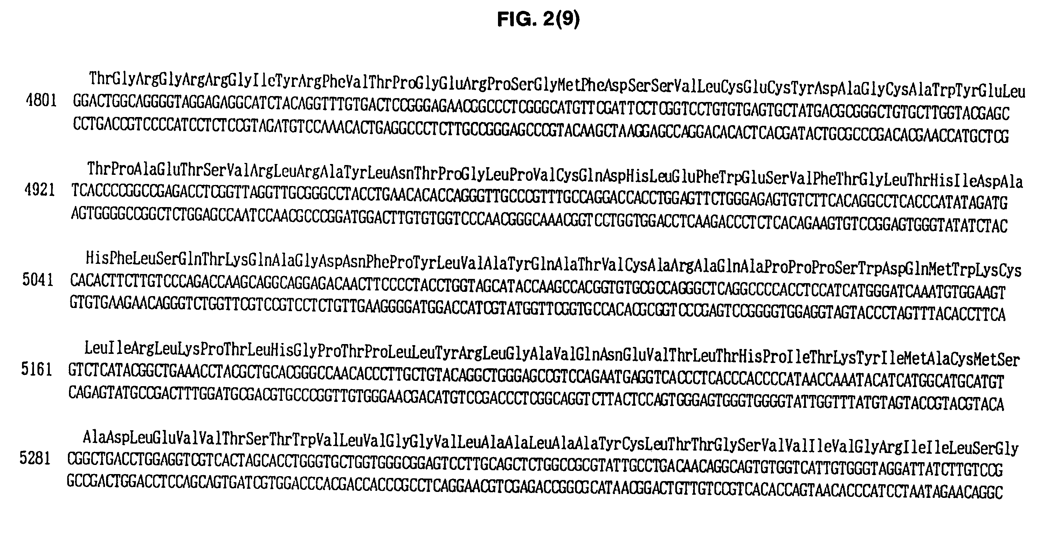

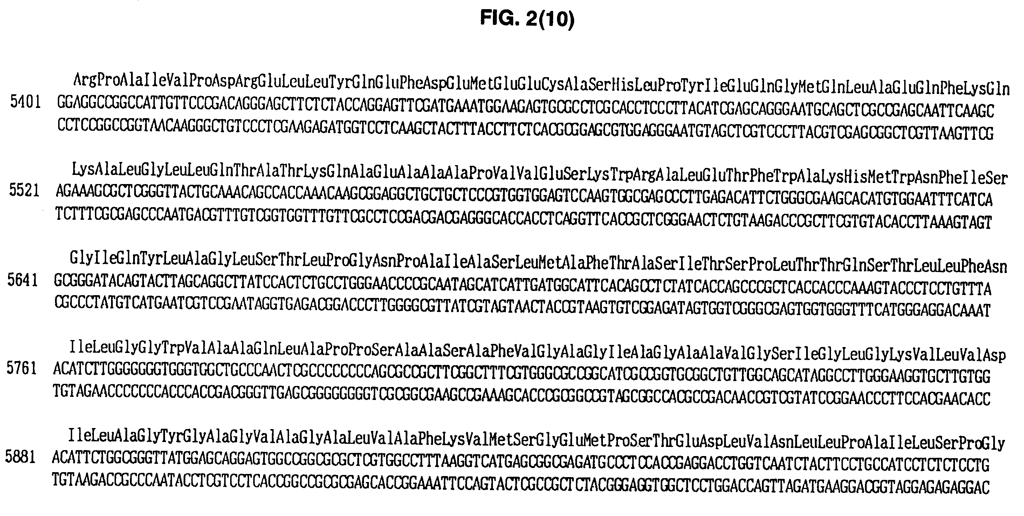

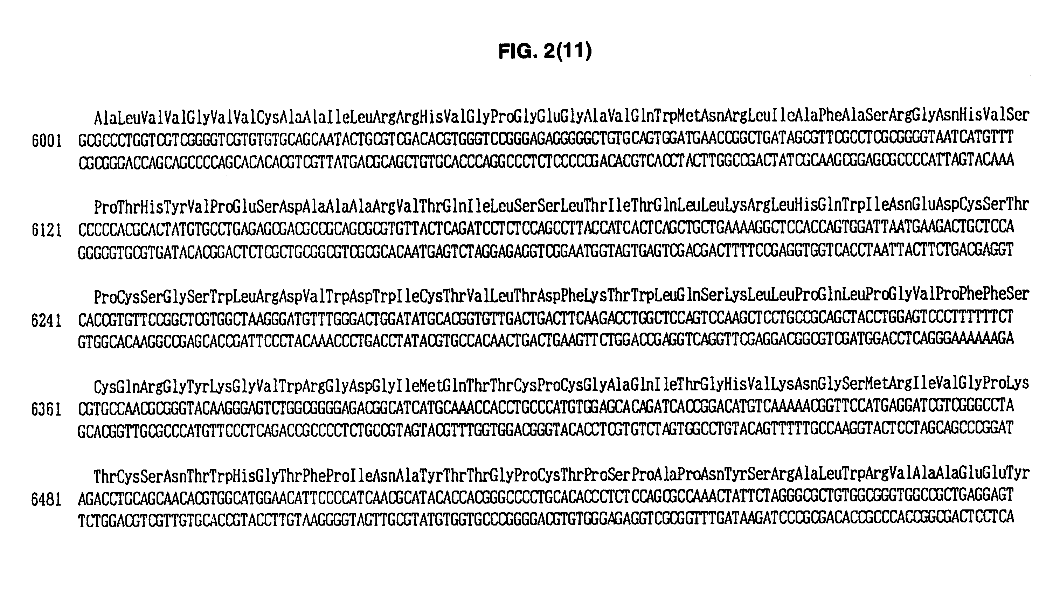

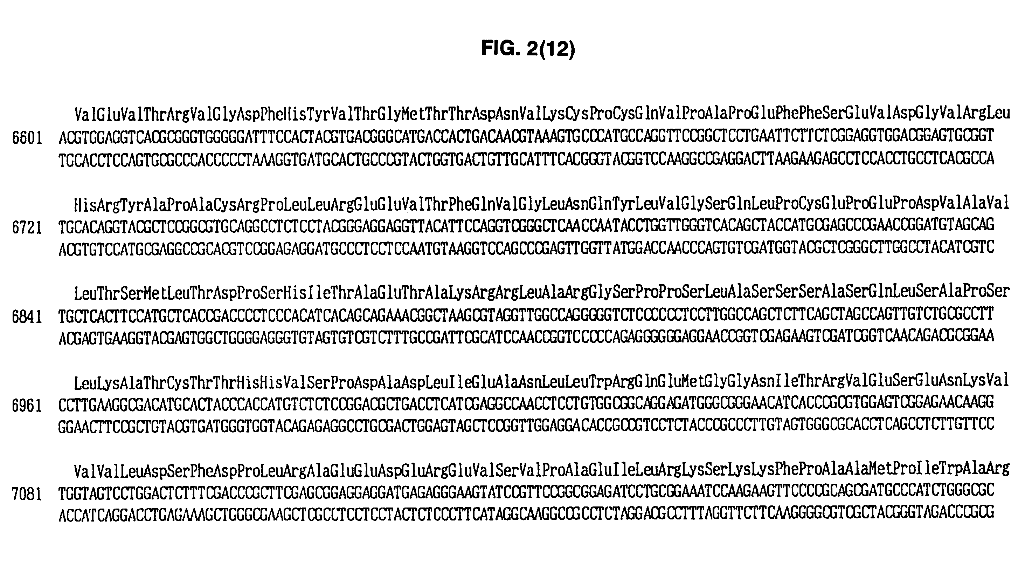

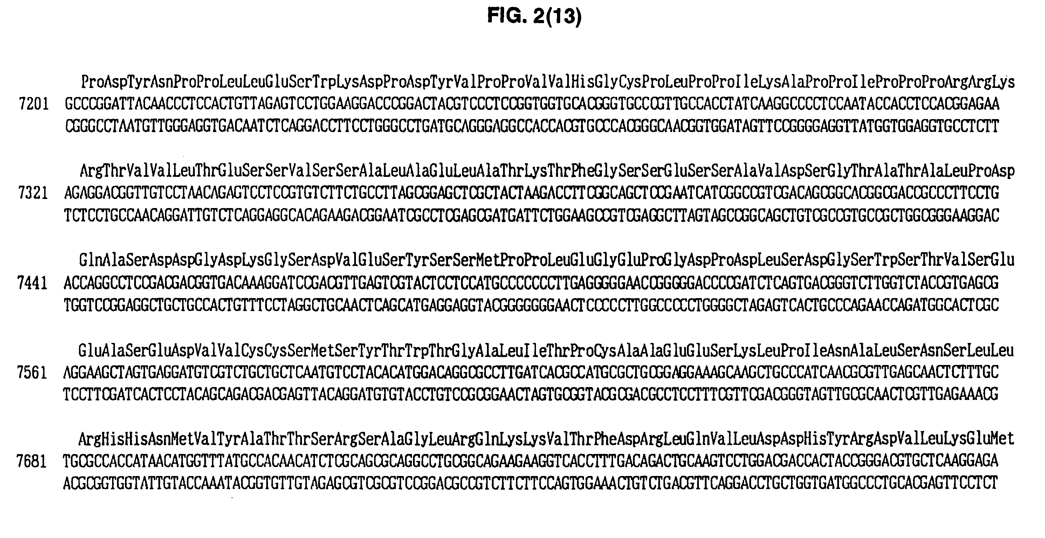

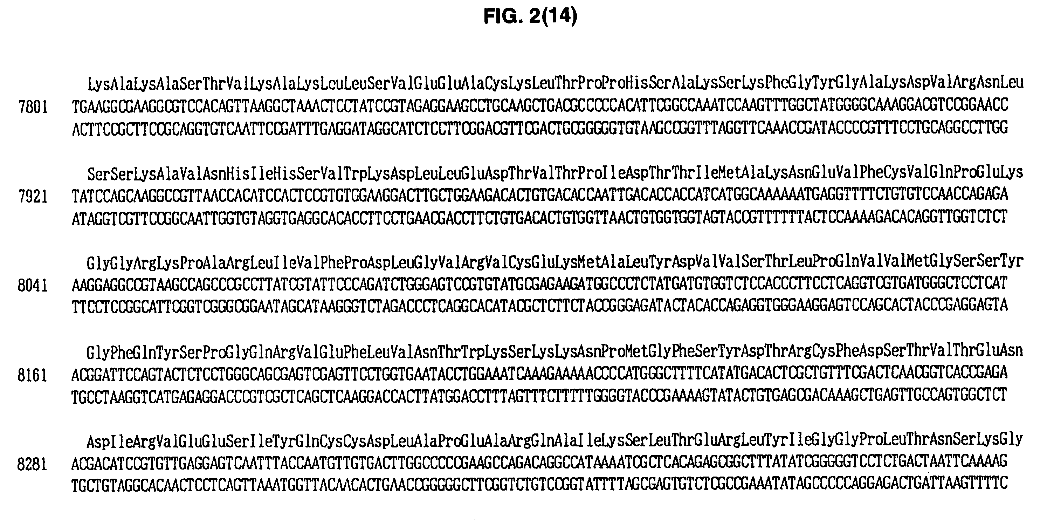

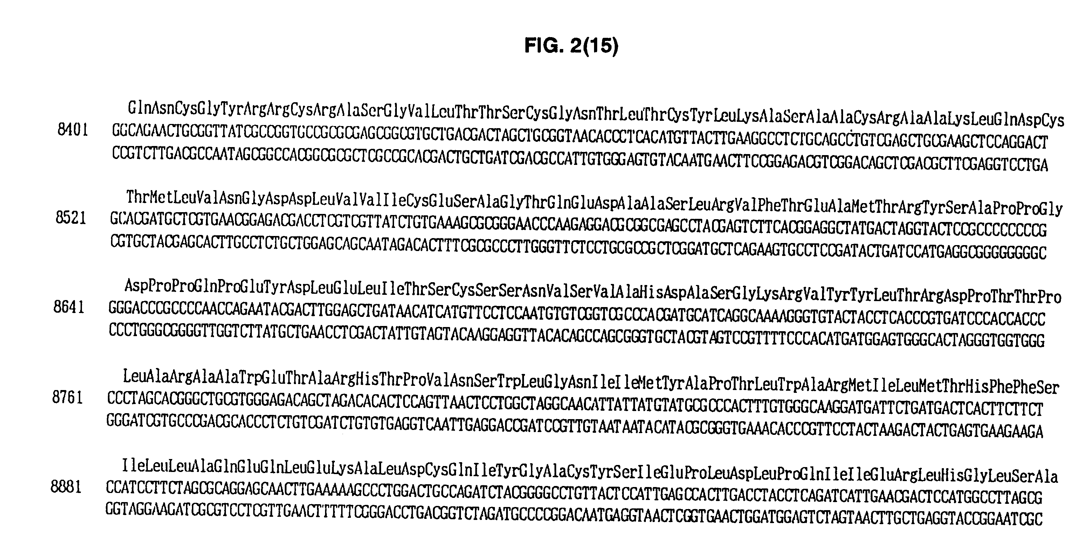

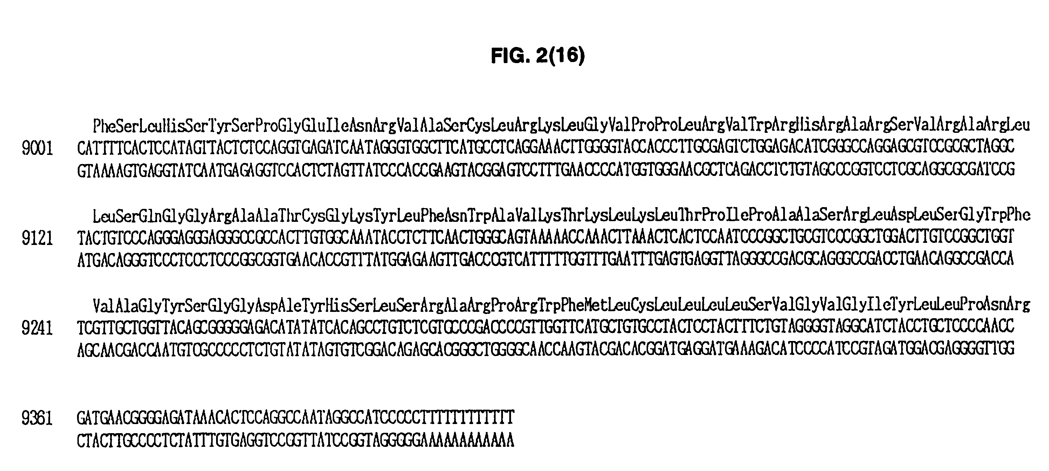

In a preferred embodiment of NANBV particle of the present invention, the core antigen, the matrix antigen and the envelope antigen are, respectively, coded for by a nucleotide sequence of the 333rd to 677th nucleotides, a nucleotide sequence of the 678th to 905th nucleotides and a nucleotide sequence of the 906th to 1499th nucleotides of the non-A, non-B hepatitis virus entire nucleotide sequence from the 1st to 9416th nucleotides shown in Fig. 2(1) through Fig. 2(16) hereof.

In another aspect of the present invention, there is provided a non-A, non-B hepatitis virus particle" which has a ribonucleic acid corresponding to at least part of the nucleotide sequence shown in Fig. 2(1) through Fig. 2(16) hereof.

In the present invention, unless otherwise specified, the left end and right end of-the sequence of deoxyribonucleotides are the 5'-end and 3'-end, respectively. Further, unless otherwise specified, the left end and right end of the amino acid sequences of peptides are the N-terminus and C-terminus, respectively.

The isolated NANBV particle of the present invention can be prepared and identified in accordance with the following steps (I) to (IX).

Step (I): Selection and collection of a material for extracting an NANBV genomic RNA.

As a material for extracting the NANBV RNA, there may be used, for example, blood, lymph, ascites and hepatocyte of an NANBV carrier, or of a human or a chimpanzee suffering from NANB hepatitis, and hepatocyte of a patient suffering from NANB hepatitis and liver cancer or hepatoma in combination. Since the materials derived from a chimpanzee may contain NANBV in a relatively small amount as compared to the materials derived from a human and a chimpanzee has unknown factors which are considered to have rendered difficult the isolation of NANBV, the use of the materials derived from a human is preferred. Of blood, lymph, ascites and hepatocytes from a human, blood can most easily be obtained in a large amount. For example, blood which is not acceptable for use as blood for transfusion is available in a large,amount from, e.g., a blood bank. Such blood can advantageously be used as a material for extracting an NANBV RNA. When blood is used as a material, blood is separated into plasma and erythrocytes. The thus obtained plasma is examined to determine whether or not the plasma is negative to the surface antigen of hepatitis B virus (WHO expert committee on viral hepatitis: Advances in viral hepatitis, WHO Technical Report Series, 602, 28-33, 1977) and negative to a genomic DNA of hepatitis B virus (Brechot, C., Hadchouel, M., Scotto, J., Degos, F., Charnay, P., Trepo, C., Tiollais, P.: Detection of hepatitis B virus DNA in liver and serum: a direct appraisal of the chronic carrier state. Lancet 2: 765-768, 1981). Further, the plasma is examined with respect to the activities of enzymes, such as GPT (Wroblewski, F. & LaDue, J. S.: Serum glutamic-pyruvic transaminase in cardiac and hepatic disease, Proc. Soc. Exp. Biol. Med., 91, 569, 1956), GOT, guanase and the like, which are employed as the criterion for the diagnosis of NANB hepatitis. The above-mentioned procedures of the separation of blood into plasma and erythrocytes and the examination of the plasma are conducted with respect to blood of different lots. The plasma which is negative to both surface antigen and genomic cDNA of hepatitis B virus and exhibits extremely high activities of the above-mentioned enzymes, for example, a GPT activity of 35 IU/ml or more, is pooled.

The number of the NANB hepatitis virus particles in blood is extremely small as compared to that of the hepatitis B virus particles as mentioned hereinbefore. From the results of the infection experiment, the number of the NANB hepatitis virus particles in blood is estimated to be about 1/10,000 of the number of the hepatitis B virus particles (Bradley, D.W., (1985): Research perspectives in post-transfusion non-A, non-B hepatitis, in "Infection, Immunity and Blood Transfusion", edited by Dodd, R.Y. & Barker, L.F., published by Alan R. Liss, Inc., New York, pp. 81-97). Therefore, for the extraction of the RNA, it is preferred to use blood in a large amount, for example, in an amount as large as about 3 to 10 liters. Fresh whole blood to be used as a material for extracting an NANB RNA from NANBV particles is stored at 1 to 5 °C in order to prevent NANBV and its gene from being denatured and to prevent its gene from being cleaved or decomposed by the action of an enzyme. It is also desirable to complete the preparation of NANBV RNAs by Step (II) within 48 to 72 hours from the collection of the fresh whole blood. When a hepatocyte is used as a material, about 1 to 3 g of a non-cancerous or a cancerous portion of a liver tissue resected from a patient having hepatoma or liver cancer which is a complication of a chronic NANB hepatitis may advantageously be used. Hepatocyte to be used as a material is stored in a frozen state at -70 °C.

Step (II): Preparation of the NANBV RNA

From the material obtained in Step (I), the RNA may be extracted and purified by conventional methods. For example, when fresh whole blood is used as the material, about 3 to 10 liters of fresh whole blood is subjected to low-speed centrifugation to collect a plasma fraction as a supernatant. The virus fraction is obtained from the plasma through purification for use in the subsequent procedure for the extraction and purification of the RNA.

On the other hand, when hepatocyte is used as a material for extracting the NANBV RNA, about 5 to 30-fold volume of a diluent containing ribonuclease inhibitor is added to the liver tissue. Then, according to the conventional method using a homogenizer and the like, the liver tissue is crushed or disrupted to obtain a homogenate of hepatocyte. As a diluent, 10 to 150 mM of a conventional buffer may be used. Then, the homogenate is subjected to low-speed centrifugation to collect a supernatant. The collected supernatant is used as an original solution for the extraction and purification of the NANBV RNA. The extraction and purification of the NANBV RNA may be conducted by the conventional method, for example, an extraction method in which a mixture of a ribonuclease inhibitor, such as heparin, diethyl pyrocarbonate, and guanidine thiocyanate, with a surfactant, a chelating agent, or a reducing agent capable of enhancing the denaturation of a protein, is used; a method in which fractionation is conducted by density gradient centrifugation using sucrose, cesium chloride, cesium trichloroacetate, Ficoll (Pharmacia Fine Chemicals AB, Sweden) or the like as a solute of a gradient; a method in which separation is conducted by affinity column utilizing the 3′-terminalpoly A chain which an mRNA specifically has; a separation method in which an mRNA-bonded polysome is obtained by the immunoprecipitation using an antibody specific for a protein synthesized on the polysome; a phenol extraction method based on a principle of two-phase separation; a precipitation method by the use of a polyethylene glycol, a dextran sulfate, an alcohol or the like. The above-mentioned methods may be used individually or in combination. The abovementioned procedure for extracting and purifying the NANBV RNA may preferably be conducted at pH 3 to 10 in order to prevent the irreversible denaturation of the RNA. Thus, NANBV RNAs are obtained.

Step (III): Preparation of a double-stranded cDNA from the NANBV RNA

Using as a template each of the NANBV RNAs abtained in Step (II), a cDNA may be prepared by a standard method. That is, using an oligodeoxythymidine and a random hexanucleotide primer as primers and using a reverse transcriptase, a cDNA complementary to the NANBV RNA is synthesized using the NANBV RNA as a template to obtain a double-strand comprising the cDNA and the NANBV RNA which are complementarily bonded to each other. Then, the thus obtained double-strand is reacted with ribonuclease H so that the NANBV RNA is decomposed and removed from the cDNA. Thus, a single-stranded cDNA is obtained. Using the obtained single-stranded cDNA as a template, a double-stranded cDNA is synthesized by means of a DNA polymerase. The double-stranded cDNA synthesis may easily be conducted using a commercially available kit for cDNA synthesis, for example, cDNA Synthesis System Plus® (manufactured and sold by Amersham, England; BRL Inc., U.S.A.), cDNA System Kit® (manufactured and sold by Pharmacia LKB, Sweden), cDNA Synthesis Kit® (manufactured and sold by Boehringer Mannheim GmbH, Germany), and the like. When the quantity of the synthesized cDNA is small, the cDNA can be amplified using a conventional method, such as PCR (polymerase chain reaction) method ("PCR Technology", edited by H.A. Erlich, published by Stockton Press, 1989) using a PCR kit, such as AmpliTaq (manufactured and sold by Perkin Elmer Cetus, U.S.A.). Thus, double-stranded cDNAs are obtained.

Step (IV): Preparation of a cDNA library

Using the cDNAs prepared in Step (III), a cDNA library is prepared by a customary method. That is, the cDNAs prepared in Step (III) are individually ligated to replicable cloning vectors, to thereby obtain a cDNA library. As a replicable cloning vector, any known or commercially available vectors, such as phage, cosmid, plasmid and animal virus may be used. When a phage or a cosmid is used as a replicable vector, in order to attain high stability and high transforming ability of the vector after each of the cDNA fragments has been individually inserted therein, the in vitro packaging of each of the cDNA-inserted vectors is conducted by a customary method. Thus, the cDNA-inserted vectors are obtained in the form of a recombinant phage particle. The obtained phage particles are used as a cDNA library for cDNA cloning. On the other hand, when a plasmid is used as a replicable vector, the above-mentioned cDNA fragments are individually inserted in the plasmid vectors and the resultant cDNA-inserted vectors are then individually introduced into sensitive host cells, such as cells of Escherichia coli, Bacilus, subtilis, yeast or the like, according to a customary method. The thus obtained transformants are used as a cDNA library for cDNA cloning. Further, when the animal virus gene is used as a replicable vector, the above-mentioned cDNA fragments are individually inserted in the virus vectors and the resultant recombinant viruses are then individually infected into sensitive animal cells according to a standard method and multiplied in the cells. In the case of the recombinant virus, the obtained recombinant viruses as such are used as a cDNA library.

The preparation of the cDNA library may easily be conducted using a commercially available kit, for example, a cDNA cloning system lambda gt10 and lambda gt11 (manufactured and sold by Amersham, England; BRL Inc., U.S.A.; and Stratagene Inc., U.S.A.), an in vitro packaging system (manufactured and sold by Amersham, England; BRL Inc., U.S.A.; and Stratagene Inc., U.S.A.) and the like.

Step (V): Cloning of a cDNA clone containing an NANBV gene from the cDNA library

In this step, a cDNA clone containing an NANBV gene is obtained. When the cDNA library is comprised of transformants, the transformants are cultured on a standard agar medium to form colonies. On the other hand, when the cDNA library is comprised of recombinant phage particles or recombinant viruses, these phage particles or recombinant viruses are used to infect known sensitive host cells, such as Escherichia coli, Bacillus subtilis, yeast, animal cell culture and the like, and cultured to form plaques, or to multiply the infected cells. The above-obtained transformant colonies, plaques or infected cells are subjected to immunoassay by at least one of the standard methods individually using serum from a convalescent patient having acute NANB hepatitis, serum from a patient having chronic NANB hepatitis, and serum from chimpanzee infected with an NANBV irrespective of whether or not the NANBV is of the type which causes a tubular structure to be formed in the cytoplasm of the hepatocyte of the chimpanzee, so that colonies, plaques or infected cells which have produced an NANBV antigen specifically reacted with at least one of the above-mentioned sera are selected and isolated. For the strict selection of the colonies, plaques and infected cells, it is preferred that the above procedure be repeated. From each of the thus selected and isolated colonies, plaques or the infected cells, a cDNA clone containing an NANBV gene is isolated according to a standard method described in T. Maniatis et al., Molecular Cloning, A Laboratory Manual, published by Cold Spring Harbor Laboratory, U.S.A., pp. 309-433 (1982). The immunoassay may be conducted by, for example, an enzyme-labeled antibody technique in which an antibody labeled with an enzyme, such as peroxidase and alkaline phosphatase is used; and a fluorescent antibody technique in which an antibody labeled with fluorescein isothiocyanate, europium or the like is used. It is preferred that the immunoassay by the above-mentioned technique be conducted by an indirect method because with the indirect method, high sensitivity immunoassay can be attained even by the use of an extremely small amount of serum from a patient. As a primary antibody to be used in the indirect method, serum from a patient having NANB hepatitis or serum from a chimpanzee having NANB hepatitis may preferably be employed because these sera contain an antibody specific for an NANBV antigen in relatively large amount. As a secondary antibody to be used in the indirect method, a commercially available anti-human Ig (immunoglobulin) antibody labeled with an enzyme, a fluorescent substance or the like may be used.

A specimen to be subjected to immunoassay may be prepared according to a conventional method, for example, a blotting method in which nucleic acids and proteins of the colonies, plaques and infected cells are adsorbed on a filter membrane, a method in which a microplate or a slide glass for microscopy is used, or the like. When the blotting method is used in combination with an indirect, enzyme-labeled antibody technique, the selection of the intended colonies, plaques or infected cells from an extremely large number of the original colonies, original plaques or original infected cells can be conducted easily and promptly. In this case, blotting is conducted by contacting a commercially available filter made of nitrocellulose, cellulose acetate, nylon or the like, with the colonies, plaques or infected cells.

The above-obtained cDNA clone is a part of the NANBV gene. Therefore, in order to obtain cDNA clones covering the entire region of the NANBV gene, it is requisite to extend the cNDA clone by a method in which cDNA fragments adjacent to the cDNA clone are isolated by using 3'- and 5'- terminals of the cDNA clone as a probe. In this case, the technique which is known as "gene walking" (also known as "genomic walking" or "chromosome walking") may be employed ("DNA cloning volume III", edited by D.M. Glover, pp.37-39, IRL Press, 1987; "Molecular Cloning - a laboratory manual" 2nd edit., T. Maniatis et al, 3.21 - 3.23, 1989). By the repetition of the cloning procedure and the gene walking, the entire region of the NANBV gene can be obtained in the form of cDNA clones.

Further, the nucleotide sequence of each of the obtained cDNA clones is determined. The determination of the nucleotide sequence of the cDNA clone may be conducted according to a conventional method, for example, the Maxam-Gilbert method, the dideoxy chain termination method (Analytical Biochemistry, 152, 232-238, 1986); or the like.

Based on the determined nucleotide sequence, the amino acid sequence can be deduced. The sequencing of the amino acids is conducted from the location of the initiation codon (ATG on the cDNA or AUG on the mRNA). Important portions of the amino acid sequence, for example, a hydrophilic portion, which is considered to constitute an epitope, can be identified by synthesizing a peptide corresponding to each hydrophilic portion and purifying the synthesized polypeptide by HPLC (high performance liquid chromatography), followed by subjecting the purified peptide to EIA (enzyme immunoassay) or RIA (radioimmunoassay).

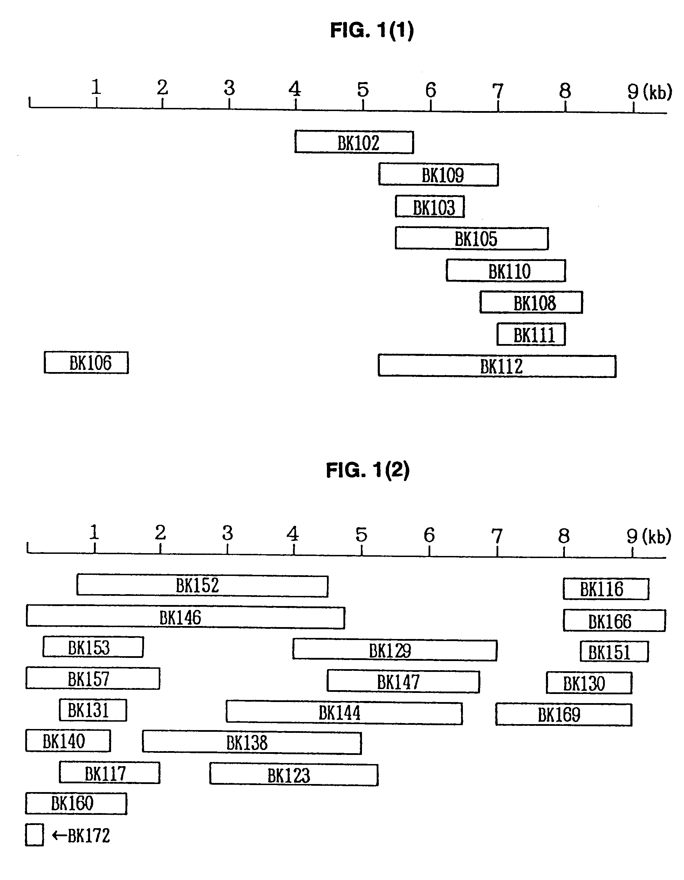

The cDNA clones are preferably classified into groups according to the respective properties of the NANBV antigens coded for by the cDNA clones in order to distinguish clones from one another. In this connection, the location of each cDNA clone on the restriction map of the NANBV gene can be used as a yardstick for the classification (see Fig. 1(1) and Fig. 1(2)]. Further, it has been found that some of NANBVs have the ability to cause a tubular structure to be formed in the cytoplasm of a hepatocyte of a chimpanzee, and some of NANBV do not have such ability (Science, 205, pp. 197-200, 1979). Therefore, the cDNA clones may be identified and classified by examining the serological reactivity of each cDNA clone with serum from a chimpanzee infected with an NANBV of the type which causes a tubular structure to be formed in the cytoplasm of the hepatocyte of the chimpanzee and with serum from a chimpanzee infected which an NANBV of the type which does not cause a tubular structure to be formed in the cytoplasm of the hepatocyte of the chimpanzee. The examination of this serological reactivity may be conducted by immunoassay mentioned above.

In the present invention, as shown in Figs. 1(1) and 1(2), the cDNA clones of the NANBV gene to be used in the present invention are identified with prefix "BK".

Fig. 1(1) is a diagram showing the relationships between the cDNA clones of the NANBV gene to be used in the present invention, shown relative to the entire region of the NANBV gene, and Fig. 1(2) is a diagram showing the relationships between the cDNA clones obtained by gene walking, shown relative to the entire region of the NANBV gene.

These BK NANBV cDNA clones include, for example, Escherichia coli BK 108 (deposited at Fermentation Research Institute, Japan under the accession number FERM BP-2971), Escherichia coli BK 129 (deposited at Fermentation Research Institute, Japan under the accession number FERM BP-2972), Escherichia coli BK 138 (deposited at Fermentation Research Institute, Japan under the accession number FERM BP-2973), Escherichia coli BK 153 (deposited at Fermentation Research Institute, Japan under the accession number FERM 2974), Escherichia coli BK 157 (deposited at Fermentation Research Institute, Japan under the accession number FERM BP-3243), escherichia coli BK 166 (deposited at Fermentation Research Institute, Japan under the accession number FERM BP-2975), and Escherichia coli BK 172 (deposited at Fermentation Research Institute, Japan under accession number FERM BP-2976). These seven BK NANBV cDNA clones are considered to cover at least the entire region of the open reading frame of the NANBV gene and probably the entire region of the NANBV gene (see Fig. 1(1) and Fig. 1(2) hereof). Further, in addition to the above-mentioned cDNA clones, the following five clones are deposited at Fermentation Research Institute, Japan as representative ones of the BK NANBV cDNA clones: Escherichia coli BK 102 (deposited at Fermentation Research Institute, Japan under the accession number FERM BP-3384), Escherichia coli BK 106 (deposited at Fermentation Research Institute, Japan under the accession number FERM BP-3385), Escherichia coli BK 112 (deposited at Fermentation Research Institute, Japan under the accession number FERM BP-3386), Escherichia coli BK 146 (deposited at Fermentation Research Institute, Japan under the accession number FERM-3387), and Escherichia coli BK 147 (deposited at Fermentation Research Institute, Japan under the accession number FERM BP-3388).

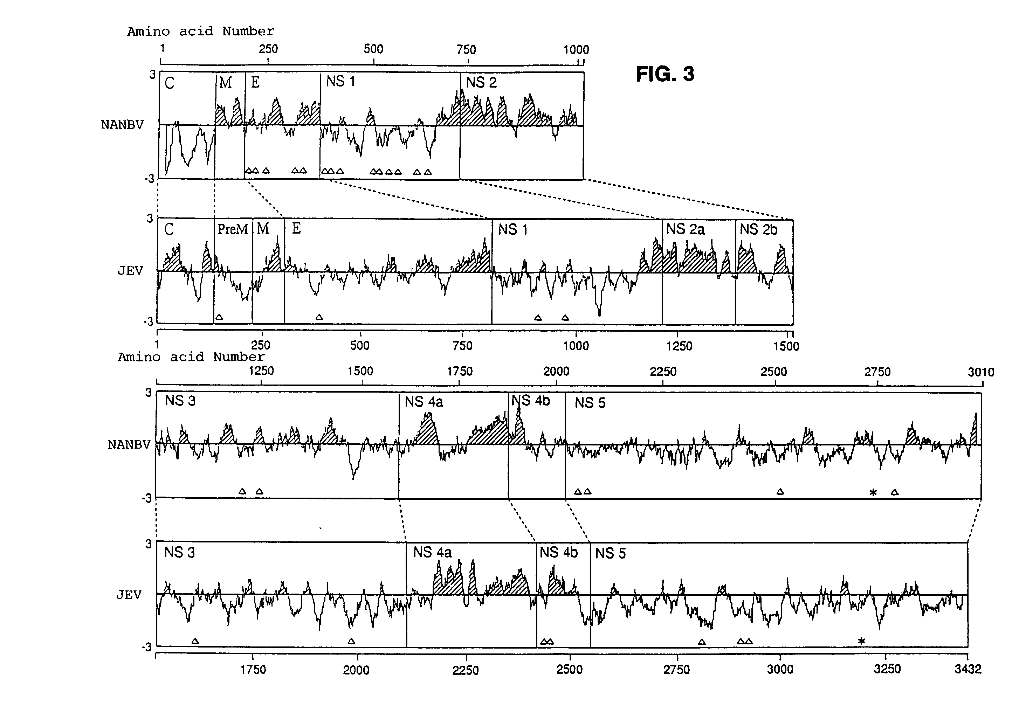

The nucleotide sequence of the entire region of the NANBV gene which is covered by the above-mentioned BK NANBV cDNA clones and the amino acid sequence which is coded for by this nucleotide sequence are shown in Fig. 2(1) through Fig. 2(16). Based on the entire NANBV nucleotide sequence and the entire NANBV amino acid sequence shown in Fig. 2(1) through Fig. 2(16), various studies and observations can be made with respect to the homology of the nucleotide sequence and amino acid sequence of the NANBV gene to those of other virus genes, the hydrophobicity index shown in Fig. 3 (hydrophobicity/hydrophilicity profile), the structure of the NANBV gene, the regions of epitopes (antigenic determinants) and the like.

With respect to the homology, studies can be made by comparison of the nucleotide sequence and amino acid sequence of the NANBV gene with those of various viruses whose genes are well known (Japanese Patent Application Laid-Open Specification No. 62-286930 and "Virology", Vol. 161, pp. 497-510, 1987) and those of other viruses, such as bovine virus diarrhea-mucosal disease virus ("Virology", Vol. 165, pp. 497-510, 1988), swine cholera virus ("Virology", Vol. 171, pp. 555-567, 1989), tobacco vein mottling virus ("Nucleic Acid Research, Vol. 165, pp. 5417-5430, 1986), etc.

With respect to the analysis of the hydrophobicity index, studies can be made by techniques using, for example, a genetic information processing software, SDC-Genetyx (manufactured and sold by SDC Software Co., Ltd., Japan), Doolittle's program (Journal of Molecular Biology, Vol. 157, pp. 105-132, 1982) and the like.

The regions of the NANBV gene coding for the various antigens (proteins) of the NANB virus particle, that is, three structural proteins, namely, virus core antigen (protein) (C antigen), matrix antigen (protein) (M antigen) and envelope antigen (protein) (E antigen) and six non-structural proteins (NS proteins) can be determined by comparing the peptides coded for by the genes with known flavivirus with respect to the hydrophobicity index and comparing the amino acid sequences of the peptides with the peptide linking sites which are acted on by the signal peptidase derived from the' host cell (Journal of Molecular Biology, 167, 391-409, 1983) and the serine protease derived from the known flavivirus (virology, 171, 637-639, 1989). With respect to the NANBV particle of the present invention, the antigens (proteins) are, respectively, coded for by the following nucleotide sequences shown in Fig. 2(1) through Fig. 2(16):

C antigen:      from the 333rd to 677th nucleotides

M antigen:      from the 678th to 905th nucleotides

E antigen :       from the 906th to 1499th nucleotides
NS1 protein:       from the 1500th to 2519th nucleotides
NS2 protein:       from the 2520th to 3350th nucleotides
NS3 protein:       from the 3351st to 5177th nucleotides
NS4a protein:       from the 5178th to 5918th nucleotides
NS4b protein:       from the 5919th to 6371th nucleotides
NS5 protein:       from the 6372nd to 9362nd nucleotides

These nucleotide sequences are useful for the diagnosis of NANB hepatitis. The antigens (proteins) which are respectively coded for by these nucleotide sequences are useful as antigens for not only vaccines but also diagnostic reagents for NANB hepatitis. Furthermore, it has been found that since the NANBV particle of the present invention has various types of epitopes, a diagnostic reagent using the NANBV particle or NANBV antigen assembly of the present invention as an antigen has a broad antigen-antibody reaction spectrum and therefore can react to a wide variety of antibodies produced by infection with NANB hepatitis virus, as compared to an antigen containing a single epitope, so that it has high sensitivity in detecting NANB hepatitis, as shown in the Examples described later.

Step (VI): Expression of the entire region of the NANBV genomic cDNA and the ORF thereof and a mass production of an NANBV antigen assembly, an incomplete NANBV particle and an infective, complete NANBV particle.

In order to express the NANBV genomic cDNA cloned in Step (V) and produce an NANBV particle on a commercial scale, part or whole of the cloned cDNA present in the cDNA clone is taken out by cutting from the replicable cloning vector and recombined with a replicable expression vector. Illustratively stated, part or whole of the cDNA of each cDNA clone is taken out by cutting with a restriction enzyme to obtain an cDNA fragment containing an NANBV antigen gene (hereafter referred to as "NANBV DNA fragment"). The NANBV DNA fragments are ligated in sequence so that the entire region of the NANBV gene or the entire region of the ORF thereof is constructed and then inserted in a replicable expression vector. In order to simplify the ligating procedure for the cloned NANBV DNA fragments and prevent the occurrence of a mistake in the ligation, not greater than ten different, preferably not greater than five different NANBV DNA fragments are used for the construction of the entire region of NANBV genomic cDNA or the entire region of the ORF thereof. To realize this, NANBV DNA fragments covering the entire region of the NANBV gene or the entire region of the ORF thereof and each having a length of at least 1000 nucleotides, preferably, 1500 nucleotides, are strictly selected and any overlapping between the fragments is deleted and then the NANBV DNA fragments are ligated in sequence to thereby construct the entire region of the NANBV gene or the entire region of the ORF thereof. That is, it is necessary to provide not more than ten different cDNA clones each comprising at least 1000 nucleotides and prepared from a NANBV genomic RNA fragment of at least 1000 nucleotides. The different cDNA clones contain their respective cloned cDNA fragments which, on the whole, cover a region of at least the 333rd to 5177th nucleotides of the non-a, non-B hepatitis virus entire nucleotide sequence from the 1st to 9416th nucleotides shown in Fig. 2(1) through Fig. 2(16) hereof. The cDNA fragments are taken out from the cDNA clones by cutting so as to respectively have predetermined nucleotide sequences such that when the predetermined nucleotide sequences are arranged in sequence, the resultant nucleotide sequence would have at least a region which coincides with the region of the 333rd to 5177th nucleotides.

The above-mentioned desired region of NANBV gene can be constructed by using, for example, BK 112, BK 146, BK 147, BK 157 and BK 166 selected from the NANBV cDNA clones disclosed in Fig. 1(1) and Fig. 1(2).

The taken-out cDNA fragments respectively having the above-mentioned predetermined nucleotide sequences are ligated in sequence to thereby construct a first deoxyribonucleic acid comprising a nucleotide sequence comprising at least the 333rd to 5177th nucleotides of the non-A, non-B hepatitis virus entire nucleotide sequence from 1st to 9416th nucleotides shown in Fig. 2(1) through Fig. 2(16) hereof.

As the replicable expression vector which may be used in this step, any conventionally known or commercially available expression vector can be used. Examples of expression vectors include plasmid vector pSN508 for enterobacteria (U.S. Patent No. 4,703,005), plasmid vector pBH103 for yeast, and its series (Japanese Patent Application Laid-Open Specification No. 63-22098), plasmid vector pJM105 (Japanese Patent application Laid-Open Specification No. 62-286930), vaccinia virus WR strain (ATCC VR-119) and vaccinia virus LC16m8 strain (Japanese Patent Application Publication 55-23252), an attenuated varicella virus Oka strain (U.S. Patent No. 3,985,615), an attenuated Marek's disease virus [The Journal of Japanese Society of Veterinary, 27, 20-24 (1984), and Gan Monograph on Cancer Research, 10, 91-107 (1971)], plasmid vector pTTQ series (manufactured and sold by Amersham, England), plasmid vector pSLV series (manufactured and sold by Pharmacia LKB, Sweden), and the like.

The NANBV DNA-inserted expression plasmid vectors are individually introduced or transfected into host cells sensitive to the vector according to a conventional method, to obtain transformants which are capable of producing an NANBV particle. Then, the transformant(s) which has produced an NANBV particle is selected. The production of an NANBV particle may be detected by the immunoassay mentioned in Step (V). Further, the production of an NANBV particle may be confirmed or detected according to a conventional method, such as electron microscopy, immunoelectron microscopy, density-gradient centrifugation, light scattering photometry or the like. As mentioned above, when a plasmid is used as an expression vector, a transformant having a capability of producing NANBV particles may be obtained. On the other hand, when an animal virus gene is used as an expression vector, a recombinant virus which is capable of producing an NANBV particle is obtained.

By culturing the transformant or recombinant virus obtained above according to a customary method, an NANBV particle can be produced in the culture of the transformant or recombinant virus on a commercial scale. With respect to the details of the method in which an animal virus gene is used as an expression vector, reference may be made to European patent Application Publication No. 0 334 530 A1.

Accordingly, in still another aspect of the present invention, there is provided a method for producing an isolated non-A, non-B hepatitis virus particle, which comprises:

(a) providing not more than ten different cDNA clones each comprising at least 1000 nucleotides and prepared from a non-A, non-B hepatitis virus genomic RNA fragment of at least 1000 nucleotides, said not more than ten different cDNA clones containing their respective cloned cDNA fragments which, on the whole, cover a region of at least the 333rd to 5177th nucleotides of the non-A, non-B hepatitis virus entire nucleotide sequence from the 1st to 9416th nucleotides shown in Fig. 2(1) through Fig. 2(16) hereof;

(b) taking out said cDNA fragments from said cDNA clones by cutting so as to respectively have predetermined nucleotide sequences such that when the predetermined nucleotide sequences are arranged in sequence, the resultant nucleotide sequence has at least a region which coincides with the region of the 333rd to 5177th nucleotides;

(c) ligating said taken-out cDNA fragments respectively having said predetermined nucleotide sequences in sequence to thereby construct a first deoxyribonucleic acid comprising a nucleotide sequence comprising at least the 333rd to 5177th nucleotides of the non-A, non-B hepatitis virus entire nucleotide sequence from the 1st to 9416th nucleotides shown in Fig. 2(1) through Fig. 2(16) hereof;

(d) introducing at least one deoxyribonucleic acid selected from said first deoxyribonucleic acid and a second deoxyribonucleic acid obtained by substituting at least one nucleotide of the nucleotide sequence of said first deoxyribonucleic acid in accordance with the degeneracy of the genetic code into a replicable expression vector selected from a plasmid and an animal virus gene to obtain a replicable recombinant DNA comprising said plasmid and said at least one deoxyribonucleic acid introduced therein when said replicable expression vector is a plasmid or obtain a recombinant virus comprising said animal virus and said at least one deoxyribonucleic acid introduced therein when said replicable expression vector is an animal virus gene;

(e) transfecting prokaryotic or eukaryotic cells with said recombinant DNA when said replicable expression vector used in step (d) is a plasmid, to thereby form a transformant, followed by selection of said transformant from parent cells of the prokaryotic or eukaryotic cell culture;

(f) culturing said transformant obtained in step (e) in prokaryotic or eukaryotic cells to thereby produce a non-A, non-B hepatitis virus particle, or culturing said recombinant virus obtained in step (d) in eukaryotic cells to thereby produce a non-A, non-B hepatitis virus particle together with an animal virus; and

(g) isolating said non-A, non-B hepatitis virus particle.

In the above method, the deoxyribonucleic acid may preferably comprise a nucleotide sequence of the 333rd to 5918th nucleotides, a nucleotide sequence of the 333rd to 6371st nucleotides, a nucleotide sequence of the 333rd to 9362nd nucleotides, or a nucleotide sequence of the 1st to 9416th nucleotides.

It should be noted that in order to produce the NANBV particle of the present invention, the region of the NANBV cDNA to be expressed is required to contain all of the nucleotide sequences respectively coding for NS1 protein, NS2 protein and NS3 protein of NANBV in addition to all of the nucleotide sequences respectively coding for core antigen, matrix antigen and envelope antigen of NANBV.

Step (VII): Purification of an NANBV particle

The NANBV particle produced in the culture of the transformant or recombinant virus may be purified using an appropriate combination of customary techniques, for example, salting-out; adsorption and desorption using a silica gel, an activated carbon or the like; precipitation by an organic solvent; fractionation by ultracentrifugation; separation by ion exchange chromatography or affinity column chromatography; fractionation by high-performance liquid chromatography or electrophoresis, and the like.

When the NANBV particle is purified from the culture of an E. coli transformant or a yeast transformant, from the viewpoint of effective removal of allergens derived from E. coli and yeast which cause the quality of the produced NANBV particle to be markedly lowered, it is preferred that the purification be conducted by, for example, the steps of (1) adsorption and elution using a silica gel, removal of impurities by adsorption on an activated carbon and (2) fractionation by density gradient centrifugation in this order (Japanese Patent Application Laid-Open Specification No. 63-297). When the NANBV particle is purified from the culture of a recombinant virus, e.g., the culture of a recombinant virus-infected cells, a high purity NANBV particle can be obtained by subjecting a crude solution containing the particle to purification by ultracentrifugation and density gradient centrifugation repeatedly. Furthermore, for inactivating the NANBV particle in the culture to secure the safe handling of the particle and for fixing the particle to stabilize the immunogenicity and the antigenicity of the particle, it is preferred to add a conventional inactivating agent to the culture of the transformant or recombinant virus-infected cells, or to a culture liquid obtained by removing the transformant cells or the recombinant virus-infected cells. For example, an inactivating agent, such as formalin, may be added in an amount of from 0.0001 to 0.001 (v/v)% in the final concentration, followed by incubation at 4 to 37 °C for 5 to 90 days to thereby inactivate the NANBV particle. It should be noted that when an attenuated virus is used as an expression vector, an NANBV particle obtained from the recombinant virus can be used as an active ingredient for a live attenuated vaccine without the step of inactivation. The thus obtained NANBV particle suspension which is highly purified can be used for the preparation of a vaccine and a diagnostic reagent, as an original NANBV particle solution (an original NANBV vaccine solution).

In a further aspect of the present invention, there is provided a recombinant comprising a replicable expression vector selected from a plasmid and an animal virus gene and a deoxyribonucleic acid comprising at least one nucleotide sequence selected from the group consisting of a first nucleotide sequence of the 333rd to 5177th nucleotides of the non-A, non-B hepatitis virus entire nucleotide sequence from the 1st to 9416th nucleotides shown in Fig. 2(1) through Fig. 2(16) hereof and a second nucleotide sequence obtained by substituting at least one nucleotide of said first nucleotide sequence in accordance with the degeneracy of the genetic code.

In the above-mentioned recombinant, the first nucleotide sequence may preferably comprise a nucleotide sequence of the 333rd to 5918th nucleotides, a nucleotide sequence of the 333rd to 6371st nucleotides, a nucleotide sequence of the 333rd to 9362nd nucleotides, or a nucleotide sequence of the 1st to 9416th nucleotides.

The replicable recombinant can be used not only for producing the NANBV particle of the present invention but also for amplifying the NANBV genomic cDNA to be used in the present invention by replication.

The purified NANBV particle of the present invention is useful as a diagnostic reagent for detecting NANB hepatitis.

The NANBV particle of the present invention can be formulated into a diagnostic reagent as follows. The purified NANBV particle solution obtained in Step (VII) mentioned above is dispensed in a vessel, such as a vial and an ampul, and sealed. The NANBV particle solution put in a vessel may be lyophilized before the sealing, in the same manner as mentioned above. The amount of the NANBV particle put in a vessel is generally about 1 μg to about 10 mg. Alternatively, the NANBV particle may also be adsorbed on the surface of a customarily employed support, such as a microplate, polystyrene beads, filter paper or a membrane.

The determination of the reactivity of the serum with the NANBV particle may be conducted in substantially the same manner as described in Step (V) mentioned above. That is, the determination of the reactivity may be conducted by a conventional immunoassay method, such as radioimmunoassay (RIA), enzyme-linked immunosorbent assay (ELISA), fluorescent antibody technique (FA), passive haemagglutination (PHA), reversed passive haemagglutination (rPHA) and the like. The amount of the NANBV particle to be used for the above immunoassay is generally from about 0.1 to about 100 mg/ml of serum. Particularly, the amounts of the NANBV particle to be used for RIA, ELISA, FA, PHA and rPHA are generally from 0.1 to 1 mg/ml, from 0.1 to 1 mg/ml, from 1 to 100 mg/ml, from 1 to 50 mg/ml and from 1 to 50 mg/ml, respectively.

The NANBV particle of the present invention may also be used for screening blood for transfusion. The screening method consists in:

a) isolating serum from whole blood;

b) contacting serum of an unknown blood with an isolated NANBV particle;

c) determining whether the serum reacts with the NANBV particle;

d) classifying the serum as positive or negative to non-A, non-B hepatitis based on the reactivity; and

e) effecting separation of the blood in accordance with the identification.

The contact of serum of an unknown blood with the NANBV particle of the present invention, and the determination of the reactivity of the serum of the blood with the NANBV particle may be conducted in the same manner as mentioned above with respect to the method for diagnosing NANB hepatitis. By the above method, a blood for transfusion free from the NANBV can be selected.

The polyclonal antibody and monoclonal antibody specific for the NANBV particle of the present invention may be used as an agent for removing NANBV from blood for transfusion. That is, NANBV present in blood can efficiently be removed by the polyclonal antibody or the monoclonal antibody by antigen-antibody reaction.

Further, the NANBV particle of the present invention may advantageously be used as an active ingredient of a vaccine for NANB hepatitis. The vaccine for NANB hepatitis may be prepared as follows. The culturing of a transformant containing a recombinant phage or plasmid carrying the cDNA coding for the NANBV particle, or a cell infected with the recombinant virus carrying the cDNA coding for the NANBV particle is conducted in the same manner as described above to thereby produce the NANBV particle in the culture. For inactivating the NANBV particle in the culture to secure the safety of the NANBV particle and for fixing the NANBV particle to stabilize the immunogenicity and the antigenicity of the particle, it is preferred to add a conventional inactivating agent to the culture of the transformant or recombinant virus-infected cell,or to a culture medium obtained by removing the transformant cells or the recombinant virus-infected cell. For example, an inactivating agent, such as formalin, may be added in an amount of from 0.0001 to 0.001 v/v%, followed by incubation at 4 to 37 °C for 5 to 90 days. Then, the resultant culture or culture medium is subjected to purification in the same manner as mentioned above. Thus, an original NANB hepatitis vaccine solution containing the purified NANBV particle is obtained.

The original NANBV hepatitis vaccine solution is filtered using a microfilter by a standard method to sterilize the solution. The filtrate is diluted with physiological saline so that the protein concentration is about 1 to about 500 µg/ml as measured by the Lowry method. Further, at least one stabilizing agent may be added. As the stabilizing agent, any commercially available stabilizing agent may be used. Examples of stabilizing agents include gelatin and hydrolysates thereof, human albumin, saccharides such as glucose, fructose, galactose, sucrose and lactose, and amino acids such as glycine, alanine, lysine, arginine and glutamine. Also, an adjuvant may be used to prepare an adsorbed vaccine. In this case, an adjuvant, such as, an aluminum hydroxide gel is added to the solution, before the addition of a stabilizing agent, so that the concentration of the added gel becomes about 0.1 to about 1.0 mg/ml, followed by mixing, thereby adsorbing the NANBV particle onto the adjuvant. As an adjuvant, there may also be employed precipitating depositary adjuvants such as calcium phosphate gel, aluminum phosphate gel, aluminum sulfate, alumina and bentonite, and adjuvants which are capable of inducing antibody production such as muramyl peptide derivatives, polynucleotides, Krestin® (manufactured and sold by Kureha Chemical Industry Co., Ltd., Japan) and picibanil (both of which are an antineoplastic agent).

Then, the thus obtained NANB hepatitis vaccine solution containing an (gel-adsorbed or non-adsorbed) NANBV particle is dispensed into a small vessel, such as an ampul and a vial, and sealed. Thus, there is obtained a purified (adsorbed or non-adsorbed) NANB hepatitis vaccine comprising an (adsorbed or non-adsorbed) NANBV particle.

The NANB hepatitis vaccine solution thus obtained may be lyophilized to obtain the NANB hepatitis vaccine in a dried form so that the product can be transported to and stored at a place of severe climate, for example, in an area in the tropics. The lyophilization may generally be conducted according to a standard method after the liquid (adsorbed or non adsorbed) NANB hepatitis vaccine is dispensed in a vessel such as a vial and an ampul. After lyophilization, a nitrogen gas is introduced in the vessel containing the dried vaccine, followed by sealing. Incidentally, the quality of the vaccine produced is examined in accordance with "Adsorbed Hepatitis B Vaccine", "Dried Japanese Encephalitis Vaccine", and "Adsorbed Pertussis Vaccine" provided for in Notification No. 159 of the Ministry of Health and Welfare, Japan, "Minimum Requirements for Biological Products".

The NANB hepatitis vaccine may be prepared in the form of a mixed vaccine which contains an adsorbed NANBV particle mentioned above and at least one antigen other than the present NANBV particle. As the antigen other than the present NANBV particle, there may be employed any antigens that are conventionally used as active ingredients of the corresponding vaccines insofar as the side effects and adverse reactions caused by such other antigens and the NANBV particle are not additively or synergistically increased by the use of the NANBV particle and such other antigens in combination and the antigenicities and immunogenicities of the NANBV particle and such other antigens are not reduced by the interference between the NANBV particle and other antigens. The number and the types of the antigens which may be mixed with the NANBV particle are not limited insofar as the side effects and adverse reactions are not increased additively or synergistically and the antigenicity and immunogenicity of each of the NANBV particle and such antigens are not reduced as mentioned above. Generally, two to six types of antigens may be mixed with the NANBV particle. Examples of antigens which may be mixed with the present NANBV particle, include detoxified antigens, inactivated antigens or toxoids which are derived from Japanese encephalitis virus, HFRS (hemorrhagic fever with renal syndrome) virus, influenza virus, parainfluenza virus, hepatitis B virus, dengue fever virus, AIDS virus, Bordetella pertussis, diphtheria bacillus, tetanus bacillus, meningococcus, pneumococcus and the like.

Generally, the vaccine comprising the NANBV particle of the present invention may be contained and sealed in a vial, an ampul or the like. The vaccine of the present invention may generally be administered in the form of a liquid or suspension. In the case where the vaccine is in a dried form, the vaccine is dissolved or suspended in sterilized distilled water before administration, the amount of the distilled water being such that the volume becomes the original volume before being subjected to lyophilization. Generally, the vaccine may be administered subcutane-ously. The dose of the vaccine may generally be about 0.5 ml. In general, the dose of the vaccine for a child may be half as much as that of the vaccine per adult. The vaccine may generally be administered twice at an interval of about one week to one month and then, about half a year later, administered once more.

Further, the NANBV particle may be used for preparing an antibody, such as a polyclonal antibody and a monoclonal antibody, specific for the NANBV particle. For example, a polyclonal antibody specific for the NANBV particle may be prepared by a conventional method as follows. The purified NANBV particle of the present invention is inoculated subcutaneously, intramuscularly, intraperitoneally or intravenously to an animal, such as mouse, guinea pig and rabbit. The inoculation of the NANBV particle is generally conducted several times at intervals of 1 to 4 weeks, to thereby completely immunize the animal. In order to enhance the immunizing effect, a conventional and commercially available adjuvant may be used. Then, blood serum is collected from the immunized animal and an anti-NANBV particle polyclonal antibody is isolated and purified from the blood serum according to a standard method.

On the other hand, a monoclonal antibody specific for the NANBV particle may be prepared by a conventional method as described, for example, in Cell Technology, 1, 23-29 (1982). For example, splenic cells obtained from a mouse immunized with the purified NANBV particle are fused with commercially available mouse myeloma cells by cell fusion technique, to obtain hybridomas. The hybridomas are screened to obtain a hybridoma capable of producing an antibody reactive with the NANBV particle. The obtained hybridoma is cultured in a standard method. From the supernatant of the culture, an anti-NANBV particle monoclonal antibody is isolated and purified by a standard method.

The above-mentioned polyclonal antibody and monoclonal antibody may also be used as a diagnostic reagent for diagnosing NANB hepatitis. The diagnosis of NANB hepatitis using the antibody may be conducted by immunoassay in substantially the same manner as mentioned above with respect to the diagnosis of NANB hepatitis using the NANBV particle. By the use of the polyclonal antibody or the monoclonal antibody, the identification and quantification of the NANBV particle present in a liver tissue and blood can be conducted.

The NABV particle of the present invention has an extremely broad spectrum of antigenicity and specifically reacts with the serum not only of a chronic NANBV patient but also of an acute NANBV patient. Therefore, the NANBV particle is able to provide a diagnostic reagent of high reliability having not only a high detection ratio for an antibody but also a high precision in the detection. Further, when the NANBV particle of the present invention is used for screening blood for transfusion, blood which is infected by NANBV can be selected easily with high reliability and removed from blood not infected by NANBV. Therefore, the post-transfusion NANB hepatitis can be prevented.

Further, the NANBV particle of the present invention may advantageously be used as an active ingredient of a vaccine for preventing NANB hepatitis, which is extremely excellent in immunogenicity. In addition, a recombinant virus, e.g., recombinant vaccinia virus prepared by inserting the NANBV genomic cDNA into a vaccinia virus, is useful as an active ingredient of a vaccine.

Further, by the use of the NANBV particle of the present invention, an antibody, particularly monoclonal antibody, specific for NANBV can easily be prepared. The antibody specific for NANBV can advantageously be used as not only a diagnostic reagent for detecting NANB hepatitis, but also an agent for removing NANBV from blood for transfusion.

Furthermore, it should be noted that the NANBV particle of the present invention is not produced by the infection of an animal with a virus, but produced in an isolated form by gene expression of the DNA coding for the present NANBV particle in a host cell. Hence, the possibility of infection during the steps for production of the present NANBV particle is substantially eliminated. Also, the production cost can be decreased. Moreover, since all of the materials used in the production process, e.g., medium for the incubation system, are well-known in respect of the composition thereof, purification is facile and an NANBV particle product having high purity can be obtained.

By the present invention, it is possible to produce an isolated NANBV particle and its gene with high purity which cannot be found in nature. The produced NANBV particle and its gene can greatly contribute to researches on NANB hepatitis, hepatoma, liver cancer, etc.

The present invention will now be described in detail with reference to the following Examples and Reference Examples, which should not be construed to be limiting the scope of the present invention. Example 1 is divided into Part I and Part II, and Reference Examples 1-3 are inserted therebetween.

Example 1 (Part I)

Step 1 (Preparation of a plasma-derived RNA for producing a cDNA, which is complementary to NANBV genome RNA)

In order to obtain NANBV from plasma, 4.8 liters of human plasma exhibiting a glutamic-pyruvic transaminase (GPT) activity of 35 IU/liter or more (as measured by the method of Wroblewski, F & J.S. LaDue: Serum glutamic-pyruvic transaminase in cardiac and hepatic disease. Proc. Soc. Exp. Biol. Med., 91:569, 1956) was applied on a 30 % (w/w) aqueous sucrose solution, and subjected to centrifugation at 48,000 x g at 4 °C and for 13 hours to obtain a precipitate. The precipitate was suspended in an aqueous solution containing 50 mM Tris·HCl (pH 8.0) and 1 mM EDTA, and once more subjected to centrifugation at 250,000 x g at 4 °C and for 3 hours to thereby obtain a precipitate. The obtained precipitate was dissolved in 75 ml of 5.5 M GTC solution containing 5.5 M quanidine thiocyanate, 20 Mm sodium citrate (pH 7.0), 0.05 % sarkosyl (sodium lauryl sarcosinate) and 0.1 M 2-mercaptoethanol. The resultant solution was applied on 16 ml of CsTFA-0.1 M EDTA solution ( $\rho$ = 1.51), and subjected to centrifugation at 140,000 x g at 15 °C and for 20 hours to thereby obtain a precipitate of RNA. The supernatant containing proteins and DNA was removed by suction, and the precipitate was dissolved in 200 µl of TE buffer solution containing 10 mM Tris·HCl (pH 8.0) and 1 mM EDTA. 20 µl of 3 M sodium chloride and ethanol were added to the solution, and allowed to stand still at -70 °C for 90 minutes. The mixture was centrifuged at 12,000 X g at 4 °C and for 30 minutes to obtain a precipitate. The precipitate was dissolved in TE, and sodium chloride and ethanol were added in the same manner as mentioned above. The mixture was allowed to stand still at -70 °C to obtain a precipitate. The precipitate was dissolved in 10 µl of TE to thereby obtain a purified RNA.

Step 2 (Preparation of a liver-derived RNA for producing a cDNA, which is complementary to NANBV genome RNA)

NANBV genome RNA was prepared from a liver tissue cut off from a NANBV hepatitis patient by the method of Okayama et al. (see H. Okayama, M. Kawaichi, M. Brown-stein, F. Lee, T. Yokota, and K. Arai: High-Efficiency Cloning of Full-Length cDNA; Construction and Screening of cDNA Expression Libraries for Mammalian Cells, Methods in Enzymology 154,.3-28, 1987).

Illustratively stated, 1 g of liver tissue was cut into small pieces. The small pieces were suspended in 100 ml of 5.5 M GTC solution as used in Step 1, and homogenized by means of a Teflon-glass homogenizer. Subsequently, the introduction of the homogenate into a syringe having #18 needle and the discharge of the homogenate from the syringe through the needle were repeated to thereby mechanically split DNA. The resultant homogenate was centrifuged at 1,500 x g (lower centrifugal force) at 4 °C and for 15 minutes to thereby obtain a supernatant. The supernatant was superposed on CsTFA solution and centrifuged in substantially the same manner as described in Step 1 to thereby obtain a precipitate as an RNA fraction. The thus obtained precipitate was suspended in 0.4 ml of 4 M GTC solution. 10 µl of 1 M acetic acid and 300 µl of ethanol were added to the suspension, and allowed to stand still at -20 °C for at least 3 hours to thereby obtain a precipitate of RNA. The precipitate was separated by centrifugation at 12,000 x g at 4 °C and for 10 minutes, and dissolved in 1 ml of TE solution. 100 µl of 2 M sodium chloride solution and 3 ml of ethanol were added to the solution, and the mixture was allowed to stand still at -20 °C for 3 hours. The resultant precipitate was collected by centrifugation and dissolved in 10 µl of TE to thereby obtain a purified, liver-derived RNA.

Step 3 (Preparation of a double-stranded cDNA using a cDNA synthesis kit)

A double-stranded cDNA was prepared using a commercially available cDNA synthesis kit (manufactured and sold by Amersham International, England).

Illustratively stated, 0.75 µg of the purified RNA obtained in Step 1 and 2 µl of random hexanucleotide primer and 2 µl of reverse transcriptase taken from the reagents included in the kit were put in a reaction tube. Then, distilled water was added in an amount such that the total volume of the resultant mixture became 20 µl. The mixture was incubated at 42 °C for 40 minutes, thereby preparing a first strand of cDNA. Subsequently, a second strand of cDNA was synthesized while cooling the reaction mixture in ice water, as follows. To 20 µl of the reaction mixture were added 37.5 µl of buffer for second strand synthetic reaction, 1 µl of E. coli ribonuclease H and 6.6 µl of DNA polymerase I, which were taken from the reagents included in the kit, followed by addition of 34.9 µl of distilled water. The mixture was incubated at 12 °C for 60 minutes, 22 °C for 60 minutes and at 70 °C for 10 minutes. Then, the mixture was once more cooled with ice water. 1 µl of T4 DNA polymerase was added, incubated at 37 °C for 10 minutes, and 4 µl of 0.25 M EDTA (pH 8.0) was added to thereby terminate the reaction. The reaction mixture was mixed well with a mixture of phenol and chloroform, and centrifuged at 12,000 x g for one minute to thereby separate an aqueous layer. The aqueous layer was again subjected to the same extraction as mentioned above, and an equal amount of chloroform was added. The mixture was agitated well and centrifuged to separate an aqueous layer. Subsequently, an equal amount of 4 M ammonium acetate and a two-fold amount of ethanol were added to the aqueous layer, and the mixture was cooled to -70 °C, thereby obtaining a precipitate of purified double-stranded cDNA. The precipitate was dissolved in 50 μl of 2 M ammonium acetate. To the mixture, 100 μl of ethanol was added, and the resultant mixture was cooled to -70 °C to thereby obtain a precipitate. The precipitate was collected by centrifugation at 12,000 x g for ten minutes. The collected precipitate was dried and then, dissolved in 20 μl of TE.

Step 4 (Preparation of a double-stranded cDNA by the Polymerase Chain Reaction (PCR) method)

The cDNAs which were prepared by means of a reverse transcriptase using as templates the RNAs prepared in Steps 1 and 2, were individually amplified by the PCR method (see Saiki, R. K., Gelfand, D. H., Stoffer, S., Scharf, S. J., Higuchi, R., Horn, G. T., Mullis, K. B., and Erlich, H. A., Primer-directed enzymatic amplification of DNA with a thermostable DNA Polymerase, Science 239:487-491, 1988). That is, 5 to 1,000 ng of the RNA was incubated in 20 μl of a reverse transcriptase solution containing 50 mM Tris·HCl (pH 8.3), 40 mM KCl, 6 mM MgCl$_2$, 1 μM 3'-primer [synthesized oligonucleotide comprised of the 7949th to 7973rd 25 nucleotides in Fig. 2(14)], 10 mM dNTP, and 0.5 unit of reverse transcriptase (product of New England Bio Lab., U.S.A.) at 37 °C for 30 minutes. To the resultant mixture was added 80 μl of a PCR reaction solution containing 18 mM Tris-HCl (pH 8.3), 48 mM KCl, 1.5 mM MgCl$_2$, 0.6 μM each of 5'-primer [synthesized oligonucleotide comprised of the 7612nd to 7636th 25 nucleotides in Fig. 2(13)] and the above-mentioned 3'-primer, 10 mM dNTP and 2.5 units of Taq DNA polymerase (manufactured and sold by Perkin Elmer Cetus Co., Ltd., U.S.A.). The mixture was subjected to incubation at 94 °C for one minute, at 50 °C for 2 minutes and at 72 °C for 3 minutes. This incubation was repeated 40 times. The resultant mixture was subjected to agarose gel electrophoresis, thereby obtaining amplified cDNA. The amplified cDNA was subjected tophenol treatment, ethanol precipitation and drying. The dried cDNA was dissolved in 10 μl of TE.

Step 5 (Preparation of a cDNA library using lambda gt11)

Using a commercially available cDNA cloning kit (manufactured and sold by Amersham International, England), a cDNA library was prepared. That is, to 130 ng of cDNA prepared in step 3 were added 2 μl of L/K buffer, 2 μl of EcoRI adaptor and 2 μl of T4 DNA ligase, which were taken from the reagents included in the cloning kit. Distilled water was added to the solution in an amount such that the total volume of the resultant mixture became 20 μl. The mixture was incubated at 15 °C for 16 to 20 hours, and 2 μl of 0.25 M EDTA was added thereto, to thereby terminate the reaction. Subsequently, the mixture was passed through a size fractionating column included in the kit, thereby removing EcoRI adaptors which were not ligated to the cDNA. To 700 μl of the cDNA having EcoRI adaptor ligated thereto were added 83 μl of L/K buffer and 8 μl of T4 polynucleotidekinase. The mixture was incubated at 37 °C for 30 minutes. The resultant mixture was subjected to phenol extraction twice, concentration to 350 to 400 μl by means of butanol and then ethanol precipitation, thereby obtaining a precipitate. The precipitate was dissolved in 5 μl of TE.

Subsequently, in order to insert the cDNA having EcoRI adaptor ligated thereto to the EcoRI site of cloning vector lambda gt11, 1 μl of L/K buffer, 2 μl (1 μg) of lambda gt11 arm DNA and 2 μl of T4 DNA ligase were added to 1 μl (10 ng) of the above-mentioned cDNA having EcoRI adaptor ligated thereto. Distilled water was added to the mixture in an amount such that the total volume of the mixture became 10 μl. The mixture was incubated at 15 °C for 16 to 20 hours. Thus, a recombinant lambda gt11 DNA solution was prepared. Further, a recombinant lambda phage was obtained by in vitro packaging using a commercially available in vitro packaging kit (manufactured and sold by Stratagene Co., Ltd., U.S.A.) including Gigapack II Gold solutions A and B, SM buffer and chloroform. That is, 10 μl of Gigapack II Gold solution A and 15 μl of Gigapack II Gold solution B were added to 4 μl of the above-mentioned recombinant lambda gt11 DNA solution. The mixture was incubated at 22 °C for 2 hours to obtain a recombinant phage. After the incubation, 470 μl of SM buffer and 10 μl of chloroform were added and the recombinant phage was stored at 4 °C.

Step 6 (Cloning of cDNA using E. coli Plasmid pUC19)

Using a commercially available DNA ligation kit (manufactured and sold by Takara Shuzo Co., Ltd., Japan) including solutions A and B, the cDNA was inserted in E. coli plasmid pUC19 (C. Yanishi-Perron, J. Vieira, J. Messing, Gene 33, 103, 1985), and cloned in E. coli. That is, 40 μl of solution A and 10 μl of solution B were

added to 5 μl of the cDNA prepared by polymerase chain reaction (PCR) in Step 4 and 5 μl (50 ng) of plasmid pUC19 DNA which had been digested with restriction enzyme SmaI and dephosphorylated. The mixture was incubated at 15 °C for 16 hours. E. coli strain JM 109 (see Messing, J., Crea, R., and Seeburg, P.H., Nucleic Acids Res. 9, 309, 1981) was transformed with the above-obtained plasmid DNA according to the calcium chloride method (see Mandel, M. and A. Higa, J. Mol. Biol., 53, 154, 1970). Thus, a transformed E. coli containing the plasmid having the cDNA ligated thereto was obtained.

Step 7 (Screening of clone having NANBV gene from a cDNA library)

E. coli strain Y 1090 (see Richard A. Young and Ronald W. Davis, Science, 222, 778, 1983) was cultured in 50 ml of LBM medium containing 1 % tryptone, 0.5 % yeast extract, 1 % sodium chloride, 50 μg/ml ampicillin and 0.4 % maltose at 37 °C. The E. coli cells in a logarithmic growth phase were suspended in 15 ml of 10 mM magnesium sulfate cooled with ice. The bacteriophage solution obtained in Step 5 was diluted with SM buffer containing 0.1 M sodium chloride, 8 mM magnesium sulfate, 50 mM Tris·HCl (pH 7.5) and 0.01 % gelatin. 0.1 ml of the diluted phage solution was mixed with an equal volume of the above-mentioned E. coli cell suspension, and the mixture was incubated at 37 °C for 15 minutes. To the mixture was added 4 ml of soft agar medium containing 1 % tryptone, 0.5 % yeast extract, 0.5 % sodium chloride, 0.25 % magnesium sulfate and 0.7 % agar (pH 7.0) heated to 45 °C. The mixture was spread on L-agar plate containing 1 % tryptone, 0.5 % yeast extract, 1 % sodium chloride, 1.5 % agar and 100 μg/ml ampicillin (pH 7.0), and incubated at 42 °C for 3 hours. Subsequently, 10 mM IPTG (isopropyl β-D-thiogalactopyranoside) was infiltrated into a nitrocellulose filter and the nitrocellulose filter was dried and closely contacted with the plate. The plate in contact with the filter was incubated at 37 °C for 3 hours. The filter was separated, and washed with TBS buffer three times. The washed filter was immersed in 2 % bovine serum albumin solution, and incubated at room temperature for one hour. 1/20 volume of E. coli lysate solution included in a commercially available immunoscreening kit (manufactured and sold by Amersham International, England) was added to pooled serum from NANB hepatitis patients, and incubated at room temperature for 30 minutes. Thereafter, the serum was diluted to 50-fold with 0.2 % bovine serum albumin-added TBS buffer, and the filter was immersed in the diluted serum solution, and incubated at room temperature for one hour.

The resultant filter was washed four times with a TBS buffer containing 0.05 % Tween 20. The washed filter was immersed in an antibody solution which had been prepared by diluting a peroxidase-labeled anti-human IgG (manufactured and sold by Cappel Co., Ltd., Germany) 1,000-fold for one hour. The filter was washed with the above-mentioned Tween-TBS buffer, and immersed in a solution prepared by adding 0.4 ml of DAB (3,3′-diaminobenzidine tetrahydrochloride) and 15 μl of a 30 % aqueous hydrogen peroxide solution to 50 ml of a TBS buffer, followed by incubation at room temperature for 5 to 30 minutes to allow color development. The resultant filter was completely washed with distilled water to terminate the reaction.

By the above-mentioned procedure, the obtained plaques were purified. As a result, 9 positive clones were isolated, which were, respectively, designated as BK 102, BK 103, BK 105, BK 106, BK 108, BK 109, BK 110, BK 111 and BK 112. All of these clones did not react with serum from a healthy human, but reacted with serum from a patient suffering from NANB hepatitis. See Table 1.

## Table 1

## Reactivity between the serum obtained from a patient suffering from NANB hepatitis and the recombinant lambda gt11 phage clone

| Clone | Serum from healthy person | Serum from NANB hepatitis patient |
|---|---|---|
| BK 102 | 0/10* | 10/11 |
| BK 103 | 0/10 | 9/11 |
| BK 105 | 0/10 | 11/11 |
| BK 106 | 0/10 | 11/11 |
| BK 108 | 0/10 | 9/11 |
| BK 109 | 0/10 | 9/11 |
| BK 110 | 0/10 | 9/11 |
| BK 111 | 0/10 | 9/11 |
| BK 112 | 0/10 | 10/11 |

\* the number of positive samples/the number of specimens.

Step 8 (Determination of the nucleotide sequence of the obtained clones)

Recombinant phage DNAs of clones BK 102 to BK 112 were purified, and the DNAs were digested with restriction enzyme EcoRI. Then, cDNA fragments of NANBV were isolated and the isolated cDNAs were individually inserted into plasmid pUC19 at EcoRI site. Using the plasmids, E. Coli strain JM 109 was transformed in substantially the same manner as in Step 6. Plasmid DNAs were obtained from the transformed E. coli and purified. The nucleotide sequence of each of the NANBV cDNAs was determined using 7-DEAZA sequencing kit (manufactured and sold by Takara Shuzo Co., Ltd., Japan; see Mizusawa, S., Nishimura, S. and Seela, F. Nucleic Acids Res., 14, 1319, 1986). The relationship between the nucleotide sequences of the obtained cDNA clones is shown in Fig. 1(1).

Step 9 (Cloning of NANBV cDNA clones from a cDNA library by Genomic Walking)

Probes were prepared by labeling with $^{32}$P-dCTP the cDNA fragments of clone BK 102, clone BK 106 and clone BK 112 which were obtained in Step 8. Using the probes, phage clones containing NANBV cDNAs were obtained by hybridization from the cDNA library of cloning vector lambda gt11 obtained in Step 5. That is, plasmid DNAs were prepared from the transformed E. coli with clone BK 102, clone BK 106 and clone BK 112 obtained in Step 8 by the alkali method (see T. Maniatis, E.F. Fritsch, and J. Sambrook: Isolation of Bacteriophage λ and Plasmid DNA: "Molecular Cloning", Cold Spring Harbor Lab., pp 75-96.).

Plasmid DNA of clone BK 102 was digested with restriction enzymes NcoI and HincII, and the resultant 0.7 kb fragments having been on the 5'-terminus side of the DNA were subjected to agarose gel electrophoresis, and collected. Plasmid DNAs of clone BK 106 and clone BK 112 were digested with restriction enzyme NcoI.

In the same manner as mentioned above, 1.1 kb DNA fragments were prepared from clone BK 106, and 0.7 kb fragments having been on the 3'-terminus side were prepared from clone BK 112. 25 ng to 1 μg of DNA fragments were incubated with [α-$^{32}$P]dCTP (3000Ci/mmol; manufactured by Amersham Co., Ltd., England) at 37 °C for 3 to 5 hours, using commercially available DNA labeling kit (manufactured by Nippon Gene Co., Ltd.). Thus, probes for hybridization were prepared.

Subsequently, the cDNA library bacteriophage obtained in Step 5 was incubated at 42 °C in L-agar medium for 3 hours, as described in Step 7. Further, the bacteriophage was incubated at 37 °C for 3 hours, and was cooled. A nitrocellulose filter was contacted with phage plaques, and was allowed to stand still for 30 to 60 seconds. Thus, the phage plaques were adsorbed onto the filter.

The filter was subjected to alkali denaturation for 1 to 5 minutes using an aqueous solution containing 0.5 N sodium hydroxide and 1.5 M sodium chloride and to the neutralization with 0.5 M Tris·HCl (pH 8.0) containing 1.5 M sodium chloride for 1 to 5 minutes. The filter was washed with 2 x SSC solution containing 0.3 M sodium chloride and 0.03 M sodium citrate, dried, and baked at 80 °C for 2 hours.

The filter was incubated at 42 °C for 6 hours in a solution for hybridization containing 50 % formamide, 5 x SSC, 5 x Denhart solution, 50 mM phosphoric acidcitric acid buffer (pH 6.5), 100 μg/ml salmon sperm DNA and 0.1 % SDS. Then, the filter was immersed in 300 ml of the hybridization solution having 1 ml of the above-mentioned probe of about 4 x 10$^8$ cpm/ml added thereto, and incubated at 42 °C for 16 to 20 hours. The filter was washed with a 2 x SSC solution containing 0.1 % (w/w) SDS four times and with a 0.1 x SSC solution containing 0.1 % (w/w) SDS twice. After the washing, the filter was dried, and was subjected to autoradiography. Thus, hybridization positive clones were isolated. As a result, 27 clones being reactive with the probe derived from clone BK 102, 14 clones being reactive with the probe derived from clone BK 106 and 13 clones being reactive with the probe derived from clone BK 112, were obtained, which were respectively designated as BK 114 to BK 169.

The nucleotide sequence of each of clones BK 114 to BK 169 was determined according to the method described in Step 8, followed by mapping for each of the clones. As a result, a map of nucleotide sequence having a length of about 9.5 kb considered to be the approximately total length of the NANBV genome was obtained [see Fig. 1(2)].

Clone BK 157 located on the 5' terminus side was digested with restriction enzyme KpnI to thereby isolate a 0.55 kb fragment having been on the 5'-terminus side. Also, clone BK 116 located on the extreme 3'-terminus side was digested with restriction enzymes HpaI and EcoRI to thereby isolate a 0.55 kb fragment having been on the 3'-terminus side. A probe labeled with $^{32}$P was prepared in the same manner as described above, and the cDNA library bacterio phageobtained in Step 5 was subjected to plaque hybridization. As a result, three new additional clones were separated by the probe derived from the clone BK 157. These new clones were, respectively, designated as clones BK 170, BK 171 and BK 172.

Step 10 (Analysis of the nucleotide sequence of cDNA)

The entire nucleotide sequence of NANBV gene was determined from the nucleotide sequences of the clones obtained in Steps 8 and 9, and shown in Figs. 2(1) to 2(16). From the Figures, it was assumed that the cloned genomic cDNAs of NANBV were composed of 9416 nucleotides, wherein there was an open reading frame composed of 9030 nucleotides coding for a protein composed of 3010 amino acid residues. The hydrophilicity/hydrophobicity pattern of this protein was similar to that of flavivirus as already reported (see H. Sumiyoshi, C. Mori, I. Fuke et al., Complete Nucleotide Sequence of the Japanese Encephalitis Virus Genome RNA. Virology, 161, 497-510, 1987). Clone BK 157 covers nucleotide numbers 1 to 1962 of Figs. 2(1) to 2(16), clone BK 172 covers nucleotide numbers 5 to 366, clone BK 153 covers nucleotide numbers 338 to 1802, clone BK 138 covers nucleotide numbers 1755 to 5124, clone BK 129 covers nucleotide numbers 4104 to 6973, clone BK 108 covers nucleotide numbers 6886 to 8344 and clone BK 166 covers nucleotide numbers 8082 to 9416. They are preserved as Escherichia coli BK 108 (deposited at Fermentation Research Institute, Japan under accession number FERM BP-2971), BK 129 (deposited at Fermentation Research Institute, Japan under accession number FERM BP-2972), BK 138 (deposited at Fermentation Research Institute, Japan under accession number FERM BP-2973), BK 153 (deposited at Fermentation Research Institute, Japan under accession number FERM BP-2974), BK 157 (deposited at Fermentation Research Institute, Japan under accession number FERM BP-3243), BK 166 (deposited at Fermentation Research Institute, Japan under accession number FERM BP-2975), and BK 172 (deposited at Fermentation Research Institute, Japan under accession number FERM BP-2976), respectively.

Reference Example 1

(Production of NANBV-related antigens in E. coli, which antigens are related with the antibody-response accompanying NANBV infection)

Respective cDNAs of clone BK 106, clone BK 111 and clone BK 112 each obtained in Step 8 of Example 1 (Part I) and cDNA of clone BK 147 obtained in Step 9 of Example 1 (Part I) were individually inserted into plasmids, and the thus obtained plasmid DNAs were collected by the conventional alkali method. Subsequently, the collected DNA of clone BK 106 was digested with restriction enzymes EcoRI and ClaI to thereby obtain 0.5 μg of a DNA fragment of 0.34 kb in length. The thus obtained DNA fragment was incubated at 37 °C for 60 minutes in a T4 DNA polymerase solution containing 67 mM Tris·HCl (pH 8.8), 6.7 mM magnesium chloride, 16.6 mM ammonium sulfate, 10 mM 2-mercaptoethanol, 6.7 μM EDTA, 0.02 % bovine serum albumin, 0.3 mM dNTP and 2-5 units of T4 DNA polymerase, thereby rendering both terminals blunt. The DNA of clone BK 102 was digested with restriction enzyme BamHI to thereby obtain 0.5 μg of a DNA fragment of 0.7 kb in length, and the terminals of the DNA fragment were rendered blunt using T4 DNA polymerase in substantially the same manner as mentioned above. The DNA of clone BK 147 was digested with restriction enzyme Sau3AI to thereby obtain 0.5 μg of a DNA fragment of 1 kb in length and the terminals of the DNA fragment were rendered blunt in the same manner as mentioned above. Also, the DNA of clone BK 111 was digested with restriction enzyme EcoRI to thereby obtain 0.5 μg of a DNA fragment of 1 kb in length, and the terminals of the DNA fragment were rendered blunt in substantially the same manner as mentioned above. Subsequently, the DNA of expression vector pKK 233-2 (Amann, E. and J. Brosius. ATG vector for regulated high-level expression of cloned genes in Escherichia coli. Gene, Vol. 40, 183, 1985) was digested with restriction enzyme HindIII. 2 μg of the resultant DNA was incubated at 37 °C for 20 minutes in a S1 nuclease solution containing 0.3 M sodium chloride, 50 mM sodium acetate (pH 4.5), 1 mM zinc sulfate and 100-200 units of S1 nuclease, and the reaction was terminated by adding 1/10 volume of each of 0.12 M EDTA and 1 M Tris·HCl solution (pH 9.0). Then, phenol extraction was performed, and the vector DNA having blunt terminals was precipitated by ethanol and collected. On the other hand, the DNA of vector pKK 233-2 was digested with restriction enzyme PstI, and the digested DNA was purified by extraction with phenol and precipitation from ethanol. The terminals of 2 μg of the purified vector DNA which had been cleaved by restriction enzyme PstI were rendered blunt by the above-mentioned T4 DNA polymerase reaction. The thus obtained DNA fragments derived from clone BK 106 and clone BK 111 were each cleaved with restriction enzyme HindIII. 0.5 μg of each of the cleaved DNA fragments was mixed with 0.5 μg of a vector DNA having blunt terminals. The DNA fragments derived from clone BK 102 and clone BK 147 were each cleaved with restriction enzyme PstI. 0.5 μg of each of the cleaved DNA fragments was mixed with 0.5 μg of a vector DNA having terminals thereof rendered blunt. The volume of each of the mixtures was adjusted to 20 μl by adding 2 μl of 10 x ligation solution containing 500 mM Tris·HCl (pH 7.5), 100 mM magnesium chloride, 100 mM DTT and 10 mM ATP, 300-400 units of T4 DNA ligase and distilled water. The mixtures were incubated at 14 °C for 12-18 hours, thereby obtaining plasmids, which were respectively designated as pCE-06, pE-11, pB-02 and pS-09. Using each of these plasmid DNAs, E. coli strain JM 109 was transformed in substantially the same manner as described in Step 6 of Example 1 (Part I), thereby obtaining transformed E. coli. The transformed E. coli was cultured at 37 °C in LB medium (pH 7.5) containing 1 (w/v)% trypton, 0.5 (w/v)% yeast extract and 1 (w/v)% sodium chloride, and when it was in logarithmic growth phase, 1 mM IPTG (isopropyl-β-D-thiogalactopyranoside) was added to the medium. The culturing was further continued for 3 hours. Then, E. coli cells were collected by centrifugation (10,000 x g for 15 minutes), and the collected cells were lysed in 50 mM Tris·HCl (pH 8.0). The mixture was subjected to ultrasonic treatment (20 KHz, 600 W, 5 minutes), and centrifuged at 10,000 x g for 15 minutes to thereby obtain a supernatant fraction and a precipitate fraction. Each of the fractions was dissolved in a sample buffer containing of 20 (v/v)% glycerol, 0.1 M Tris·HCl (pH 6.8), 2 (w/v)% SDS, 2 (v/v)% 2-mercaptoethanol and 0.02 % BPB, heated at 100 °C for 3 minutes, and subjected to electrophoresis using 0.1 % SDS-7.5 % polyacrylamide gel to separate protein. After the electrophoresis, the protein was transferred to a nitrocellulose filter by trans blot cell (manufactured and sold by BIO·RAD Co., Ltd., U.S.A.). The filter was immersed in 3 % gelatin solution, and allowed to stand still for 60 minutes. The filter was incubated together with serum from a patient suffering from NANB hepatitis, which had been diluted 100-fold, for 2 to 3 hours at room temperature. The filter was washed with distilled water and then with TTBS solution containing 0.02 M Tris·HCl (pH 7.5), 0.5 M sodium chloride and 0.05 (v/v)% Tween 20. Subsequently, the washed filter was immersed in a 2,000 fold-diluted solution of peroxidase-labeled anti-human IgG antibody, and incubated at room temperature for 90 minutes. The filter was washed with distilled water and then with TTBS solution. The washed filter was immersed in a buffer having, added thereto, coloring agent DAB and 30 %, based on substrate, hydrogen peroxide as described in Step 7 of Example 1 (Part I) for 5 to 30 minutes, following by washing with water, to terminate the reaction.

As a result, as shown in Table 2, all of the antigens produced by the plasmids specifically react with serum from a patient suffering from NANB hepatitis, thereby demonstrating that the proteins produced by the cDNAs inserted in the plasmids are clinically important.

## Table 2

### Reactivity evaluated by the Western blot method between proteins produced by various plasmids and sera from a patient suffering from NANB hepatitis

| Plasmid | origin of cDNA | Extract | Serum from NANB hepatitis patient | Serum from healthy human |
|---|---|---|---|---|
| pCE-066 | BK 106 | S | ± | - |
|  |  | P | + | - |
| pE-11-89 | BK 111 | S | ± | - |
|  |  | P | + | - |
| pB-02-10 | BK 102 | S | + | - |
|  |  | P | - | - |
| ps-09-07 | BK 109 | S | ± | - |
|  |  | P | + | - |
| pKK233-3 | - | S | - | - |
|  |  | P | - | - |

S: Supernatant by centrifugation

P: Precipitate by centrifugation

+: positive

±: slightly positive

-: negative

Reference Example 2

(Purification of NANBV-related antigens produced by E. coli and reactivity thereof with serum from a patient suffering from hepatitis)

The usefulness of the protein produced by the cDNA which was inserted into an expression vector was demonstrated by purifying the protein and using the purified protein as an antigen for ELISA or radioimmunoassay. That is, the lysate of the transformed E. coli which was obtained in Reference Example 1 was subjected to centrifugation at 10,000 x g for 15 minutes, thereby obtaining a supernatant and a precipitate. For example, the precipitate obtained from transformant JM 109/pCE 066 was suspended in a solution of 100 mM Tris·HCl (pH 8.0) and 0.1 % Triton X-100, and the resultant suspension was subjected to ultrasonic treatment at a frequency of 20 KHz (600 W) for one minute, followed by centrifugation at 21,000 x g for 15 minutes, thereby obtaining a precipitate. The precipitate was re-suspended in a solution of 100 mM Tris·HCl (pH 8.0) and 6 M urea, and then subjected to ultrasonic treatment followed by centrifugation.

The resultant supernatant was dialyzed against a solution of 10 mM phosphate buffer (pH 7.5) and 6 M urea to thereby obtain an antigen solution. 20 ml of the antigen solution was passed through a column (21.5 x 250 mm) packed with hydroxyapatite, which had been equilibrated with the above-mentioned buffer, to cause the antigen to be adsorbed onto the packing material. The column was subjected to high speed liquid chromatography (HPLC) wherein elution was performed with the above-mentioned buffer having, added thereto, sodium chloride, the concentration of which was varied from 0 to 2 M with a linear concentration gradient, thereby obtaining a fraction containing an antigen. The obtained fraction was dialyzed against 50 mM carbonate buffer (pH 9.6) containing 0.05 % sodium dodecyl sulfate (SDS).

Further, the supernatant obtained by centrifugation (at 10,000 g for 15 minutes) of the lysate of transformant JM 109/pB-02-10 was treated with 35 % saturated ammonium sulfate, and the obtained precipitate was dissolved in 50 mM Tris·HCl (pH 8.5) buffer containing 100 mM 2-mercaptoethanol. The resultant solution was dialyzed against the above-mentioned buffer. Subsequently, 100 ml of the dialysed solution was passed through a column (22.0 x 200 mm) packed with DEAE cellulose, which had been equilibrated with the above-mentioned buffer, to cause the antigen to be adsorbed onto the packing material. The column was subjected to high performance liquid chromatography wherein elution was performed with 50 mM Tris·HCl (pH 8.5) buffer containing 100 mM 2-mercaptoethanol having, added thereto, sodium chloride, the concentration of which was varied from 0 to 2 M with a linear concentration gradient, thereby pooling a fraction containing the antigen.

The fraction was dialyzed against a solution of 10 mM phosphate buffer (pH 6.8) and 100 mM 2-mercaptoethanol. The dialyzed solution was passed through the column of hydroxyapatite for high performance liquid chromatography, which had been equilibrated by the above-mentioned buffer, to cause the antigen to be adsorbed onto the packing material. The column was subjected to high performance liquid chromatography wherein elution was performed with phosphoric acid, the concentration of which was varied with a linear concentration gradient from 10 to 400 mM, thereby pooling a fraction containing the antigen. The resultant fraction was dialyzed against 50 mM carbonate buffer (pH 9.6) containing 0.05 % SDS.

The precipitate obtained by centrifugation of the lysate of transformant JM 109/pE-11-89 was suspended in 10 mM phosphate buffer (pH 5.5). The suspension was subjected to the above-mentioned ultrasonic treatment for one minute, and then subjected to centrifugation at 21,000 x g for 15 minutes. The resultant precipitate was suspended in 100 mM carbonate buffer (pH 10.5) containing 500 mM sodium chloride and 10 mM EDTA. The resultant suspension was again subjected to the ultrasonic treatment for one minute, followed by centrifugation. The resultant supernatant was dialyzed against 30 mM phosphate buffer containing 6 M urea. Subsequently, 20 ml of the dialyzed solution was passed through a CM cellulose column (22 x 200 mm) for high performance liquid chromatography (HPLC), which had been equilibrated with the same buffer as used for the above-mentioned dialysis, to thereby cause the antigen to be adsorbed onto the packing material. The column was subjected to high performance liquid chromatography wherein elution was performed with the above-mentioned buffer having, added thereto, sodium chloride, the concentration of which was varied from 0 to 1.5 M with a linear concentration gradient, obtaining a fraction containing the antigen. The fraction was dialyzed against 50 mM carbonate buffer (pH 9.6) containing 0.05 % SDS, thereby obtaining a solution containing the antigen.

The antigens prepared above were used as an antigen for ELISA for the clinical diagnosis of infection with non-A, non-B hepatitis virus. That is, the protein concentration of each of the above-mentioned purified antigens was adjusted to 1 μg/ml, and put in each well of Microplate Immulone 600 (manufactured and sold by Greiner, Co., Ltd., Germany) in an amount of 100 μl for use in ELISA, which well was allowed to stand still at 4 °C overnight. The contents of the individual wells were washed well three times with PBS-T buffer containing 10 mM phosphate buffer (pH 7.2), 0.8 % sodium chloride and 0.05 % Tween 20, and sample serum diluted with the PBS-T buffer was added in an amount of 100 μl/well, followed by reaction at 37 °C for one hour. The contents of the individual wells were washed three times with the PBS-T buffer, and a peroxidase-labeled anti-human IgG antibody (manufactured and sold by Cappel Co., Ltd., Germany) which had been diluted 8000-fold with PBS-T buffer containing 10 % fetal calf serum was added in an amount of 100 μl/well. The individual well contents were reacted at 37 °C for one hour, and washed with the PBS-T buffer four times. A substrate coloring agent solution composed of 9 ml of 0.05 M citric acid-phosphate buffer and, contained therein, 0.5 μg of o-phenylenediamine and 20 μl of aqueous hydrogen peroxide, was added in an amount of 100 μl/well. The plate was light shielded, and allowed to stand still at room temperature for 60 minutes. 75 μl of 4 N sulfuric acid was added to each of the wells, and the absorbance at 490 nm was determined. The results are shown in Table 3. As apparent from the table, all of the antigens derived from the transformants specifically react with the serum from NANB hepatitis patient, thereby attesting to the usefulness in clinical diagnosis of the antigens produced by the transofrmants.

## Table 3

Reactivity in ELISA between the purified antigens

from various transformed Escherichia coli and

the serum from NANB hepatitis patient

Serum from blood transfused
patient of hepatitis

| origin of antigen (transformed Escherichia coli) | acute | chronic | hepato-cirrhosis | hepatoma | healthy human serum |
|---|---|---|---|---|---|
| JM109/pCE-066 | 2/3* | 7/8 | 3/4 | 3/3 | 0/10 |
| JM109/pB-02-10 | 2/3 | 8/8 | 4/4 | 3/3 | 0/10 |
| JM109/pE-11-89 | 2/3 | 8/8 | 2/4 | 3/3 | 0/10 |

*: the number of positive samples/the number of

samples examined

The same results as shown in Table 3 were also obtained by radioimmunoassay using the above-mentioned antigens. That is, a polystyrene bead of 1/4 inch in diameter (manufactured and sold by Pesel Co., Ltd., Germany) was put in 0.2 ml of each of the above-mentioned purified antigen solutions of 1 $\mu$g/ml in concentration, and allowed to stand still at 4 °C overnight. Then, the polystyrene bead was washed five times with the same PBS-T buffer as used in the above-mentioned ELISA, and a sample serum diluted 20 to 2500-fold with the PBS-T buffer was added in an amount of 200 $\mu$l/bead. Reaction was performed at 37 °C for 60 min. The polystyrene bead was washed five times with the PBS-T buffer, and $^{125}$I-labeled anti-human IgG antibody was added in an amount of 200 $\mu$l/bead. Reaction was performed at 37 °C for one hour and the bead was washed five times with the PBS-T buffer. The cpm of $^{125}$I bound to the polystyrene bead was measured, thereby obtaining the same results as shown in Table 3. Thus, the usefulness of the purified antigens obtained above in the clinical diagnosis of infection with NANB hepatitis virus, was demonstrated.

Reference Example 3

[Detection of NANBV nucleic acid according to PCR (Polymerase Chain Reaction) method]

For preventing NANB hepatitis caused by blood transfusion, itis important to determine whether or not any NANBV infection exists in the blood supplied for transfusion. Further, for diagnosing hepatitis, it is extremely clinically important to study whether or not any NANBV infection exists in liver tissue. The NANBV cDNA obtained according to the present invention can be advantageously used for producing a primer for polymerase chain reaction (PCR) useful for detecting NANB hepatitis. That is, as described in Step 1 of Example 1 (Part I), the purification of RNA was performed from 1 ml of each of sera derived from a patient and a healthy human. Likewise, RNA was prepared from liver cells as described in Step 2 of Example 1 (Part I). Subsequently, as described in Step 4 of Example 1 (Part I), PCR and electrophoresis were conducted to thereby prepare cDNAs. According to the customary procedure, whether or not the amplified cDNA was derived from NANBV, was investigated by Southern hybridization using $^{32}$P-labeled probe prepared from the cDNA derived from NANBV cDNA clone BK 108.

The results are shown in Table 4. From the table, it is apparent that the NANBV nucleic acid in serum can be detected and the serum infection with NANBV can be diagnosed by the use of the primer prepared from the nucleotide sequence of the NANBV cDNA obtained according to the present invention and the fragment of cloned NANBV cDNA as a probe.

## Table 4

### Detection of NANBV nucleic acid by PCR

| sample | | | antibody against NANBV | PCR |
|---|---|---|---|---|
| serum from chronic hepatitis patient | | | | |
| NANB | 1 | | + | + |
| | 2 | | + | + |
| HBV carrier | 1 | | – | – |
| | 2 | | – | – |
| healthy human | 1 | | – | – |
| | 2 | | – | – |
| excised liver from NANB hepatoma-1 | | | + | |
| | cancerous site | | | + |
| | non-cancerous site | | | + |
| excised liver from NANB hepatoma-2 | | | + | |
| | cancerous site | | | + |
| | non-cancerous site | | | + |

Example 1 (Part II)

step 1 (Construction of the plasmids for the expression of the entire coding region of the NANBV genomic cDNA in E. coli)

cDNA was isolated from each of clones BK112, BK146, BK147, BK157 and BK166 shown in Fig. 1 (1) and Fig. 1 (2), and plasmids for the expression of the entire coding region of the NANBV gene in E. coli were prepared as follows.

The plasmid DNA of clone BK157 was digested with restriction enzyme BamHI and subjected to agarose gel electrophoresis to thereby obtain a DNA fragment of 1.3 kb in length. The DNA fragment was inserted in plasmid pUC19 (manufactured and sold by Takara Shuzo Co., Ltd., Japan) at its BamHI site to thereby obtain plasmid pBam157. The plasmid pBam157 was digested with restriction enzymes XbaI and NcoI to thereby obtain a DNA fragment of about 3.9 kb in length. Separately, an oligonucleotide of 93bp (having 4 nucleotides deleted from the 3′-terminus side) was synthesized by ligating the sequence of the promoter region of 20bp (TAATAC-GACTCACTATAGGG) of bacteriophage T7 RNA polymerase having, attached thereto, XbaI linker sequence to the sequence of nucleotide numbers 1 to 73 shown in Fig. 2(1) having, attached thereto, NcoI linker sequence. The thus obtained oligonucleotide was ligated to the above-mentioned DNA fragment of 3.9 kb, thereby obtaining plasmid pDM-16. Then, pDM-16 was digested with restriction enzymes ClaI and EcoRI to obtain a DNA fragment of about 3.5 kb. Separately, the DNA of clone BK146 was digested with restriction enzymes ClaI and EcoRI to obtain a DNA fragment of 4.1 kb in length. The above-mentioned DNA fragment of about 3.5 kb was ligated to the thus obtained DNA fragment of 4.1 kb, to thereby obtain plasmid pDM-9. Then, plasmid pDM-9 was digested with SacII, thereby obtaining a DNA fragment of 2.7 kb and a DNA fragment of 4.9 kb. The DNA fragment of 4.9 kb was ligated at its SacII site with T4 DNA ligase and then digested with BamHI and EcoRI, thereby obtaining a DNA fragment of about 7.5 kb. Separately, the DNA of clone BK147 was digested with BamHI and EcoRI, thereby obtaining a DNA fragment of about 2 kb. The thus obtained DNA fragment was ligated to the above-mentioned DNA fragment of 7.5 kb to thereby obtain plasmid pBE147. Plasmid pBE147 was digested with SacII. The above-mentioned DNA fragment of 2.7 kb derived from pDM-9 was inserted into the SacII-digested pBE147, thereby obtaining plasmid pDM-B3. Plasmid pDM-B3 was digested with XbaI and EcoRI to thereby obtain a DNA fragment of 6.7 kb.

The DNA of clone BK166 was digested with BamHI to obtain a DNA fragment of 1.3 kb. This fragment was inserted in pUC19 at its BamHI site to obtain pBam166. pBam166 was digested with NdeI and HindIII to thereby obtain a DNA fragment of 2.8 kb. The DNA of clone BK112 was digested with EcoRI and NdeI to obtain a DNA fragment of about 1.6 kb. pUC19 was digested with EcoRI and HindIII to obtain a DNA fragment of about 2.6 kb. The above-obtained three types of DNA fragments were mixed and reacted with T4 DNA ligase to thereby obtain plasmid pEN112 in which these fragments were ligated together at their EcoRI site, NdeI site and HindIII site. Plasmid pEN112 was digested with EcoRI and XbaI to obtain a DNA fragment of 2.7 kb. pDM-B3 was digested with EcoRI and XbaI to obtain a fragment of 6.7 kb. The above-mentioned fragment of 2.7 kb was ligated to the fragment of 6.7 kb, and the resultant ligated DNA fragment was inserted in pUC19 at its XbaI site, thereby obtaining plasmids pDM-22 and pDM-18. Plasmid pDM-18 can be used for the transformation of an animal cell or the like so that the cell can produce an NANBV particle. The transformation can also be performed using an RNA prepared by transcripting pDM-18 by means of in vitro Transcription Kit (manufactured and sold by Boehringer Mannheim Yamanouchi, Japan). Plasmid pDM-22, in which the cDNA was inserted in an orientation opposite to that of pDM-18, was digested with HindIII and ClaI to obtain a DNA fragment of about 9 kb.

The DNA of clone BK106 was digested with BamHI to obtain a DNA fragment of about 1.0 kb. This fragment was inserted in plasmid pUC19 at its BamHI site to obtain plasmid pBam106. The thus obtained plasmid DNA was digested with NcoI, and the sticky terminus was rendered blunt with Mang Bean nuclease (manufactured and sold by Takara Shuzo Co., Ltd., Japan). The resultant plasmid was further digested with XbaI, thereby obtaining a fragment of about 3.6 kb. A synthetic oligonucleotide prepared by ligating the sequence of nucleotide numbers 333 to 372 shown in Fig. 2(1) to the downstream of XbaI linker, was ligated to this fragment to thereby obtain plasmid pXb106. Plasmid pXb106 was digested with HindIII and ClaI to obtain a DNA fragment of 0.4 kb. This fragment was ligated to the above-mentioned fragment of about 9 kb derived from plasmid pDM-22 to thereby obtain plasmid pORF-24. Plasmid pORF-24 was digested with XbaI to obtain a DNA fragment of about 9.0 kb. This fragment was ligated to an expression vector (see F. William Studier and B.A.Moffatt, J. Mol. Biol., 189, 113, 1986) having, ligated thereto, T7 RNA polymerase gene promotor, thereby obtaining expression plasmid pJF-22.

Step 2 (Preparation of transformant E. coli and culturing thereof)

Using expression plasmid pJF-22 constructed in Step 1, Escherichia coli strain JM109 (DE3) (manufactured and sold by Promega Co., U.S.A.) was transformed by the calcium chloride method ( Journal of Molecular Biology, 53, 154, 1970), thereby obtaining trans formant JM109 (DE3) /pJF-22.

Transformant E. coli JM109 (DE3)/pJF-22 was subjected to the subsequent procedure as described in Reference Example 1. That is, the E. coli was cultured on LB culture medium, and then 0.5 mM IPTG was added thereto, followed by further culturing for 3 hours. After that period, the cultured cells were collected and heated in a buffer containing 2 % SDS and 2 % 2-mercaptoethanol at 100 °C for 3 minutes. The resultant cells were subjected to electrophoresis using a gel containing 0.1 (w/v)% SDS and 12.5 (w/v)% acrylamide. The resulant protein isolated on the gel was blotted onto a nitrocellulose membrane by means of a trans blot apparatus (manufactured and sold by Nippon Eido Co., Ltd., Japan) and subjected to Western blotting analysis to identify the obtained protein. In the Western blotting analysis, the specific antisera was used which was obtained by purifying the NANBV-related antigen prepared from the transformant obtained in Reference Example 1 and immunizing guinea pigs therewith. As a result of the Western blotting analysis, it was found that the protein produced by transformant JM109 (DE3)/pJF-22 reacted with all of the antisera (see Table 5).

## Table 5

### Reactivity of protein produced in transformant

### E. coli JM109 (DE3)/pJF-22 with NANBV-related

antibody

| cell extract | serum from NANB hepatitis patient | | | guinea pig antiserum | | |
|---|---|---|---|---|---|---|
| | pooled serum | acute | chronic | anti-core | anti-NS3 | anti-NS5 |
| JM109(DE3)/ pJF22 | +* | + | + | + | + | + |
| JM109(DE3) | - | - | - | - | - | - |

*: Reactivity as measured by Western blotting analysis.

Thus, it was demonstrated that in this transformant, the expression was attained of the entire coding region of the NANBV gene from the 5'-end of the genome coding for the core antigen through the 3'-end of the genome coding for the non-structural protein NS5. From the results, it is apparent that this transformant can provide an antigen which is extremely useful for producing not only a diagnostic reagent for NANBV infection but also a vaccine for NANBV.

Step 3 (Production of NANBV particles by expression of NANBV genomic cDNA in animal cells)

Plasmid pORF-24 obtained in Step 1 was partially digested with XbaI and subjected to low melting point agarose gel electrophoresis to obtain a DNA fragment of about 9 kb in length. The thus obtained DNA fragment was inserted into plasmid pMAM-neo (available from Clontech, U.S.A.) which had been cleaved with NheI, to thereby construct expression plasmid pMAM-neo10. The expression plasmid was transfected into cells of human hepatocyte Chang Liver (ATCC CCL 13) and Chimpanzee hepatocyte (purchased from Dainippon Pharmaceutical Company, Ltd., Japan) by a calcium phosphate method ("Molecular Cloning", 16.33-16.39, Cold Spring Harbor Laboratory, 1989). The hepatocyte cells having plasmid pMAM-neo10 introduced thereto were rendered resistant to aminoglycoside antibiotic G418 so that the cells were able to form colonies in the presence of G418 in an amount of 600 μg/ml. Utilizing this resistance as a criterion, transformants were selected, followed by cloning. Transformant clones HL-A1 and HL-A2 produced from human hepatocyte and transformant clones CL-B11 and CL-B14 produced from chimpanzee hepatocyte were individually cultured in Eagle's MEM medium having, incorporated therein, 5 (v/v)% fetal calf serum, at 37 °C for 4 days on a cover glass placed in a Petri dish, in the same manner as in Reference Example 4 which will be described later. With respect to the protein produced by the cell culture of each of the above-obtained clones, determination of NANBV antigenicity was conducted by indirect fluorescent antibody technique using specific antisera described in Step 2. As a result, it was found that the protein produced in the G418 resistant cell clone reacted with all of the antisera of guinea pigs immunized with the NANBV-related antigens obtained in Reference Example 1 (see Table 6).

Table 6

Detection by fluorescent antibody technique of
NANBV-related antigens produced in transformant
human or chimpanzee hepatocyte

| cell<br>extract | serum from NANB<br>hepatitis patient | | | guinea pig antiserum | | |
| | pooled<br>serum | acute | chronic | anti-<br>core | anti-<br>NS3 | anti-<br>NS5 |
| --- | --- | --- | --- | --- | --- | --- |
| human hepatocyte-derived | | | | | | |
| HL-A1 | + | + | + | + | + | + |
| HL-A2 | + | + | + | + | + | + |
| normal<br>Chang Liver | – | – | – | – | – | – |
| chimpanzee hepatocyte-derived | | | | | | |
| CL-B11 | + | + | + | + | + | + |
| CL-B14 | + | + | + | + | + | + |
| normal<br>Chimp Liver | – | – | – | – | – | – |

This fact means that the entire coding region of NANBV gene covering the region coding for the core antigen through the region coding for NS5 was expressed.

Step 4 (Sucrose density-gradient centrifugation of NANBV-related antigens produced by human hepatocyte- and chimpanzee hepatocyte-derived transformant cell clones HL-A1 and CL-B11)

Clones HL-A1 and CL-B11 were individually cultured at 37 °C for 4 days on 5 Petri dishes having a diameter of 9 cm in a CO$_2$ incubator, in the same manner as in Step 3. The cells were scraped off with a rubber policeman and pooled together with the culture liquid and subjected to ultrasonic treatment at 20 kHz (200 W) for 2 minutes and centrifugation at 5000 x g and at 4 °C for 15 minutes. The resultant supernatant was further subjected to centrifugation at 48000 x g and at 4 °C for 14 hours to obtain a precipitate. The precipitate was suspended in 1 ml of M/75 PBS and subjected to ultrasonic treatment for 2 minutes and sucrose density-gradient centrifugation at 160000 x g and at 4 °C for 15 hours, followed by fractionation. Each fraction was subjected to SDS-polyacrylamide electrophoresis and Western blotting analysis in the same manner as in Step 2, thereby conducting the detection of a core antigen and an envelope antigen. As a result, both antigens were detected at a sucrose density of 40 (w/v)% to 50 (w/v)%, indicating that there were obtained NANBV particles.

Example 2

Step 1 (Construction of a plasmid for the expression in yeast of the entire coding region of the NANBV genomic cDNA and preparation of a transformant yeast)

Plasmid pORF24 obtained in Step 1 of Example 1 (Part 11) was digested with XbaI to thereby obtain an NANBV cDNA fragment of about 9 kb in the same manner as in Step 1 of Example 1 (Part II). 0.5 μg of this cDNA fragment was dissolved in a T4 DNA polymerase solution containing 67 mM Tris·HCl (pH 8.8), 6.7 mM magnesium chloride, 16.6 mM ammonium sulfate, 10 mM 2-ME, 6.7 mM EDTA-2Na 0.02 (w/v)% bovine albumine and 0.3 mM dNTP, and 2 to 5 units of T4 DNA polymerase (Takara Shuzo Co., Ltd., Japan) was added thereto and the resultant mixture was incubated at 37 °C for 60 minutes, thereby rendering blunt the both terminals of the fragment. Then, XhoI linker (CCTCGAGG) was ligated thereto by means of T4 DNA ligase. Illustratively stated, 0.3 μg of the DNA was dissolved in 21 μl of a 10 x ligation solution containing 500 mM Tris·HCl (pH 7.5), 100 mM magnesium chloride, 100 mM DTT and 10 mM ATP. Added to the resultant mixture were 300 to 400 units of T4 DNA ligase (Takara Shuzo Co., Ltd., Japan) and distilled water in an amount such that the total volume became 210 μl, followed by incubation at 14 °C for 18 hours. The thus prepared cDNA fragment was inserted in an expression vector for use in yeast, namely, YEp133PCT (described in U.S. Patent No. 4,810,492) at its XhoI site to thereby obtain expression plasmid pYHC5. With this expression plasmid pYHC5, yeast S. cerevisiae (ATCC No. 44772) was transformed by the alkali cation method (Ito, H. et al, J. Bacteriol., 153:163-168, 1983), to thereby obtain transformant yeast YHC5-1. This transformant was designed so that when the culture medium was lack of phosphate ions, the gene for repressive acid phosphatase was activated to cause the transcription of the NANBV cDNA ligated downstream thereof, thereby producing NANBV-related antigens.

Step 2 (production of NANBV-related antigens by yeast and characterization thereof)

Transformant yeast YHC5-1 obtained in Step 1 was inoculated into 100 ml of a culture medium prepared by adding 20 μg/ml of each of uracil, L-tryptophan and L-histidine into Burkholder's medium (see Burkholder, P.R. et al, Am.J. Botany, 30, 206-211, 1943) that was a totally synthesized medium containing 1.5 g/l of potassium phosphate monobasic. The inoculated medium was cultured at 30 °C for 24 hours while shaking. The cultured yeast was washed with physiological saline and inoculated into 1000 ml of a fresh medium of the same type as described above except that 1.5 g/l of potassium chloride was contained instead of potassium phosphate monobasic. The inoculated medium was cultured at 30 °C for 24 hours and the resultant cells were collected. The collected cells were suspended in M/75PBS and glass beads (diameter: 0.45-0.55 mm) were added thereto and the suspension was subjected to a Bead Beater (manufactured and sold by Biospec Products, U.S.A.) to thereby disrupt the cells, and then to centrifugation at 10000 x g and at 4 °C for 10 minutes, thereby obtaining a supernatant. The thus obtained supernatant was subjected to SDS-polyacrylamide gel electrophoresis and Western blotting analysis in the same manner as in Step 2 of Example 1 (Part II), thereby examining whether or not NANBV-related antigens had been produced. As a result, it was found that the extract of transformant yeast YHC5-1 reacted with all of the antibodies respectively specific for the core antigen, envelope antigen, NS3 protein and NS5 protein. This fact means that the entire cording region of NANBV gene from the core antigen region through the NS5 region was expressed (see Table 7).

## Table 7

### Reactivity of proteins produced by transformant yeast YHC5-1 with NANBV-related antibodies

| cell extract | serum from NANB hepatitis patient | | | guinea pig antiserum | | |
|---|---|---|---|---|---|---|
| | pooled serum | acute | chronic | anti-core | anti-NS3 | anti-NS5 |
| YHC5-1 | +* | + | + | + | + | + |
| normal S.cerevisiae | – | – | – | – | – | – |

\* Reactivity as measured by Western blotting analysis

Further, the cell extract was subjected to sucrose density-gradient centrifugation in the same manner as in Step 4 of Example 1 (Part II). As a result, both of the core antigen and the envelope antigen were detected in a fraction at a sucrose density of 40 (w/v)% to 50 (w/v)%, indicating that there were obtained NANBV particles.

Example 3

Step 1 (Construction of a plasmid for introduction into vaccinia virus)

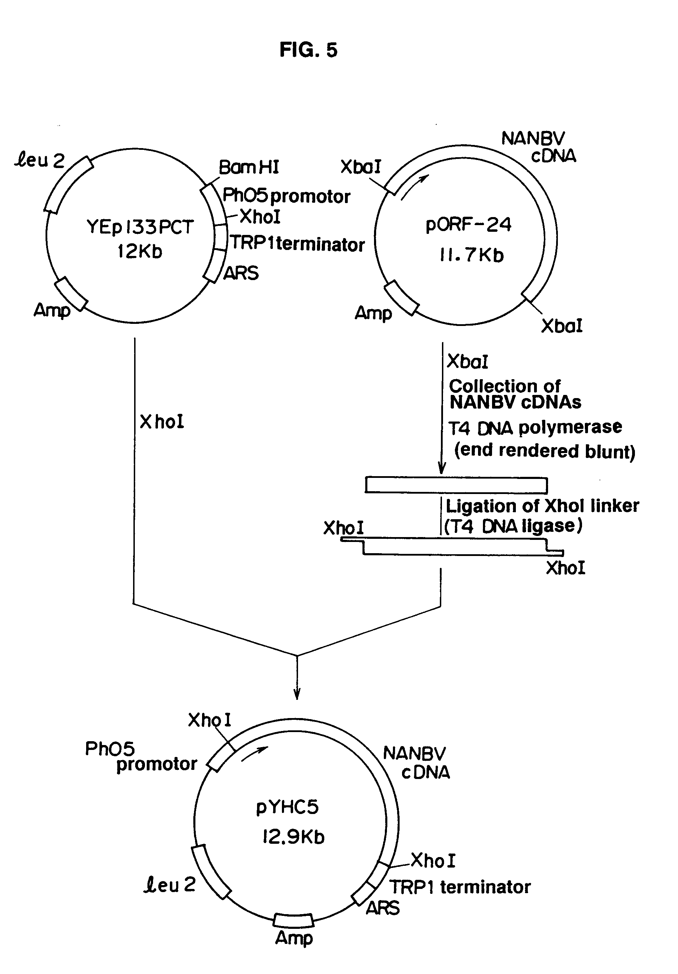

Plasmid pUV1 (Falko G. Falkner, Sekhar Chakrabarti and Bernard Moss; Nucleic Acid Res., 15 (17), 7192, 1987) was digested with restriction enzyme EcoRI and subjected to phenol extraction and ethanol precipitation, thereby obtaining a DNA. In the same manner as in Reference Example 1, 0.5 µg of this DNA was dissolved in a T4 DNA polymerase solution and 2 to 5 units of T4 DNA polymerase (manufactured and sold by Takara Shuzo Co., Ltd., Japan) was added, followed by incubation at 37 °C for 60 minutes, to thereby render blunt both terminals. Separately, a DNA fragment carrying the entire nucleotide sequence of NANBV gene coding for an NANBV protein was obtained by a method in which plasmid pORF-24 described in Step 1 of Example 1 (Part II) was digested with XbaI or with XbaI and EcoRI to thereby obtain a DNA fragment of about 9 kb or a DNA fragment of 6.4 kb and the obtained DNA fragment was then treated with T4 DNA polymerase, thereby rendering both terminals blunt. 0.5 µg of the thus obtained DNA derived from pUV1 and 0.5 µg of the thus obtained DNA derived from pORF-24 were dissolved in 21 µl of 10 x ligation solution in the same manner as in Reference Example 1 and added thereto were 300 to 400 units of T4 DNA ligase (manufactured and sold by Takara Shuzo Ltd., Japan) and distilled water in an amount such that the total volume became 210 µl, followed by incubation at 14 °C for 12 to 18 hours. Thus, a cDNA derived from NANBV was ligated to pUV1 at its EcoRI site located downstream of the promoter. The ligation reaction mixture was subjected to phenol extraction and the aqueous layer was subjected to ethanol precipitation to collect a DNA. With the DNA, E. coli strain JM109 was transformed in accordance with the calcium chloride method, as described in Step 6 of Example 1 (Part I), thereby obtaining plasmid clones pXX-49 and pXX-51 each having an NANBV cDNA fragment of about 9 kb. In addition, plasmid pXE-39 having an NANBV cDNA fragment of about 6.4 kb and lacking the NS5 region of NANBV. The results are shown in Fig. 5.

Reference Example 4

(Culturing of vaccinia virus WR strain)
Vaccinia virus WR strain was cultured by the customary method. Illustratively stated, monolayer-cultured cells [such as mouse-derived thymidine kinase (TK)-defective cell line L-M(TK⁻) (ATCC CCL-1.3, Dainippon Pharmaceutical Co., Ltd., Japan), simian kidney-derived Vero cells and adult human hepatocyte Chang Liver (ATCC CCL-13, Dainippon Pharmaceutical Co., Ltd., Japan)] were cultured in a Petri dish having a diameter of 6 cm. The resultant cells were inoculated with 0.5 ml of vaccinia virus and allowed to stand at 37 °C for 1 to 2 hours, followed by removing the virus liquid. Then, 5 ml of Eagle's MEM medium (manufactured and sold by Nissui Pharmaceutical Co., Ltd., Japan) having, added thereto, 5 (v/v)% fatal calf serum and the cells were cultured at 37 °C for 24 to 48 hours until satisfactory cytopathiceffect was observed. Then, the virus culture liquid or the infected cells were collected and suspended in MEM and subjected to ultrasonic treatment at 20 kHz (200W) for 2 minutes, thereby obtaining a virus liquid.

Refernce Example 5

(Infectivity assay for vaccinia virus by plaque method)

The plaque method was conducted by the customary procedure. Illustratively stated, the cell culture described in Reference Example 4 in a 6 cm-diameter Petri dish is inoculated with the virus liquid obtained in Reference Example 4, which had been diluted 10-fold with M-199 (manufactured and sold by Sigma, U.S.A.), in an amount of 0.1 ml/dish and allowed to stand at 37 °C for 2 hours, thereby adsorbing the virus onto the cells. Then, the inoculum liquid was removed and an agar-containing medium (prepared by adding 3 (v/v)% fetal calf serum, 0.14 (w/v)% $NaHCO_3$ and 0.8 (w/v)% agar to M-199) was added in an amount of 5 ml/dish. After the agar had solidified at room temperature, culturing was conducted at 37 °C for 24 hours. Then a medium prepared by adding neutral red (manufactured and sold by Wako Pure Chemical Industries, Ltd., Japan) to the above-mentioned agar in an amount of about 0.006 (w/v)% was overlaid in an amount of 2.5 ml/dish, followed by further culturing at 37 °C. The number of the resultant plaques was counted, to thereby determine the infectivity.

Step 2 (Preparation of recombinant vaccinia virus)

Vero cells were cultured for 24 hours in a 9 cm-diameter Petri dish to be used for tissue culture (manufactured and sold by Falcon, U.S.A.). The cultured Vero cells were inoculated with vaccinia virus WR strain (ATCC VR-119) at an MOI (multiplicity of infection) of 0.05 and allowed to stand at 37 °C for 2 hours so as to adsorb the virus onto the cells. After that period, the virus liquid was removed and the cells were washed with MEM twice. Then, 1 μg and 5 μg of the plasmid DNAs obtained in Step 1 of Example 3 were individually subjected to calcium phosphate precipitation of DNA in accordance with the calcium phosphate method (Graham, F.L., van der Eb, A.J.: Virology, 52, 456-467, 1973), thereby obtaining 1 ml of a DNA-calcium phosphate precipitation solution with respect to each plasmid DNA. The thus obtained 1 ml each of DNA-calcium phosphate precipitate solution was added to the above-obtained virus-infected cells and allowed to stand at room temperature for 30 minutes. Then, 15 ml of the virus culture medium as described in Reference Example 4 was added and the resultant mixture was incubated at 37 °C for 3.5 hours. Then the virus culture medium was removed and 15 ml of a fresh virus culture medium was added, followed by culturing at 37 °C for 48 hours. Then the cell culture was subjected to freezing and thawing 3 times, to thereby obtain a virus suspension. The virus suspension was inoculated into L-M(TK⁻) cells cultured in a 6 cm-diameter Petri dish to be used for culturing, in the same manner as in Reference Example 5. After the virus had been adsorbed onto the cells, an agar medium containing 25 μg/ml of 5-Bromo-2'-deoxyuridine (BUdR, manufactured and sold by Sigma, U.S.A.) was added in an amount of 5 ml/dish, followed by culturing at 37 °C for 8 hours. Then, an agar medium containing 25 μg/ml of BUdR and 25 μg/ml of 5-Bromo-4-Chloro-3-Indolyl-β-D-Galactoside (X-Gal, Takara Shuzo Co., Ltd., Japan) was overlaid in an amount 2.5 ml/dish and the cells were further cultured at 37 °C for 2 days. The blue plaques that appeared were collected together with the overlaying agar medium and suspended in 0.5 ml of M-199 and the supernatant was inoculated into L-M(TK⁻) cells in the same manner as described above, followed by plaque cloning 3 times to thereby purify the clones. The thus obtained vaccinia virus was a recombinant vaccinia virus transformed by recombination with a plasmid vector DNA having an NANBV cDNA, and had the β-galactosidase gene and lacked thymidine kinase. The results are shown in Table 8.

## Table 8

### Characteristics of recombinant vaccinia virus

| recombinant vaccinia virus clone | plasmid employed for re-combination | NANBV genome (kb) | thymidine kinase activity | β-galacto-sidase activity |
|---|---|---|---|---|
| vUV17 | pUV1 | X | X | O |
| vUV27 | pUV1 | X | X | O |
| VXE17 | pXD39 | O(6.4) | X | O |
| VXE28 | pXE39 | O(6.4) | X | O |
| vXX19 | pXX49 | O(9.0) | X | O |
| vXX29 | pXX51 | O(9.0) | X | O |
| vXX39 | pXX51 | O(9.0) | X | O |

Note)  O :  Observed

X :  Not observed

Step 3 (Detection and confirmation by fluorescent antibody technique of production of NANBV-related antigens by recombinant vaccinia virus)

Antigens produced by recombinant vaccinia virus were detected and confirmed by indirect fluorescent antibody technique. L-M(TK⁻) cells were cultured on a cover glass and inoculated with recombinant vaccinia virus which had been diluted 10-fold, and the cells were cultured by the method described in Reference Example 4. After culturing for 48 hours, the cover glass was taken out and washed with M/75 phosphate buffer saline (M/75 PBS) (pH 7.4) three times and then with distilled water one time, followed by air-drying. Then, the cells were fixed by acetone at -20 °C for 5 minutes. An anti-NANBV mouse monoclonal antibody to be used as a primary antibody was obtained from a hybridoma obtained by fusing a mouse myeloma cell with a lymphocyte separated from BALD/C mouse immunized by the customary method using the core antigen and non-structural protein antigens NS-3 and NS-5. A monoclonal antibody specific for the envelope was prepared by using an antigen produced by binding a 16-mer oligopeptide to bovine serum albumin, which 16-mer oligopeptide was comprised of an amino acid sequence (1st to 16th amino acids on the N-terminal side) deduced from the envelope gene. Indirect fluorescent antibody technique was conducted by the customary method. That is, the infected cells fixed by acetone were reacted with the primary antibody at 37 °C for 1 hour and washed with M/75 PBS three times. Then, the cells were reacted with FITC (fluorescent dye)-labeled anti-human or mouse IgG antibody (manufactured and sold by Cappel Co., Ltd., Germany) at 37 °C for 1 hour and washed with M/75 PBS three times, followed by the observation by a fluorescence microscope. The results are shown in Table 9.

Table 9 (1)

Detection by fluorescent antibody technique of
NANBV-related antigens produced by cells infected
with recombinant vaccinia virus

| recombinant vaccinia virus | serum from healthy human | | | serum from NANB hepatitis patient | | | |
| | #6 | #8 | #9 | pooled serum #II-1 | pooled serum #PS-1 | acute | chronic |
|---|---|---|---|---|---|---|---|
| vUV17 | − | − | − | − | − | − | − |
| vUV27 | − | − | − | − | − | − | − |
| vXE17 | − | − | − | + | + | + | + |
| vXE28 | − | − | − | + | + | + | + |
| vXX19 | − | − | − | + | + | + | + |
| vXX29 | − | − | − | + | + | + | + |
| vXX39 | − | − | − | + | + | + | + |

(to be continued)

## Table 9 (2) (continued)

| recombinant vaccinia virus | monoclonal antibody | | | |
| --- | --- | --- | --- | --- |
| | anti-core #11 | anti-EnV #755 | anti-NS3 #74-1 | anti-NS5 #8905 |
| vUV17 | - | - | - | - |
| vUV27 | - | - | - | - |
| vXE17 | + | + | + | - |
| vXE28 | + | + | + | - |
| vXX19 | + | + | + | + |
| vXX29 | + | + | + | + |
| vXX39 | + | + | + | + |

Recombinant vaccinia virus clones vXE17 and vXE28, both of which lacked a portion of the nucleotide sequence coding for the NS5 region of NANBV, and clones vXX19, vXX29 and vXX39, all of which had a complete ORF coding for the protein of NANBV, reacted with all of the sera from NANB hepatitis patients but did not react with any of the sera from healthy humans. When a mouse monoclonal antibody was employed, all of clones vXE17 and vXE28 and clones vXX19, vXX29 and vXX39 reacted with the anti-core antigen monoclonal antibody, the anti-envelope antigen monoclonal antibody and the anti-NS3 monoclonal antibody. With respect to the anti-NS5 monoclonal antibody, vXE17 and vXE28, both of which lacked the NS5 region of NANBV, did not reacted therewith, but vXX19, vXX29 and vXX39 reacted therewith. These facts mean that the desired expression products were advantageously produced by means of recombinant vaccinia virus and that particularly by means of clones vXX19, vXX29 and vXX39, the entire region was expressed, from the core antigen on the N-terminal side of the NANBV protein through the NS5 protein on the C-terminal side thereof.

Step 4 (Analysis of supernatant of culture of cells infected with recombinant vaccinia virus by sucrose density-gradient centrifugation)

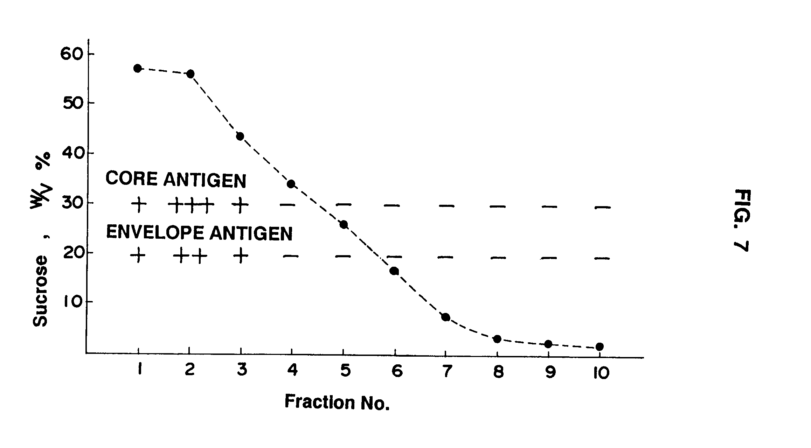

As described in Reference Example 4, 1.0 ml (1.2 x 10⁷ PFU) of recombinant vaccinia virus vXX39 (1.2 x 10⁷ PFU/ml) was inoculated into a cell culture of human hepatocyte Chang Liver and adsorbed onto the cells at 37 °C for 2 hours and then cultured at 37 °C for 3 days with only M-199. 200 ml of the supernatant of the culture was subjected to centrifugation at 3000 x g for 5 minutes to obtain a supernatant, which was further subjected to centrifugation at 48000 x g and at 4 °C for 14 hours, to obtain a precipitate. The precipitate was suspended in 2 ml of M/75 PBS and subjected to ultrasonic treatment at 20 kHz (200W) for 2 minutes and the resultant product was subjected to sucrose density-gradient centrifugation at 160,000 x g and at 4 °C for 15 hours and wherein the sucrose density was changed from 20 (w/v)% to 60 (w/v)%, thereby obtaining fractions. Each of the fractions was mixed with a sample buffer containing, in the final concentration, 20 (v/v)% glycerol, 100 mM (pH 6.8) Tris·HCl, 2 (w/v)% SDS, 2 (v/v)% 2-mercaptoethanol and 0.02 (w/v)% BPB, and heated at 100 °C for 5 minutes and then subjected to 0.1 (w/v)% SDS-12.5 % polyacrylamide gel electrophoresis, to thereby separate proteins from each other. Then the gel was subjected to a trans blot apparatus (manufactured and sold by Nippon Eido Co., Ltd., Japan), to thereby blot the proteins which were separated by electrophoresis onto Hybond-ECL membrane (manufactured and sold by Amershan, England). The Hybond-ECL membrane was then immersed in a solution composed of 10 mM Tris·HCl (pH 7.2), 150 mM NaCl, 0.05 (w/v)% Tween-20 (T-TBS) and 5 (w/v)% skim milk, and incubated at room temperature for 1 hour, thereby blocking the membrane. Then, the anti-core antigen monoclonal antibody (clone 29) which had been diluted 500-fold with a T-TBS buffer containing 1 % skim milk was reacted with the Hybond-ECL membrane at 37 °C for 1 hour and then the membrane was washed well 2 times with a fresh one of the above-mentioned T-TBS buffer containing 1 % skim milk. Then, the membrane was reacted with biotin-labeled anti-mouse IgG (manufactured and sold by Cappel Co., ltd., Germany; diluted 500-fo1d) at room temperature for 1 hour. The Hybond-ECL membrane was then washed 2 times and reacted with HRPO-labeled streptoavidin (manufactured and sold by Amersham, England; diluted 500-fold) at room temperature for 1 hour, and washed well 4 times with T-TBS. The membrane was subjected to chemical luminescene reaction by means of ECL Western blotting detection system (manufactured and sold by Amersham, England). The membrane was wrapped with Saran Wrap and kept in contact with an X-ray film for 30 seconds, followed by the development of the film. As a result, it was found that activities of a core antigen and an envelope antigen of NANBV were observed at a sucrose concentration of 44 to 58 (w/v)% (as shown in Fig. 7). This fact indicates that there were obtained NANBV particles.

Step 5 (Observation of recombinant vaccinia virus-infected cells under electron microscope)

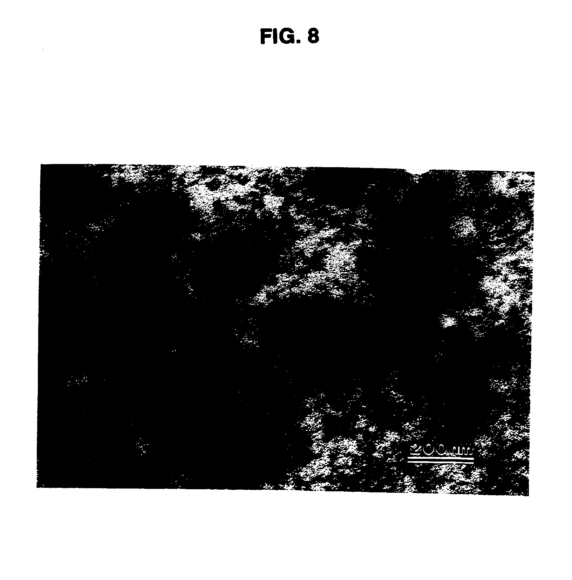

Recombinant vaccinia virus clones vXX39 and vUV17 (the later having no NANBV genome) were cultured on human hepatocyte for 2 days in the same manner as described in Reference Example 4, and the infected cells were collected by means of a rubber police. The collected cells were embedded in an epoxy resin (manufactured and sold by Nissin EM Co., Ltd., Japan) by the customary method and ultra-thin section samples were prepared by slicing. The samples were subjected to uranium-lead double staining, using 2 % uranium acetate and lead citrate, and examined under an electron microscope. As a result, particles were observed in the cells infected with vXX39 as shown in Fig. 8, but not in the cells infected with vUV17 and having no NANBV genome in the cytoplasm. These results, taken together with the results of Step 4, show that vXX39 had produced NANBV particles.

SEQUENCE LISTING

(1) GENERAL INFORMATION:

    (i) APPLICANT: THE RESEARCH FOUNDATION OF MICROBIAL DISEASES
            OF OSAKA UNIVERSITY

    (ii) TITLE OF INVENTION: NON-A, NON-B HEPATITIS VIRUS GENOMIC
           cDNA AND ANTIGEN POLYPEPTIDE

    (iii) NUMBER OF SEQUENCES: 2

    (iv) CORRESPONDENCE ADDRESS:
        (A) ADDRESSEE: Brookes & Martin
        (B) STREET: High Holborn House, 52/54, High Holborn
        (C) CITY: London
        (E) COUNTRY: United Kingdom
        (F) ZIP: WC1V 6SE

    (v) COMPUTER READABLE FORM:
        (A) MEDIUM TYPE: Floppy disk
        (B) COMPUTER: IBM PC compatible
        (C) OPERATING SYSTEM: PC-DOS/MS-DOS
        (D) SOFTWARE: PatentIn Release #1.0, Version #1.25

    (vi) CURRENT APPLICATION DATA:
        (A) APPLICATION NUMBER: EP 91305717.0
        (B) FILING DATE: 25-JUNE-1991
        (C) CLASSIFICATION:

    (vii) PRIOR APPLICATION DATA:
        (A) APPLICATION NUMBER: JP 2-167466
        (B) FILING DATE: 25-JUN-1990

    (vii) PRIOR APPLICATION DATA:
        (A) APPLICATION NUMBER: JP 2-230921
        (B) FILING DATE: 31-AUG-1990

    (vii) PRIOR APPLICATION DATA:
        (A) APPLICATION NUMBER: JP 2-305605
        (B) FILING DATE: 09-NOV-1990

    (vii) PRIOR APPLICATION DATA:
        (A) APPLICATION NUMBER: US 07/635,451
        (B) FILING DATE: 28-DEC-1990

    (vii) PRIOR APPLICATION DATA:
        (A) APPLICATION NUMBER: JP 3-132090
        (B) FILING DATE: 08-MAY-1991

```
    (vii) PRIOR APPLICATION DATA:
          (A) APPLICATION NUMBER: JP 3-138493
          (B) FILING DATE: 14-MAY-1991

   (viii) ATTORNEY/AGENT INFORMATION:
          (A) NAME: BLAKE, John H.
          (C) REFERENCE/DOCKET NUMBER: JHB/91-1011

     (ix) TELECOMMUNICATION INFORMATION:
          (A) TELEPHONE: (44) 892-510600
          (B) TELEFAX: (44) 71-831-0586


(2) INFORMATION FOR SEQ ID NO:1:

     (i) SEQUENCE CHARACTERISTICS:
          (A) LENGTH: 9416 base pairs
          (B) TYPE: nucleic acid
          (C) STRANDEDNESS: double
          (D) TOPOLOGY: linear

    (ii) MOLECULE TYPE: DNA (genomic)

   (iii) HYPOTHETICAL: NO

    (iv) ANTI-SENSE: NO

     (v) FRAGMENT TYPE: internal

    (vi) ORIGINAL SOURCE:
          (A) ORGANISM: Hepatitis virus
          (B) STRAIN: Non-A, Non-B
          (C) INDIVIDUAL ISOLATE: Human
          (D) DEVELOPMENTAL STAGE: Suspension cells
          (E) HAPLOTYPE: Diploid
          (F) TISSUE TYPE: Liver
          (G) CELL TYPE: Hepatocyte
          (H) CELL LINE: ATCC CCL 13

   (vii) IMMEDIATE SOURCE:
          (A) LIBRARY: BK170, BK171, BK172
          (B) CLONE: pDEL-NS5

  (viii) POSITION IN GENOME:
          (A) CHROMOSOME/SEGMENT: N/A
          (B) MAP POSITION: Infectious Agent
          (C) UNITS: bp

    (ix) FEATURE:
          (A) NAME/KEY: CDS
```

(B) LOCATION: 333..9362

(xi) SEQUENCE DESCRIPTION: SEQ ID NO:1:

```
CGATTGGGGG CGACACTCCA CCATAGATCA CTCCCCTGTG AGGAACTACT GTCTTCACGC        60

AGAAAGCGTC TAGCCATGGC GTTAGTATGA GTGTCGTGCA GCCTCCAGGA CCCCCCCTCC       120

CGGGAGAGCC ATAGTGGTCT GCGGAACCGG TGAGTACACC GGAATTGCCA GGACGACCGG       180

GTCCTTTCTT GGATCAACCC GCTCAATGCC TGGAGATTTG GGCGTGCCCC CGCGAGACTG       240

CTAGCCGAGT AGTGTTGGGT CGCGAAAGGC CTTGTGGTAC TGCCTGATAG GGTGCTTGCG       300

AGTGCCCCGG GAGGTCTCGT AGACCGTGCA CC ATG AGC ACG AAT CCT AAA CCT       353
                                   Met Ser Thr Asn Pro Lys Pro
                                    1                   5

CAA AGA AAA ACC AAA CGT AAC ACC AAC CGC CGC CCA CAG GAC GTC AAG       401
Gln Arg Lys Thr Lys Arg Asn Thr Asn Arg Arg Pro Gln Asp Val Lys
            10                  15                  20

TTC CCG GGC GGT GGT CAG ATC GTT GGT GGA GTT TAC CTG TTG CCG CGC       449
Phe Pro Gly Gly Gly Gln Ile Val Gly Gly Val Tyr Leu Leu Pro Arg
        25                  30                  35

AGG GGC CCC AGG TTG GGT GTG CGC GCG CCC AGG AAG ACT TCC GAG CGG       497
Arg Gly Pro Arg Leu Gly Val Arg Ala Pro Arg Lys Thr Ser Glu Arg
40                  45                  50                  55

TCG CAA CCT CGT GGA AGG CGA CAA CCT ATC CCC AAG GCT CGC CGG CCC       545
Ser Gln Pro Arg Gly Arg Arg Gln Pro Ile Pro Lys Ala Arg Arg Pro
                60                  65                  70

GAG GGC AGG ACC TGG GCT CAG CCC GGG TAC CCT TGG CCT CTC TAT GGC       593
Glu Gly Arg Thr Trp Ala Gln Pro Gly Tyr Pro Trp Pro Leu Tyr Gly
                75                  80                  85

AAT GAG GGC TTA GGG TGG GCA GGA TGG CTC CTG TCA CCC CGC GGC TCC       641
Asn Glu Gly Leu Gly Trp Ala Gly Trp Leu Leu Ser Pro Arg Gly Ser
            90                  95                  100

CGG CCT AGT TGG GGC CCC ACG GAC CCC CGG CGT AGG TCG CGT AAT TTG       689
Arg Pro Ser Trp Gly Pro Thr Asp Pro Arg Arg Arg Ser Arg Asn Leu
        105                 110                 115

GGT AAG GTC ATC GAT ACC CTC ACA TGC GGC TTC GCC GAT CTC ATG GGG       737
Gly Lys Val Ile Asp Thr Leu Thr Cys Gly Phe Ala Asp Leu Met Gly
```

```
                120                  125                     130                    135

TAC ATT CCG CTC GTC GGC GCC CCC CTG GGG GGC GCT GCC AGG GCC CTG        785
Tyr Ile Pro Leu Val Gly Ala Pro Leu Gly Gly Ala Ala Arg Ala Leu
                    140                  145                  150

GCA CAT GGT GTC CGG GTT CTG GAG GAC GGC GTG AAC TAT GCA ACA GGG        833
Ala His Gly Val Arg Val Leu Glu Asp Gly Val Asn Tyr Ala Thr Gly
                    155                  160                  165

AAT CTG CCC GGT TGC TCT TTT TCT ATC TTC CTC TTG GCT CTG CTG TCC        881
Asn Leu Pro Gly Cys Ser Phe Ser Ile Phe Leu Leu Ala Leu Leu Ser
                    170                  175                  180

TGC CTG ACC ACC CCA GCT TCC GCT TAC GAA GTG CAC AAC GTG TCC GGG        929
Cys Leu Thr Thr Pro Ala Ser Ala Tyr Glu Val His Asn Val Ser Gly
                185                  190                  195

ATA TAT CAT GTC ACG AAC GAC TGC TCC AAC GCA AGC ATT GTG TAT GAG        977
Ile Tyr His Val Thr Asn Asp Cys Ser Asn Ala Ser Ile Val Tyr Glu
200                  205                  210                  215

GCA GCG GAC TTG ATC ATG CAT ACT CCT GGG TGC GTG CCC TGC GTT CGG       1025
Ala Ala Asp Leu Ile Met His Thr Pro Gly Cys Val Pro Cys Val Arg
                    220                  225                  230

GAA GGC AAC TCC TCC CGC TGC TGG GTA GCG CTC ACT CCC ACG CTC GCA       1073
Glu Gly Asn Ser Ser Arg Cys Trp Val Ala Leu Thr Pro Thr Leu Ala
                    235                  240                  245

GCC AGG AAC GTC ACC ATC CCC ACC ACG ACG ATA CGA CGC CAC GTC GAT       1121
Ala Arg Asn Val Thr Ile Pro Thr Thr Thr Ile Arg Arg His Val Asp
                    250                  255                  260

CTG CTC GTT GGG GCG GCT GCT TTC TGT TCC GCT ATG TAC GTG GGG GAC       1169
Leu Leu Val Gly Ala Ala Ala Phe Cys Ser Ala Met Tyr Val Gly Asp
                265                  270                  275

CTC TGC GGA TCT GTT TTC CTC GTC TCT CAG CTG TTC ACC TTC TCG CCT       1217
Leu Cys Gly Ser Val Phe Leu Val Ser Gln Leu Phe Thr Phe Ser Pro
280                  285                  290                  295

CGC CGG CAT GTG ACA TTA CAG GAC TGT AAC TGC TCA ATT TAT CCC GGC       1265
Arg Arg His Val Thr Leu Gln Asp Cys Asn Cys Ser Ile Tyr Pro Gly
                    300                  305                  310

CAT GTG TCG GGT CAC CGT ATG GCT TGG GAC ATG ATG ATG AAC TGG TCG       1313
His Val Ser Gly His Arg Met Ala Trp Asp Met Met Met Asn Trp Ser
                    315                  320                  325
```

```
CCC ACA ACA GCC CTA GTG GTG TCG CAG TTA CTC CGG ATC CCA CAA GCC      1361
Pro Thr Thr Ala Leu Val Val Ser Gln Leu Leu Arg Ile Pro Gln Ala
        330                     335                 340

GTC GTG GAC ATG GTG GCG GGG GCC CAC TGG GGA GTC CTG GCG GGC CTT      1409
Val Val Asp Met Val Ala Gly Ala His Trp Gly Val Leu Ala Gly Leu
        345                 350                 355

GCC TAC TAT TCC ATG GCG GGG AAC TGG GCT AAG GTT CTG ATT GTG ATG      1457
Ala Tyr Tyr Ser Met Ala Gly Asn Trp Ala Lys Val Leu Ile Val Met
360                 365                 370                 375

CTA CTT TTT GCT GGC GTT GAC GGG GAT ACC CAC GTG ACA GGG GGG GCG      1505
Leu Leu Phe Ala Gly Val Asp Gly Asp Thr His Val Thr Gly Gly Ala
            380                 385                 390

CAA GCC AAA ACC ACC AAC AGG CTC GTG TCC ATG TTC GCA AGT GGG CCG      1553
Gln Ala Lys Thr Thr Asn Arg Leu Val Ser Met Phe Ala Ser Gly Pro
        395                 400                 405

TCT CAG AAA ATC CAG CTT ATA AAC ACC AAT GGG AGT TGG CAC ATC AAC      1601
Ser Gln Lys Ile Gln Leu Ile Asn Thr Asn Gly Ser Trp His Ile Asn
        410                 415                 420

AGG ACT GCC CTG AAC TGC AAT GAC TCT CTC CAG ACT GGG TTT CTT GCC      1649
Arg Thr Ala Leu Asn Cys Asn Asp Ser Leu Gln Thr Gly Phe Leu Ala
        425                 430                 435

GCG CTG TTC TAC ACA CAT AGT TTC AAC TCG TCC GGG TGC CCA GAG CGC      1697
Ala Leu Phe Tyr Thr His Ser Phe Asn Ser Ser Gly Cys Pro Glu Arg
440                 445                 450                 455

ATG GCC CAG TGC CGC ACC ATT GAC AAG TTC GAC CAG GGA TGG GGT CCC      1745
Met Ala Gln Cys Arg Thr Ile Asp Lys Phe Asp Gln Gly Trp Gly Pro
            460                 465                 470

ATT ACT TAT GCT GAG TCT AGC AGA TCA GAC CAG AGG CCA TAT TGC TGG      1793
Ile Thr Tyr Ala Glu Ser Ser Arg Ser Asp Gln Arg Pro Tyr Cys Trp
            475                 480                 485

CAC TAC CCA CCT CCA CAA TGT ACC ATC GTA CCT GCG TCG GAG GTG TGC      1841
His Tyr Pro Pro Pro Gln Cys Thr Ile Val Pro Ala Ser Glu Val Cys
        490                 495                 500

GGC CCA GTG TAC TGC TTC ACC CCA AGC CCT GTC GTC GTG GGG ACG ACC      1889
Gly Pro Val Tyr Cys Phe Thr Pro Ser Pro Val Val Val Gly Thr Thr
        505                 510                 515

GAT CGT TTC GGT GTC CCT ACG TAT AGA TGG GGG GAG AAC GAG ACT GAC      1937
Asp Arg Phe Gly Val Pro Thr Tyr Arg Trp Gly Glu Asn Glu Thr Asp
```

```
        520                     525                     530                     535

        GTG CTG CTG CTC AAC AAC ACG CGG CCG CCG CAA GGC AAC TGG TTC GGC          1985
        Val Leu Leu Leu Asn Asn Thr Arg Pro Pro Gln Gly Asn Trp Phe Gly
                        540                     545                     550

        TGC ACA TGG ATG AAT AGC ACC GGG TTC ACC AAG ACA TGT GGG GGG CCC          2033
        Cys Thr Trp Met Asn Ser Thr Gly Phe Thr Lys Thr Cys Gly Gly Pro
                        555                     560                     565

        CCG TGT AAC ATC GGG GGG GTC GGC AAC AAC ACC CTG ACC TGC CCC ACG          2081
        Pro Cys Asn Ile Gly Gly Val Gly Asn Asn Thr Leu Thr Cys Pro Thr
                    570                     575                     580

        GAC TGC TTC CGG AAG CAC CCC GAG GCT ACC TAC ACA AAA TGT GGT TCG          2129
        Asp Cys Phe Arg Lys His Pro Glu Ala Thr Tyr Thr Lys Cys Gly Ser
                    585                     590                     595

        GGG CCT TGG CTG ACA CCT AGG TGC ATG GTT GAC TAT CCA TAC AGG CTC          2177
        Gly Pro Trp Leu Thr Pro Arg Cys Met Val Asp Tyr Pro Tyr Arg Leu
        600                     605                     610                     615

        TGG CAT TAC CCC TGC ACT GTT AAC TTT ACC ATC TTC AAG GTT AGG ATG          2225
        Trp His Tyr Pro Cys Thr Val Asn Phe Thr Ile Phe Lys Val Arg Met
                        620                     625                     630

        TAT GTG GGG GGG GTG GAG CAC AGG CTC AAT GCT GCA TGC AAT TGG ACC          2273
        Tyr Val Gly Gly Val Glu His Arg Leu Asn Ala Ala Cys Asn Trp Thr
                        635                     640                     645

        CGA GGA GAG CGT TGT GAC TTG GAG GAC AGG GAT AGG CCG GAG CTC AGC          2321
        Arg Gly Glu Arg Cys Asp Leu Glu Asp Arg Asp Arg Pro Glu Leu Ser
                        650                     655                     660

        CCG CTG CTG CTG TCT ACA ACA GAG TGG CAG GTA CTG CCC TGT TCC TTC          2369
        Pro Leu Leu Leu Ser Thr Thr Glu Trp Gln Val Leu Pro Cys Ser Phe
                    665                     670                     675

        ACC ACC CTA CCA GCT CTG TCC ACT GGC TTG ATT CAC CTC CAT CAG AAC          2417
        Thr Thr Leu Pro Ala Leu Ser Thr Gly Leu Ile His Leu His Gln Asn
                680                     685                     690                     695

        ATC GTG GAC GTG CAA TAC CTA TAC GGT ATA GGG TCA GCG GTT GTC TCC          2465
        Ile Val Asp Val Gln Tyr Leu Tyr Gly Ile Gly Ser Ala Val Val Ser
                        700                     705                     710

        TTT GCA ATC AAA TGG GAG TAT GTC CTG TTG CTT TTC CTT CTC CTA GCG          2513
        Phe Ala Ile Lys Trp Glu Tyr Val Leu Leu Leu Phe Leu Leu Leu Ala
                        715                     720                     725
```

```
GAC GCA CGT GTC TGT GCC TGC TTG TGG ATG ATG CTG CTG ATA GCC CAG      2561
Asp Ala Arg Val Cys Ala Cys Leu Trp Met Met Leu Leu Ile Ala Gln
        730             735             740

GCC GAG GCC GCC TTG GAG AAC CTG GTG GTC CTC AAT TCG GCG TCT GTG      2609
Ala Glu Ala Ala Leu Glu Asn Leu Val Val Leu Asn Ser Ala Ser Val
        745             750             755

GCC GGC GCA CAT GGC ATC CTC TCC TTC CTT GTG TTC TTC TGT GCC GCC      2657
Ala Gly Ala His Gly Ile Leu Ser Phe Leu Val Phe Phe Cys Ala Ala
760             765             770             775

TGG TAC ATC AAA GGC AGG CTG GTC CCT GGG GCG ACA TAT GCT CTT TAT      2705
Trp Tyr Ile Lys Gly Arg Leu Val Pro Gly Ala Thr Tyr Ala Leu Tyr
            780             785             790

GGC GTG TGG CCG CTG CTC CTG CTC TTG CTG GCA TTA CCA CCG CGA GCT      2753
Gly Val Trp Pro Leu Leu Leu Leu Leu Leu Ala Leu Pro Pro Arg Ala
        795             800             805

TAC GCC ATG GAC CGG GAG ATG GCT GCA TCG TGC GGA GGC GCG GTT TTT      2801
Tyr Ala Met Asp Arg Glu Met Ala Ala Ser Cys Gly Gly Ala Val Phe
        810             815             820

GTG GGT CTG GTA CTC CTG ACT TTG TCA CCA TAC TAC AAG GTG TTC CTC      2849
Val Gly Leu Val Leu Leu Thr Leu Ser Pro Tyr Tyr Lys Val Phe Leu
        825             830             835

GCT AGG CTC ATA TGG TGG TTA CAA TAT TTT ACC ACC AGA GCC GAG GCG      2897
Ala Arg Leu Ile Trp Trp Leu Gln Tyr Phe Thr Thr Arg Ala Glu Ala
840             845             850             855

GAC TTA CAT GTG TGG ATC CCC CCC CTC AAC GCT CGG GGA GGC CGC GAT      2945
Asp Leu His Val Trp Ile Pro Pro Leu Asn Ala Arg Gly Gly Arg Asp
            860             865             870

GCC ATC ATC CTC CTC ATG TGC GCA GTC CAT CCA GAG CTA ATC TTT GAC      2993
Ala Ile Ile Leu Leu Met Cys Ala Val His Pro Glu Leu Ile Phe Asp
            875             880             885

ATC ACC AAA CTT CTA ATT GCC ATA CTC GGT CCG CTC ATG GTG CTC CAA      3041
Ile Thr Lys Leu Leu Ile Ala Ile Leu Gly Pro Leu Met Val Leu Gln
        890             895             900

GCT GGC ATA ACC AGA GTG CCG TAC TTC GTG CGC GCT CAA GGG CTC ATT      3089
Ala Gly Ile Thr Arg Val Pro Tyr Phe Val Arg Ala Gln Gly Leu Ile
        905             910             915

CAT GCA TGC ATG TTA GTG CGG AAG GTC GCT GGG GGT CAT TAT GTC CAA      3137
His Ala Cys Met Leu Val Arg Lys Val Ala Gly Gly His Tyr Val Gln
```

```
                920                     925                      930                     935

                ATG GCC TTC ATG AAG CTG GGC GCG CTG ACA GGC ACG TAC ATT TAC AAC          3185
                Met Ala Phe Met Lys Leu Gly Ala Leu Thr Gly Thr Tyr Ile Tyr Asn
                            940                 945                 950

                CAT CTT ACC CCG CTA CGG GAT TGG CCA CGC GCG GGC CTA CGA GAC CTT          3233
                His Leu Thr Pro Leu Arg Asp Trp Pro Arg Ala Gly Leu Arg Asp Leu
                            955                 960                 965

                GCG GTG GCA GTG GAG CCC GTC GTC TTC TCC GAC ATG GAG ACC AAG ATC          3281
                Ala Val Ala Val Glu Pro Val Val Phe Ser Asp Met Glu Thr Lys Ile
                            970                 975                 980

                ATC ACC TGG GGA GCA GAC ACC GCG GCG TGT GGG GAC ATC ATC TTG GGT          3329
                Ile Thr Trp Gly Ala Asp Thr Ala Ala Cys Gly Asp Ile Ile Leu Gly
                            985                 990                 995

                CTG CCC GTC TCC GCC CGA AGG GGA AAG GAG ATA CTC CTG GGC CCG GCC          3377
                Leu Pro Val Ser Ala Arg Arg Gly Lys Glu Ile Leu Leu Gly Pro Ala
                1000                1005                1010                1015

                GAT AGT CTT GAA GGG CGG GGG TTG CGA CTC CTC GCG CCC ATC ACG GCC          3425
                Asp Ser Leu Glu Gly Arg Gly Leu Arg Leu Leu Ala Pro Ile Thr Ala
                            1020                1025                1030

                TAC TCC CAA CAG ACG CGG GGC CTA CTT GGT TGC ATC ATC ACT AGC CTT          3473
                Tyr Ser Gln Gln Thr Arg Gly Leu Leu Gly Cys Ile Ile Thr Ser Leu
                            1035                1040                1045

                ACA GGC CGG GAC AAG AAC CAG GTC GAG GGA GAG GTT CAG GTG GTT TCC          3521
                Thr Gly Arg Asp Lys Asn Gln Val Glu Gly Glu Val Gln Val Val Ser
                            1050                1055                1060

                ACC GCA ACA CAA TCC TTC CTG GCG ACC TGC GTC AAC GGC GTG TGT TGG          3569
                Thr Ala Thr Gln Ser Phe Leu Ala Thr Cys Val Asn Gly Val Cys Trp
                1065                1070                1075

                ACC GTT TAC CAT GGT GCT GGC TCA AAG ACC TTA GCC GCG CCA AAG GGG          3617
                Thr Val Tyr His Gly Ala Gly Ser Lys Thr Leu Ala Ala Pro Lys Gly
                1080                1085                1090                1095

                CCA ATC ACC CAG ATG TAC ACT AAT GTG GAC CAG GAC CTC GTC GGC TGG          3665
                Pro Ile Thr Gln Met Tyr Thr Asn Val Asp Gln Asp Leu Val Gly Trp
                            1100                1105                1110

                CCC AAG CCC CCC GGG GCG CGT TCC TTG ACA CCA TGC ACC TGT GGC AGC          3713
                Pro Lys Pro Pro Gly Ala Arg Ser Leu Thr Pro Cys Thr Cys Gly Ser
                            1115                1120                1125
```

```
TCA GAC CTT TAC TTG GTC ACG AGA CAT GCT GAC GTC ATT CCG GTG CGC    3761
Ser Asp Leu Tyr Leu Val Thr Arg His Ala Asp Val Ile Pro Val Arg
        1130                1135                1140

CGG CGG GGC GAC AGT AGG GGG AGC CTG CTC TCC CCC AGG CCT GTC TCC    3809
Arg Arg Gly Asp Ser Arg Gly Ser Leu Leu Ser Pro Arg Pro Val Ser
        1145                1150                1155

TAC TTG AAG GGC TCT TCG GGT GGT CCA CTG CTC TGC CCC TTC GGG CAC    3857
Tyr Leu Lys Gly Ser Ser Gly Gly Pro Leu Leu Cys Pro Phe Gly His
1160                 1165                1170                1175

GCT GTG GGC ATC TTC CGG GCT GCC GTA TGC ACC CGG GGG GTT GCG AAG    3905
Ala Val Gly Ile Phe Arg Ala Ala Val Cys Thr Arg Gly Val Ala Lys
                1180                1185                1190

GCG GTG GAC TTT GTG CCC GTA GAG TCC ATG GAA ACT ACT ATG CGG TCT    3953
Ala Val Asp Phe Val Pro Val Glu Ser Met Glu Thr Thr Met Arg Ser
                1195                1200                1205

CCG GTC TTC ACG GAC AAC TCA TCC CCC CCG GCC GTA CCG CAG TCA TTT    4001
Pro Val Phe Thr Asp Asn Ser Ser Pro Pro Ala Val Pro Gln Ser Phe
                1210                1215                1220

CAA GTG GCC CAC CTA CAC GCT CCC ACT GGC AGC GGC AAG AGT ACT AAA    4049
Gln Val Ala His Leu His Ala Pro Thr Gly Ser Gly Lys Ser Thr Lys
        1225                1230                1235

GTG CCG GCT GCA TAT GCA GCC CAA GGG TAC AAG GTG CTC GTC CTC AAT    4097
Val Pro Ala Ala Tyr Ala Ala Gln Gly Tyr Lys Val Leu Val Leu Asn
1240                 1245                1250                1255

CCG TCC GTT GCC GCT ACC TTA GGG TTT GGG GCG TAT ATG TCT AAG GCA    4145
Pro Ser Val Ala Ala Thr Leu Gly Phe Gly Ala Tyr Met Ser Lys Ala
                1260                1265                1270

CAC GGT ATT GAC CCC AAC ATC AGA ACT GGG GTA AGG ACC ATT ACC ACA    4193
His Gly Ile Asp Pro Asn Ile Arg Thr Gly Val Arg Thr Ile Thr Thr
                1275                1280                1285

GGC GCC CCC GTC ACA TAC TCT ACC TAT GGC AAG TTT CTT GCC GAT GGT    4241
Gly Ala Pro Val Thr Tyr Ser Thr Tyr Gly Lys Phe Leu Ala Asp Gly
            1290                1295                1300

GGT TGC TCT GGG GGC GCT TAT GAC ATC ATA ATA TGT GAT GAG TGC CAT    4289
Gly Cys Ser Gly Gly Ala Tyr Asp Ile Ile Ile Cys Asp Glu Cys His
        1305                1310                1315

TCA ACT GAC TCG ACT ACA ATC TTG GGC ATC GGC ACA GTC CTG GAC CAA    4337
Ser Thr Asp Ser Thr Thr Ile Leu Gly Ile Gly Thr Val Leu Asp Gln
```

```
         1320                1325    ·              1330              1335

GCG GAG ACG GCT GGA GCG CGG CTT GTC GTG CTC GCC ACC GCT ACG CCT        4385
Ala Glu Thr Ala Gly Ala Arg Leu Val Val Leu Ala Thr Ala Thr Pro
                1340                1345                1350

CCG GGA TCG GTC ACC GTG CCA CAC CCA AAC ATC GAG GAG GTG GCC CTG        4433
Pro Gly Ser Val Thr Val Pro His Pro Asn Ile Glu Glu Val Ala Leu
                1355                1360                1365

TCT AAT ACT GGA GAG ATC CCC TTC TAT GGC AAA GCC ATC CCC ATT GAA        4481
Ser Asn Thr Gly Glu Ile Pro Phe Tyr Gly Lys Ala Ile Pro Ile Glu
                1370                1375                1380

GCC ATC AGG GGG GGA AGG CAT CTC ATT TTC TGT CAT TCC AAG AAG AAG        4529
Ala Ile Arg Gly Gly Arg His Leu Ile Phe Cys His Ser Lys Lys Lys
                1385                1390                1395

TGC GAC GAG CTC GCC GCA AAG CTG TCA GGC CTC GGA ATC AAC GCT GTG        4577
Cys Asp Glu Leu Ala Ala Lys Leu Ser Gly Leu Gly Ile Asn Ala Val
1400                1405                1410                1415

GCG TAT TAC CGG GGG CTC GAT GTG TCC GTC ATA CCA ACT ATC GGA GAC        4625
Ala Tyr Tyr Arg Gly Leu Asp Val Ser Val Ile Pro Thr Ile Gly Asp
                1420                1425                1430

GTC GTT GTC GTG GCA ACA GAC GCT CTG ATG ACG GGC TAT ACG GGC GAC        4673
Val Val Val Val Ala Thr Asp Ala Leu Met Thr Gly Tyr Thr Gly Asp
                1435                1440                1445

TTT GAC TCA GTG ATC GAC TGT AAC ACA TGT GTC ACC CAG ACA GTC GAC        4721
Phe Asp Ser Val Ile Asp Cys Asn Thr Cys Val Thr Gln Thr Val Asp
                1450                1455                1460

TTC AGC TTG GAT CCC ACC TTC ACC ATT GAG ACG ACG ACC GTG CCT CAA        4769
Phe Ser Leu Asp Pro Thr Phe Thr Ile Glu Thr Thr Thr Val Pro Gln
                1465                1470                1475

GAC GCA GTG TCG CGC TCG CAG CGG CGG GGT AGG ACT GGC AGG GGT AGG        4817
Asp Ala Val Ser Arg Ser Gln Arg Arg Gly Arg Thr Gly Arg Gly Arg
1480                1485                1490                1495

AGA GGC ATC TAC AGG TTT GTG ACT CCG GGA GAA CGG CCC TCG GGC ATG        4865
Arg Gly Ile Tyr Arg Phe Val Thr Pro Gly Glu Arg Pro Ser Gly Met
                1500                1505                1510

TTC GAT TCC TCG GTC CTG TGT GAG TGC TAT GAC GCG GGC TGT GCT TGG        4913
Phe Asp Ser Ser Val Leu Cys Glu Cys Tyr Asp Ala Gly Cys Ala Trp
                1515                1520                1525
```

```
TAC GAG CTC ACC CCG GCC GAG ACC TCG GTT AGG TTG CGG GCC TAC CTG    4961
Tyr Glu Leu Thr Pro Ala Glu Thr Ser Val Arg Leu Arg Ala Tyr Leu
        1530            1535            1540

AAC ACA CCA GGG TTG CCC GTT TGC CAG GAC CAC CTG GAG TTC TGG GAG    5009
Asn Thr Pro Gly Leu Pro Val Cys Gln Asp His Leu Glu Phe Trp Glu
        1545            1550            1555

AGT GTC TTC ACA GGC CTC ACC CAT ATA GAT GCA CAC TTC TTG TCC CAG    5057
Ser Val Phe Thr Gly Leu Thr His Ile Asp Ala His Phe Leu Ser Gln
1560            1565            1570            1575

ACC AAG CAG GCA GGA GAC AAC TTC CCC TAC CTG GTA GCA TAC CAA GCC    5105
Thr Lys Gln Ala Gly Asp Asn Phe Pro Tyr Leu Val Ala Tyr Gln Ala
            1580            1585            1590

ACG GTG TGC GCC AGG GCT CAG GCC CCA CCT CCA TCA TGG GAT CAA ATG    5153
Thr Val Cys Ala Arg Ala Gln Ala Pro Pro Pro Ser Trp Asp Gln Met
        1595            1600            1605

TGG AAG TGT CTC ATA CGG CTG AAA CCT ACG CTG CAC GGG CCA ACA CCC    5201
Trp Lys Cys Leu Ile Arg Leu Lys Pro Thr Leu His Gly Pro Thr Pro
        1610            1615            1620

TTG CTG TAC AGG CTG GGA GCC GTC CAG AAT GAG GTC ACC CTC ACC CAC    5249
Leu Leu Tyr Arg Leu Gly Ala Val Gln Asn Glu Val Thr Leu Thr His
        1625            1630            1635

CCC ATA ACC AAA TAC ATC ATG GCA TGC ATG TCG GCT GAC CTG GAG GTC    5297
Pro Ile Thr Lys Tyr Ile Met Ala Cys Met Ser Ala Asp Leu Glu Val
1640            1645            1650            1655

GTC ACT AGC ACC TGG GTG CTG GTG GGC GGA GTC CTT GCA GCT CTG GCC    5345
Val Thr Ser Thr Trp Val Leu Val Gly Gly Val Leu Ala Ala Leu Ala
            1660            1665            1670

GCG TAT TGC CTG ACA ACA GGC AGT GTG GTC ATT GTG GGT AGG ATT ATC    5393
Ala Tyr Cys Leu Thr Thr Gly Ser Val Val Ile Val Gly Arg Ile Ile
            1675            1680            1685

TTG TCC GGG AGG CCG GCC ATT GTT CCC GAC AGG GAG CTT CTC TAC CAG    5441
Leu Ser Gly Arg Pro Ala Ile Val Pro Asp Arg Glu Leu Leu Tyr Gln
        1690            1695            1700

GAG TTC GAT GAA ATG GAA GAG TGC GCC TCG CAC CTC CCT TAC ATC GAG    5489
Glu Phe Asp Glu Met Glu Glu Cys Ala Ser His Leu Pro Tyr Ile Glu
        1705            1710            1715

CAG GGA ATG CAG CTC GCC GAG CAA TTC AAG CAG AAA GCG CTC GGG TTA    5537
Gln Gly Met Gln Leu Ala Glu Gln Phe Lys Gln Lys Ala Leu Gly Leu
```

```
       1720                  1725                  1730                  1735

CTG CAA ACA GCC ACC AAA CAA GCG GAG GCT GCT GCT CCC GTG GTG GAG        5585
Leu Gln Thr Ala Thr Lys Gln Ala Glu Ala Ala Ala Pro Val Val Glu
                1740                  1745                  1750

TCC AAG TGG CGA GCC CTT GAG ACA TTC TGG GCG AAG CAC ATG TGG AAT        5633
Ser Lys Trp Arg Ala Leu Glu Thr Phe Trp Ala Lys His Met Trp Asn
                1755                  1760                  1765

TTC ATC AGC GGG ATA CAG TAC TTA GCA GGC TTA TCC ACT CTG CCT GGG        5681
Phe Ile Ser Gly Ile Gln Tyr Leu Ala Gly Leu Ser Thr Leu Pro Gly
                1770                  1775                  1780

AAC CCC GCA ATA GCA TCA TTG ATG GCA TTC ACA GCC TCT ATC ACC AGC        5729
Asn Pro Ala Ile Ala Ser Leu Met Ala Phe Thr Ala Ser Ile Thr Ser
                1785                  1790                  1795

CCG CTC ACC ACC CAA AGT ACC CTC CTG TTT AAC ATC TTG GGG GGG TGG        5777
Pro Leu Thr Thr Gln Ser Thr Leu Leu Phe Asn Ile Leu Gly Gly Trp
     1800                  1805                  1810                  1815

GTG GCT GCC CAA CTC GCC CCC CCC AGC GCC GCT TCG GCT TTC GTG GGC        5825
Val Ala Ala Gln Leu Ala Pro Pro Ser Ala Ala Ser Ala Phe Val Gly
                1820                  1825                  1830

GCC GGC ATC GCC GGT GCG GCT GTT GGC AGC ATA GGC CTT GGG AAG GTG        5873
Ala Gly Ile Ala Gly Ala Ala Val Gly Ser Ile Gly Leu Gly Lys Val
                1835                  1840                  1845

CTT GTG GAC ATT CTG GCG GGT TAT GGA GCA GGA GTG GCC GGC GCG CTC        5921
Leu Val Asp Ile Leu Ala Gly Tyr Gly Ala Gly Val Ala Gly Ala Leu
                1850                  1855                  1860

GTG GCC TTT AAG GTC ATG AGC GGC GAG ATG CCC TCC ACC GAG GAC CTG        5969
Val Ala Phe Lys Val Met Ser Gly Glu Met Pro Ser Thr Glu Asp Leu
                1865                  1870                  1875

GTC AAT CTA CTT CCT GCC ATC CTC TCT CCT GGC GCC CTG GTC GTC GGG        6017
Val Asn Leu Leu Pro Ala Ile Leu Ser Pro Gly Ala Leu Val Val Gly
     1880                  1885                  1890                  1895

GTC GTG TGT GCA GCA ATA CTG CGT CGA CAC GTG GGT CCG GGA GAG GGG        6065
Val Val Cys Ala Ala Ile Leu Arg Arg His Val Gly Pro Gly Glu Gly
                1900                  1905                  1910

GCT GTG CAG TGG ATG AAC CGG CTG ATA GCG TTC GCC TCG CGG GGT AAT        6113
Ala Val Gln Trp Met Asn Arg Leu Ile Ala Phe Ala Ser Arg Gly Asn
                1915                  1920                  1925
```

```
CAT GTT TCC CCC ACG CAC TAT GTG CCT GAG AGC GAC GCC GCA GCG CGT          6161
His Val Ser Pro Thr His Tyr Val Pro Glu Ser Asp Ala Ala Ala Arg
        1930             1935             1940

GTT ACT CAG ATC CTC TCC AGC CTT ACC ATC ACT CAG CTG CTG AAA AGG          6209
Val Thr Gln Ile Leu Ser Ser Leu Thr Ile Thr Gln Leu Leu Lys Arg
        1945             1950             1955

CTC CAC CAG TGG ATT AAT GAA GAC TGC TCC ACA CCG TGT TCC GGC TCG          6257
Leu His Gln Trp Ile Asn Glu Asp Cys Ser Thr Pro Cys Ser Gly Ser
1960             1965             1970             1975

TGG CTA AGG GAT GTT TGG GAC TGG ATA TGC ACG GTG TTG ACT GAC TTC          6305
Trp Leu Arg Asp Val Trp Asp Trp Ile Cys Thr Val Leu Thr Asp Phe
            1980             1985             1990

AAG ACC TGG CTC CAG TCC AAG CTC CTG CCG CAG CTA CCT GGA GTC CCT          6353
Lys Thr Trp Leu Gln Ser Lys Leu Leu Pro Gln Leu Pro Gly Val Pro
            1995             2000             2005

TTT TTC TCG TGC CAA CGC GGG TAC AAG GGA GTC TGG CGG GGA GAC GGC          6401
Phe Phe Ser Cys Gln Arg Gly Tyr Lys Gly Val Trp Arg Gly Asp Gly
        2010             2015             2020

ATC ATG CAA ACC ACC TGC CCA TGT GGA GCA CAG ATC ACC GGA CAT GTC          6449
Ile Met Gln Thr Thr Cys Pro Cys Gly Ala Gln Ile Thr Gly His Val
        2025             2030             2035

AAA AAC GGT TCC ATG AGG ATC GTC GGG CCT AAG ACC TGC AGC AAC ACG          6497
Lys Asn Gly Ser Met Arg Ile Val Gly Pro Lys Thr Cys Ser Asn Thr
2040             2045             2050             2055

TGG CAT GGA ACA TTC CCC ATC AAC GCA TAC ACC ACG GGC CCC TGC ACA          6545
Trp His Gly Thr Phe Pro Ile Asn Ala Tyr Thr Thr Gly Pro Cys Thr
            2060             2065             2070

CCC TCT CCA GCG CCA AAC TAT TCT AGG GCG CTG TGG CGG GTG GCC GCT          6593
Pro Ser Pro Ala Pro Asn Tyr Ser Arg Ala Leu Trp Arg Val Ala Ala
            2075             2080             2085

GAG GAG TAC GTG GAG GTC ACG CGG GTG GGG GAT TTC CAC TAC GTG ACG          6641
Glu Glu Tyr Val Glu Val Thr Arg Val Gly Asp Phe His Tyr Val Thr
            2090             2095             2100

GGC ATG ACC ACT GAC AAC GTA AAG TGC CCA TGC CAG GTT CCG GCT CCT          6689
Gly Met Thr Thr Asp Asn Val Lys Cys Pro Cys Gln Val Pro Ala Pro
            2105             2110             2115

GAA TTC TTC TCG GAG GTG GAC GGA GTG CGG TTG CAC AGG TAC GCT CCG          6737
Glu Phe Phe Ser Glu Val Asp Gly Val Arg Leu His Arg Tyr Ala Pro
```

```
          2120                    2125                    2130                    2135

GCG TGC AGG CCT CTC CTA CGG GAG GAG GTT ACA TTC CAG GTC GGG CTC        6785
Ala Cys Arg Pro Leu Leu Arg Glu Glu Val Thr Phe Gln Val Gly Leu
            2140                    2145                    2150

AAC CAA TAC CTG GTT GGG TCA CAG CTA CCA TGC GAG CCC GAA CCG GAT        6833
Asn Gln Tyr Leu Val Gly Ser Gln Leu Pro Cys Glu Pro Glu Pro Asp
            2155                    2160                    2165

GTA GCA GTG CTC ACT TCC ATG CTC ACC GAC CCC TCC CAC ATC ACA GCA        6881
Val Ala Val Leu Thr Ser Met Leu Thr Asp Pro Ser His Ile Thr Ala
            2170                    2175                    2180

GAA ACG GCT AAG CGT AGG TTG GCC AGG GGG TCT CCC CCC TCC TTG GCC        6929
Glu Thr Ala Lys Arg Arg Leu Ala Arg Gly Ser Pro Pro Ser Leu Ala
            2185                    2190                    2195

AGC TCT TCA GCT AGC CAG TTG TCT GCG CCT TCC TTG AAG GCG ACA TGC        6977
Ser Ser Ser Ala Ser Gln Leu Ser Ala Pro Ser Leu Lys Ala Thr Cys
2200                    2205                    2210                    2215

ACT ACC CAC CAT GTC TCT CCG GAC GCT GAC CTC ATC GAG GCC AAC CTC        7025
Thr Thr His His Val Ser Pro Asp Ala Asp Leu Ile Glu Ala Asn Leu
            2220                    2225                    2230

CTG TGG CGG CAG GAG ATG GGC GGG AAC ATC ACC CGC GTG GAG TCG GAG        7073
Leu Trp Arg Gln Glu Met Gly Gly Asn Ile Thr Arg Val Glu Ser Glu
            2235                    2240                    2245

AAC AAG GTG GTA GTC CTG GAC TCT TTC GAC CCG CTT CGA GCG GAG GAG        7121
Asn Lys Val Val Val Leu Asp Ser Phe Asp Pro Leu Arg Ala Glu Glu
            2250                    2255                    2260

GAT GAG AGG GAA GTA TCC GTT CCG GCG GAG ATC CTG CGG AAA TCC AAG        7169
Asp Glu Arg Glu Val Ser Val Pro Ala Glu Ile Leu Arg Lys Ser Lys
            2265                    2270                    2275

AAG TTC CCC GCA GCG ATG CCC ATC TGG GCG CGC CCG GAT TAC AAC CCT        7217
Lys Phe Pro Ala Ala Met Pro Ile Trp Ala Arg Pro Asp Tyr Asn Pro
2280                    2285                    2290                    2295

CCA CTG TTA GAG TCC TGG AAG GAC CCG GAC TAC GTC CCT CCG GTG GTG        7265
Pro Leu Leu Glu Ser Trp Lys Asp Pro Asp Tyr Val Pro Pro Val Val
            2300                    2305                    2310

CAC GGG TGC CCG TTG CCA CCT ATC AAG GCC CCT CCA ATA CCA CCT CCA        7313
His Gly Cys Pro Leu Pro Pro Ile Lys Ala Pro Pro Ile Pro Pro Pro
            2315                    2320                    2325
```

```
CGG AGA AAG AGG ACG GTT GTC CTA ACA GAG TCC TCC GTG TCT TCT GCC        7361
Arg Arg Lys Arg Thr Val Val Leu Thr Glu Ser Ser Val Ser Ser Ala
        2330            2335            2340

TTA GCG GAG CTC GCT ACT AAG ACC TTC GGC AGC TCC GAA TCA TCG GCC        7409
Leu Ala Glu Leu Ala Thr Lys Thr Phe Gly Ser Ser Glu Ser Ser Ala
        2345            2350            2355

GTC GAC AGC GGC ACG GCG ACC GCC CTT CCT GAC CAG GCC TCC GAC GAC        7457
Val Asp Ser Gly Thr Ala Thr Ala Leu Pro Asp Gln Ala Ser Asp Asp
2360            2365            2370            2375

GGT GAC AAA GGA TCC GAC GTT GAG TCG TAC TCC TCC ATG CCC CCC CTT        7505
Gly Asp Lys Gly Ser Asp Val Glu Ser Tyr Ser Ser Met Pro Pro Leu
            2380            2385            2390

GAG GGG GAA CCG GGG GAC CCC GAT CTC AGT GAC GGG TCT TGG TCT ACC        7553
Glu Gly Glu Pro Gly Asp Pro Asp Leu Ser Asp Gly Ser Trp Ser Thr
            2395            2400            2405

GTG AGC GAG GAA GCT AGT GAG GAT GTC GTC TGC TGC TCA ATG TCC TAC        7601
Val Ser Glu Glu Ala Ser Glu Asp Val Val Cys Cys Ser Met Ser Tyr
            2410            2415            2420

ACA TGG ACA GGC GCC TTG ATC ACG CCA TGC GCT GCG GAG GAA AGC AAG        7649
Thr Trp Thr Gly Ala Leu Ile Thr Pro Cys Ala Ala Glu Glu Ser Lys
        2425            2430            2435

CTG CCC ATC AAC GCG TTG AGC AAC TCT TTG CTG CGC CAC CAT AAC ATG        7697
Leu Pro Ile Asn Ala Leu Ser Asn Ser Leu Leu Arg His His Asn Met
2440            2445            2450            2455

GTT TAT GCC ACA ACA TCT CGC AGC GCA GGC CTG CGG CAG AAG AAG GTC        7745
Val Tyr Ala Thr Thr Ser Arg Ser Ala Gly Leu Arg Gln Lys Lys Val
            2460            2465            2470

ACC TTT GAC AGA CTG CAA GTC CTG GAC GAC CAC TAC CGG GAC GTG CTC        7793
Thr Phe Asp Arg Leu Gln Val Leu Asp Asp His Tyr Arg Asp Val Leu
            2475            2480            2485

AAG GAG ATG AAG GCG AAG GCG TCC ACA GTT AAG GCT AAA CTC CTA TCC        7841
Lys Glu Met Lys Ala Lys Ala Ser Thr Val Lys Ala Lys Leu Leu Ser
            2490            2495            2500

GTA GAG GAA GCC TGC AAG CTG ACG CCC CCA CAT TCG GCC AAA TCC AAG        7889
Val Glu Glu Ala Cys Lys Leu Thr Pro Pro His Ser Ala Lys Ser Lys
        2505            2510            2515

TTT GGC TAT GGG GCA AAG GAC GTC CGG AAC CTA TCC AGC AAG GCC GTT        7937
Phe Gly Tyr Gly Ala Lys Asp Val Arg Asn Leu Ser Ser Lys Ala Val
```

```
           2520              2525            2530                   2535

AAC CAC ATC CAC TCC GTG TGG AAG GAC TTG CTG GAA GAC ACT GTG ACA        7985
Asn His Ile His Ser Val Trp Lys Asp Leu Leu Glu Asp Thr Val Thr
              2540             2545             2550

CCA ATT GAC ACC ACC ATC ATG GCA AAA AAT GAG GTT TTC TGT GTC CAA        8033
Pro Ile Asp Thr Thr Ile Met Ala Lys Asn Glu Val Phe Cys Val Gln
          2555             2560             2565

CCA GAG AAA GGA GGC CGT AAG CCA GCC CGC CTT ATC GTA TTC CCA GAT        8081
Pro Glu Lys Gly Gly Arg Lys Pro Ala Arg Leu Ile Val Phe Pro Asp
          2570             2575             2580

CTG GGA GTC CGT GTA TGC GAG AAG ATG GCC CTC TAT GAT GTG GTC TCC        8129
Leu Gly Val Arg Val Cys Glu Lys Met Ala Leu Tyr Asp Val Val Ser
          2585             2590             2595

ACC CTT CCT CAG GTC GTG ATG GGC TCC TCA TAC GGA TTC CAG TAC TCT        8177
Thr Leu Pro Gln Val Val Met Gly Ser Ser Tyr Gly Phe Gln Tyr Ser
2600             2605             2610                   2615

CCT GGG CAG CGA GTC GAG TTC CTG GTG AAT ACC TGG AAA TCA AAG AAA        8225
Pro Gly Gln Arg Val Glu Phe Leu Val Asn Thr Trp Lys Ser Lys Lys
              2620             2625             2630

AAC CCC ATG GGC TTT TCA TAT GAC ACT CGC TGT TTC GAC TCA ACG GTC        8273
Asn Pro Met Gly Phe Ser Tyr Asp Thr Arg Cys Phe Asp Ser Thr Val
              2635             2640             2645

ACC GAG AAC GAC ATC CGT GTT GAG GAG TCA ATT TAC CAA TGT TGT GAC        8321
Thr Glu Asn Asp Ile Arg Val Glu Glu Ser Ile Tyr Gln Cys Cys Asp
          2650             2655             2660

TTG GCC CCC GAA GCC AGA CAG GCC ATA AAA TCG CTC ACA GAG CGG CTT        8369
Leu Ala Pro Glu Ala Arg Gln Ala Ile Lys Ser Leu Thr Glu Arg Leu
          2665             2670             2675

TAT ATC GGG GGT CCT CTG ACT AAT TCA AAA GGG CAG AAC TGC GGT TAT        8417
Tyr Ile Gly Gly Pro Leu Thr Asn Ser Lys Gly Gln Asn Cys Gly Tyr
2680             2685             2690                   2695

CGC CGG TGC CGC GCG AGC GGC GTG CTG ACG ACT AGC TGC GGT AAC ACC        8465
Arg Arg Cys Arg Ala Ser Gly Val Leu Thr Thr Ser Cys Gly Asn Thr
              2700             2705             2710

CTC ACA TGT TAC TTG AAG GCC TCT GCA GCC TGT CGA GCT GCG AAG CTC        8513
Leu Thr Cys Tyr Leu Lys Ala Ser Ala Ala Cys Arg Ala Ala Lys Leu
              2715             2720             2725
```

```
CAG GAC TGC ACG ATG CTC GTG AAC GGA GAC GAC CTC GTC GTT ATC TGT          8561
Gln Asp Cys Thr Met Leu Val Asn Gly Asp Asp Leu Val Val Ile Cys
        2730                2735                2740

GAA AGC GCG GGA ACC CAA GAG GAC GCG GCG AGC CTA CGA GTC TTC ACG          8609
Glu Ser Ala Gly Thr Gln Glu Asp Ala Ala Ser Leu Arg Val Phe Thr
        2745                2750                2755

GAG GCT ATG ACT AGG TAC TCC GCC CCC CCC GGG GAC CCG CCC CAA CCA          8657
Glu Ala Met Thr Arg Tyr Ser Ala Pro Pro Gly Asp Pro Pro Gln Pro
2760                2765                2770                2775

GAA TAC GAC TTG GAG CTG ATA ACA TCA TGT TCC TCC AAT GTG TCG GTC          8705
Glu Tyr Asp Leu Glu Leu Ile Thr Ser Cys Ser Ser Asn Val Ser Val
        2780                2785                2790

GCC CAC GAT GCA TCA GGC AAA AGG GTG TAC TAC CTC ACC CGT GAT CCC          8753
Ala His Asp Ala Ser Gly Lys Arg Val Tyr Tyr Leu Thr Arg Asp Pro
        2795                2800                2805

ACC ACC CCC CTA GCA CGG GCT GCG TGG GAG ACA GCT AGA CAC ACT CCA          8801
Thr Thr Pro Leu Ala Arg Ala Ala Trp Glu Thr Ala Arg His Thr Pro
        2810                2815                2820

GTT AAC TCC TGG CTA GGC AAC ATT ATT ATG TAT GCG CCC ACT TTG TGG          8849
Val Asn Ser Trp Leu Gly Asn Ile Ile Met Tyr Ala Pro Thr Leu Trp
        2825                2830                2835

GCA AGG ATG ATT CTG ATG ACT CAC TTC TTC TCC ATC CTT CTA GCG CAG          8897
Ala Arg Met Ile Leu Met Thr His Phe Phe Ser Ile Leu Leu Ala Gln
2840                2845                2850                2855

GAG CAA CTT GAA AAA GCC CTG GAC TGC CAG ATC TAC GGG GCC TGT TAC          8945
Glu Gln Leu Glu Lys Ala Leu Asp Cys Gln Ile Tyr Gly Ala Cys Tyr
        2860                2865                2870

TCC ATT GAG CCA CTT GAC CTA CCT CAG ATC ATT GAA CGA CTC CAT GGC          8993
Ser Ile Glu Pro Leu Asp Leu Pro Gln Ile Ile Glu Arg Leu His Gly
        2875                2880                2885

CTT AGC GCA TTT TCA CTC CAT AGT TAC TCT CCA GGT GAG ATC AAT AGG          9041
Leu Ser Ala Phe Ser Leu His Ser Tyr Ser Pro Gly Glu Ile Asn Arg
        2890                2895                2900

GTG GCT TCA TGC CTC AGG AAA CTT GGG GTA CCA CCC TTG CGA GTC TGG          9089
Val Ala Ser Cys Leu Arg Lys Leu Gly Val Pro Pro Leu Arg Val Trp
        2905                2910                2915

AGA CAT CGG GCC AGG AGC GTC CGC GCT AGG CTA CTG TCC CAG GGA GGG          9137
Arg His Arg Ala Arg Ser Val Arg Ala Arg Leu Leu Ser Gln Gly Gly
```

EP 0 463 848 A2

```
2920                 2925              2930                 2935

AGG GCC GCC ACT TGT GGC AAA TAC CTC TTC AAC TGG GCA GTA AAA ACC      9185
Arg Ala Ala Thr Cys Gly Lys Tyr Leu Phe Asn Trp Ala Val Lys Thr
            2940              2945              2950

AAA CTT AAA CTC ACT CCA ATC CCG GCT GCG TCC CGG CTG GAC TTG TCC      9233
Lys Leu Lys Leu Thr Pro Ile Pro Ala Ala Ser Arg Leu Asp Leu Ser
            2955              2960              2965

GGC TGG TTC GTT GCT GGT TAC AGC GGG GGA GAC ATA TAT CAC AGC CTG      9281
Gly Trp Phe Val Ala Gly Tyr Ser Gly Gly Asp Ile Tyr His Ser Leu
            2970              2975              2980

TCT CGT GCC CGA CCC CGT TGG TTC ATG CTG TGC CTA CTC CTA CTT TCT      9329
Ser Arg Ala Arg Pro Arg Trp Phe Met Leu Cys Leu Leu Leu Leu Ser
            2985              2990              2995

GTA GGG GTA GGC ATC TAC CTG CTC CCC AAC CGA TGAACGGGGA GATAAACACT    9382
Val Gly Val Gly Ile Tyr Leu Leu Pro Asn Arg
3000              3005              3010

CCAGGCCAAT AGGCCATCCC CCTTTTTTTT TTTT                                9416
```

(2) INFORMATION FOR SEQ ID NO:2:

    (i) SEQUENCE CHARACTERISTICS:
        (A) LENGTH: 3010 amino acids
        (B) TYPE: amino acid
        (D) TOPOLOGY: linear

    (ii) MOLECULE TYPE: protein

    (xi) SEQUENCE DESCRIPTION: SEQ ID NO:2:

```
Met Ser Thr Asn Pro Lys Pro Gln Arg Lys Thr Lys Arg Asn Thr Asn
 1               5              10              15

Arg Arg Pro Gln Asp Val Lys Phe Pro Gly Gly Gly Gln Ile Val Gly
            20              25              30

Gly Val Tyr Leu Leu Pro Arg Arg Gly Pro Arg Leu Gly Val Arg Ala
            35              40              45

Pro Arg Lys Thr Ser Glu Arg Ser Gln Pro Arg Gly Arg Arg Gln Pro
        50              55              60

Ile Pro Lys Ala Arg Arg Pro Glu Gly Arg Thr Trp Ala Gln Pro Gly
    65              70              75              80
```

53

```
Tyr Pro Trp Pro Leu Tyr Gly Asn Glu Gly Leu Gly Trp Ala Gly Trp
              85              90              95
                                                      ,

Leu Leu Ser Pro Arg Gly Ser Arg Pro Ser Trp Gly Pro Thr Asp Pro
          100             105             110

Arg Arg Arg Ser Arg Asn Leu Gly Lys Val Ile Asp Thr Leu Thr Cys
      115             120             125

Gly Phe Ala Asp Leu Met Gly Tyr Ile Pro Leu Val Gly Ala Pro Leu
    130             135             140

Gly Gly Ala Ala Arg Ala Leu Ala His Gly Val Arg Val Leu Glu Asp
145             150             155             160

Gly Val Asn Tyr Ala Thr Gly Asn Leu Pro Gly Cys Ser Phe Ser Ile
              165             170             175

Phe Leu Leu Ala Leu Leu Ser Cys Leu Thr Thr Pro Ala Ser Ala Tyr
          180             185             190

Glu Val His Asn Val Ser Gly Ile Tyr His Val Thr Asn Asp Cys Ser
      195             200             205

Asn Ala Ser Ile Val Tyr Glu Ala Ala Asp Leu Ile Met His Thr Pro
      210             215             220

Gly Cys Val Pro Cys Val Arg Glu Gly Asn Ser Ser Arg Cys Trp Val
225             230             235             240

Ala Leu Thr Pro Thr Leu Ala Ala Arg Asn Val Thr Ile Pro Thr Thr
              245             250             255

Thr Ile Arg Arg His Val Asp Leu Leu Val Gly Ala Ala Ala Phe Cys
          260             265             270

Ser Ala Met Tyr Val Gly Asp Leu Cys Gly Ser Val Phe Leu Val Ser
          275             280             285

Gln Leu Phe Thr Phe Ser Pro Arg Arg His Val Thr Leu Gln Asp Cys
      290             295             300

Asn Cys Ser Ile Tyr Pro Gly His Val Ser Gly His Arg Met Ala Trp
305             310             315             320

Asp Met Met Met Asn Trp Ser Pro Thr Thr Ala Leu Val Val Ser Gln
              325             330             335

Leu Leu Arg Ile Pro Gln Ala Val Val Asp Met Val Ala Gly Ala His
          340             345             350
```

```
Trp Gly Val Leu Ala Gly Leu Ala Tyr Tyr Ser Met Ala Gly Asn Trp
        355             360             365

Ala Lys Val Leu Ile Val Met Leu Leu Phe Ala Gly Val Asp Gly Asp
        370             375             380

Thr His Val Thr Gly Gly Ala Gln Ala Lys Thr Thr Asn Arg Leu Val
385             390             395             400

Ser Met Phe Ala Ser Gly Pro Ser Gln Lys Ile Gln Leu Ile Asn Thr
                405             410             415

Asn Gly Ser Trp His Ile Asn Arg Thr Ala Leu Asn Cys Asn Asp Ser
                420             425             430

Leu Gln Thr Gly Phe Leu Ala Ala Leu Phe Tyr Thr His Ser Phe Asn
        435             440             445

Ser Ser Gly Cys Pro Glu Arg Met Ala Gln Cys Arg Thr Ile Asp Lys
        450             455             460

Phe Asp Gln Gly Trp Gly Pro Ile Thr Tyr Ala Glu Ser Ser Arg Ser
465             470             475             480

Asp Gln Arg Pro Tyr Cys Trp His Tyr Pro Pro Pro Gln Cys Thr Ile
                485             490             495

Val Pro Ala Ser Glu Val Cys Gly Pro Val Tyr Cys Phe Thr Pro Ser
                500             505             510

Pro Val Val Val Gly Thr Thr Asp Arg Phe Gly Val Pro Thr Tyr Arg
        515             520             525

Trp Gly Glu Asn Glu Thr Asp Val Leu Leu Leu Asn Asn Thr Arg Pro
        530             535             540

Pro Gln Gly Asn Trp Phe Gly Cys Thr Trp Met Asn Ser Thr Gly Phe
545             550             555             560

Thr Lys Thr Cys Gly Gly Pro Pro Cys Asn Ile Gly Gly Val Gly Asn
                565             570             575

Asn Thr Leu Thr Cys Pro Thr Asp Cys Phe Arg Lys His Pro Glu Ala
                580             585             590

Thr Tyr Thr Lys Cys Gly Ser Gly Pro Trp Leu Thr Pro Arg Cys Met
        595             600             605

Val Asp Tyr Pro Tyr Arg Leu Trp His Tyr Pro Cys Thr Val Asn Phe
        610             615             620
```

55

```
Thr Ile Phe Lys Val Arg Met Tyr Val Gly Gly Val Glu His Arg Leu
625             630             635                 640

Asn Ala Ala Cys Asn Trp Thr Arg Gly Glu Arg Cys Asp Leu Glu Asp
            645             650                 655

Arg Asp Arg Pro Glu Leu Ser Pro Leu Leu Leu Ser Thr Thr Glu Trp
            660             665                 670

Gln Val Leu Pro Cys Ser Phe Thr Thr Leu Pro Ala Leu Ser Thr Gly
        675             680                 685

Leu Ile His Leu His Gln Asn Ile Val Asp Val Gln Tyr Leu Tyr Gly
        690             695                 700

Ile Gly Ser Ala Val Val Ser Phe Ala Ile Lys Trp Glu Tyr Val Leu
705             710             715                 720

Leu Leu Phe Leu Leu Leu Ala Asp Ala Arg Val Cys Ala Cys Leu Trp
            725             730                 735

Met Met Leu Leu Ile Ala Gln Ala Glu Ala Ala Leu Glu Asn Leu Val
            740             745                 750

Val Leu Asn Ser Ala Ser Val Ala Gly Ala His Gly Ile Leu Ser Phe
        755             760                 765

Leu Val Phe Phe Cys Ala Ala Trp Tyr Ile Lys Gly Arg Leu Val Pro
        770             775                 780

Gly Ala Thr Tyr Ala Leu Tyr Gly Val Trp Pro Leu Leu Leu Leu Leu
785             790             795                 800

Leu Ala Leu Pro Pro Arg Ala Tyr Ala Met Asp Arg Glu Met Ala Ala
            805             810                 815

Ser Cys Gly Gly Ala Val Phe Val Gly Leu Val Leu Leu Thr Leu Ser
            820             825                 830

Pro Tyr Tyr Lys Val Phe Leu Ala Arg Leu Ile Trp Trp Leu Gln Tyr
        835             840                 845

Phe Thr Thr Arg Ala Glu Ala Asp Leu His Val Trp Ile Pro Pro Leu
        850             855                 860

Asn Ala Arg Gly Gly Arg Asp Ala Ile Ile Leu Leu Met Cys Ala Val
865             870             875                 880

His Pro Glu Leu Ile Phe Asp Ile Thr Lys Leu Leu Ile Ala Ile Leu
            885             890                 895
```

```
Gly Pro Leu Met Val Leu Gln Ala Gly Ile Thr Arg Val Pro Tyr Phe
            900                 905                 910

Val Arg Ala Gln Gly Leu Ile His Ala Cys Met Leu Val Arg Lys Val
            915                 920                 925

Ala Gly Gly His Tyr Val Gln Met Ala Phe Met Lys Leu Gly Ala Leu
            930                 935                 940

Thr Gly Thr Tyr Ile Tyr Asn His Leu Thr Pro Leu Arg Asp Trp Pro
945                 950                 955                 960

Arg Ala Gly Leu Arg Asp Leu Ala Val Ala Val Glu Pro Val Val Phe
                965                 970                 975

Ser Asp Met Glu Thr Lys Ile Ile Thr Trp Gly Ala Asp Thr Ala Ala
            980                 985                 990

Cys Gly Asp Ile Ile Leu Gly Leu Pro Val Ser Ala Arg Arg Gly Lys
            995                 1000                1005

Glu Ile Leu Leu Gly Pro Ala Asp Ser Leu Glu Gly Arg Gly Leu Arg
        1010                1015                1020

Leu Leu Ala Pro Ile Thr Ala Tyr Ser Gln Gln Thr Arg Gly Leu Leu
1025                1030                1035                1040

Gly Cys Ile Ile Thr Ser Leu Thr Gly Arg Asp Lys Asn Gln Val Glu
                1045                1050                1055

Gly Glu Val Gln Val Val Ser Thr Ala Thr Gln Ser Phe Leu Ala Thr
            1060                1065                1070

Cys Val Asn Gly Val Cys Trp Thr Val Tyr His Gly Ala Gly Ser Lys
            1075                1080                1085

Thr Leu Ala Ala Pro Lys Gly Pro Ile Thr Gln Met Tyr Thr Asn Val
        1090                1095                1100

Asp Gln Asp Leu Val Gly Trp Pro Lys Pro Pro Gly Ala Arg Ser Leu
1105                1110                1115                1120

Thr Pro Cys Thr Cys Gly Ser Ser Asp Leu Tyr Leu Val Thr Arg His
                1125                1130                1135

Ala Asp Val Ile Pro Val Arg Arg Arg Gly Asp Ser Arg Gly Ser Leu
            1140                1145                1150

Leu Ser Pro Arg Pro Val Ser Tyr Leu Lys Gly Ser Ser Gly Gly Pro
        1155                1160                1165
```

```
Leu Leu Cys Pro Phe Gly His Ala Val Gly Ile Phe Arg Ala Ala Val
    1170              1175              1180

Cys Thr Arg Gly Val Ala Lys Ala Val Asp Phe Val Pro Val Glu Ser
1185              1190              1195              1200

Met Glu Thr Thr Met Arg Ser Pro Val Phe Thr Asp Asn Ser Ser Pro
              1205              1210              1215

Pro Ala Val Pro Gln Ser Phe Gln Val Ala His Leu His Ala Pro Thr
              1220              1225              1230

Gly Ser Gly Lys Ser Thr Lys Val Pro Ala Ala Tyr Ala Ala Gln Gly
          1235              1240              1245

Tyr Lys Val Leu Val Leu Asn Pro Ser Val Ala Ala Thr Leu Gly Phe
    1250              1255              1260

Gly Ala Tyr Met Ser Lys Ala His Gly Ile Asp Pro Asn Ile Arg Thr
1265              1270              1275              1280

Gly Val Arg Thr Ile Thr Thr Gly Ala Pro Val Thr Tyr Ser Thr Tyr
              1285              1290              1295

Gly Lys Phe Leu Ala Asp Gly Gly Cys Ser Gly Gly Ala Tyr Asp Ile
          1300              1305              1310

Ile Ile Cys Asp Glu Cys His Ser Thr Asp Ser Thr Thr Ile Leu Gly
          1315              1320              1325

Ile Gly Thr Val Leu Asp Gln Ala Glu Thr Ala Gly Ala Arg Leu Val
    1330              1335              1340

Val Leu Ala Thr Ala Thr Pro Pro Gly Ser Val Thr Val Pro His Pro
1345              1350              1355              1360

Asn Ile Glu Glu Val Ala Leu Ser Asn Thr Gly Glu Ile Pro Phe Tyr
              1365              1370              1375

Gly Lys Ala Ile Pro Ile Glu Ala Ile Arg Gly Gly Arg His Leu Ile
          1380              1385              1390

Phe Cys His Ser Lys Lys Lys Cys Asp Glu Leu Ala Ala Lys Leu Ser
    1395              1400              1405

Gly Leu Gly Ile Asn Ala Val Ala Tyr Tyr Arg Gly Leu Asp Val Ser
    1410              1415              1420

Val Ile Pro Thr Ile Gly Asp Val Val Val Val Ala Thr Asp Ala Leu
1425              1430              1435              1440
```

```
Met Thr Gly Tyr Thr Gly Asp Phe Asp Ser Val Ile Asp Cys Asn Thr
            1445                1450                1455

Cys Val Thr Gln Thr Val Asp Phe Ser Leu Asp Pro Thr Phe Thr Ile
            1460                1465                1470

Glu Thr Thr Thr Val Pro Gln Asp Ala Val Ser Arg Ser Gln Arg Arg
            1475                1480                1485

Gly Arg Thr Gly Arg Gly Arg Arg Gly Ile Tyr Arg Phe Val Thr Pro
            1490                1495                1500

Gly Glu Arg Pro Ser Gly Met Phe Asp Ser Ser Val Leu Cys Glu Cys
1505                1510                1515                1520

Tyr Asp Ala Gly Cys Ala Trp Tyr Glu Leu Thr Pro Ala Glu Thr Ser
                1525                1530                1535

Val Arg Leu Arg Ala Tyr Leu Asn Thr Pro Gly Leu Pro Val Cys Gln
            1540                1545                1550

Asp His Leu Glu Phe Trp Glu Ser Val Phe Thr Gly Leu Thr His Ile
            1555                1560                1565

Asp Ala His Phe Leu Ser Gln Thr Lys Gln Ala Gly Asp Asn Phe Pro
            1570                1575                1580

Tyr Leu Val Ala Tyr Gln Ala Thr Val Cys Ala Arg Ala Gln Ala Pro
1585                1590                1595                1600

Pro Pro Ser Trp Asp Gln Met Trp Lys Cys Leu Ile Arg Leu Lys Pro
                1605                1610                1615

Thr Leu His Gly Pro Thr Pro Leu Leu Tyr Arg Leu Gly Ala Val Gln
            1620                1625                1630

Asn Glu Val Thr Leu Thr His Pro Ile Thr Lys Tyr Ile Met Ala Cys
            1635                1640                1645

Met Ser Ala Asp Leu Glu Val Val Thr Ser Thr Trp Val Leu Val Gly
            1650                1655                1660

Gly Val Leu Ala Ala Leu Ala Ala Tyr Cys Leu Thr Thr Gly Ser Val
1665                1670                1675                1680

Val Ile Val Gly Arg Ile Ile Leu Ser Gly Arg Pro Ala Ile Val Pro
                1685                1690                1695

Asp Arg Glu Leu Leu Tyr Gln Glu Phe Asp Glu Met Glu Glu Cys Ala
            1700                1705                1710
```

```
Ser His Leu Pro Tyr Ile Glu Gln Gly Met Gln Leu Ala Glu Gln Phe
        1715            1720            1725

Lys Gln Lys Ala Leu Gly Leu Leu Gln Thr Ala Thr Lys Gln Ala Glu
        1730            1735            1740

Ala Ala Ala Pro Val Val Glu Ser Lys Trp Arg Ala Leu Glu Thr Phe
1745            1750            1755            1760

Trp Ala Lys His Met Trp Asn Phe Ile Ser Gly Ile Gln Tyr Leu Ala
            1765            1770            1775

Gly Leu Ser Thr Leu Pro Gly Asn Pro Ala Ile Ala Ser Leu Met Ala
            1780            1785            1790

Phe Thr Ala Ser Ile Thr Ser Pro Leu Thr Thr Gln Ser Thr Leu Leu
            1795            1800            1805

Phe Asn Ile Leu Gly Gly Trp Val Ala Ala Gln Leu Ala Pro Pro Ser
        1810            1815            1820

Ala Ala Ser Ala Phe Val Gly Ala Gly Ile Ala Gly Ala Ala Val Gly
1825            1830            1835            1840

Ser Ile Gly Leu Gly Lys Val Leu Val Asp Ile Leu Ala Gly Tyr Gly
            1845            1850            1855

Ala Gly Val Ala Gly Ala Leu Val Ala Phe Lys Val Met Ser Gly Glu
            1860            1865            1870

Met Pro Ser Thr Glu Asp Leu Val Asn Leu Leu Pro Ala Ile Leu Ser
        1875            1880            1885

Pro Gly Ala Leu Val Val Gly Val Val Cys Ala Ala Ile Leu Arg Arg
        1890            1895            1900

His Val Gly Pro Gly Glu Gly Ala Val Gln Trp Met Asn Arg Leu Ile
1905            1910            1915            1920

Ala Phe Ala Ser Arg Gly Asn His Val Ser Pro Thr His Tyr Val Pro
            1925            1930            1935

Glu Ser Asp Ala Ala Ala Arg Val Thr Gln Ile Leu Ser Ser Leu Thr
            1940            1945            1950

Ile Thr Gln Leu Leu Lys Arg Leu His Gln Trp Ile Asn Glu Asp Cys
        1955            1960            1965

Ser Thr Pro Cys Ser Gly Ser Trp Leu Arg Asp Val Trp Asp Trp Ile
        1970            1975            1980
```

```
Cys Thr Val Leu Thr Asp Phe Lys Thr Trp Leu Gln Ser Lys Leu Leu
1985                1990                1995                2000

Pro Gln Leu Pro Gly Val Pro Phe Phe Ser Cys Gln Arg Gly Tyr Lys
                2005                2010                2015

Gly Val Trp Arg Gly Asp Gly Ile Met Gln Thr Thr Cys Pro Cys Gly
                2020                2025                2030

Ala Gln Ile Thr Gly His Val Lys Asn Gly Ser Met Arg Ile Val Gly
                2035                2040                2045

Pro Lys Thr Cys Ser Asn Thr Trp His Gly Thr Phe Pro Ile Asn Ala
                2050                2055                2060

Tyr Thr Thr Gly Pro Cys Thr Pro Ser Pro Ala Pro Asn Tyr Ser Arg
2065                2070                2075                2080

Ala Leu Trp Arg Val Ala Ala Glu Glu Tyr Val Glu Val Thr Arg Val
                2085                2090                2095

Gly Asp Phe His Tyr Val Thr Gly Met Thr Thr Asp Asn Val Lys Cys
                2100                2105                2110

Pro Cys Gln Val Pro Ala Pro Glu Phe Phe Ser Glu Val Asp Gly Val
                2115                2120                2125

Arg Leu His Arg Tyr Ala Pro Ala Cys Arg Pro Leu Leu Arg Glu Glu
                2130                2135                2140

Val Thr Phe Gln Val Gly Leu Asn Gln Tyr Leu Val Gly Ser Gln Leu
2145                2150                2155                2160

Pro Cys Glu Pro Glu Pro Asp Val Ala Val Leu Thr Ser Met Leu Thr
                2165                2170                2175

Asp Pro Ser His Ile Thr Ala Glu Thr Ala Lys Arg Arg Leu Ala Arg
                2180                2185                2190

Gly Ser Pro Pro Ser Leu Ala Ser Ser Ser Ala Ser Gln Leu Ser Ala
                2195                2200                2205

Pro Ser Leu Lys Ala Thr Cys Thr Thr His His Val Ser Pro Asp Ala
                2210                2215                2220

Asp Leu Ile Glu Ala Asn Leu Leu Trp Arg Gln Glu Met Gly Gly Asn
2225                2230                2235                2240

Ile Thr Arg Val Glu Ser Glu Asn Lys Val Val Val Leu Asp Ser Phe
                2245                2250                2255
```

Asp Pro Leu Arg Ala Glu Glu Asp Glu Arg Glu Val Ser Val Pro Ala
          2260                2265                2270

Glu Ile Leu Arg Lys Ser Lys Lys Phe Pro Ala Ala Met Pro Ile Trp
          2275                2280                2285

Ala Arg Pro Asp Tyr Asn Pro Pro Leu Leu Glu Ser Trp Lys Asp Pro
          2290                2295                2300

Asp Tyr Val Pro Pro Val Val His Gly Cys Pro Leu Pro Pro Ile Lys
2305                2310                2315                2320

Ala Pro Pro Ile Pro Pro Pro Arg Arg Lys Arg Thr Val Val Leu Thr
               2325                2330                2335

Glu Ser Ser Val Ser Ser Ala Leu Ala Glu Leu Ala Thr Lys Thr Phe
          2340                2345                2350

Gly Ser Ser Glu Ser Ser Ala Val Asp Ser Gly Thr Ala Thr Ala Leu
          2355                2360                2365

Pro Asp Gln Ala Ser Asp Asp Gly Asp Lys Gly Ser Asp Val Glu Ser
          2370                2375                2380

Tyr Ser Ser Met Pro Pro Leu Glu Gly Glu Pro Gly Asp Pro Asp Leu
2385                2390                2395                2400

Ser Asp Gly Ser Trp Ser Thr Val Ser Glu Glu Ala Ser Glu Asp Val
               2405                2410                2415

Val Cys Cys Ser Met Ser Tyr Thr Trp Thr Gly Ala Leu Ile Thr Pro
          2420                2425                2430

Cys Ala Ala Glu Glu Ser Lys Leu Pro Ile Asn Ala Leu Ser Asn Ser
          2435                2440                2445

Leu Leu Arg His His Asn Met Val Tyr Ala Thr Thr Ser Arg Ser Ala
          2450                2455                2460

Gly Leu Arg Gln Lys Lys Val Thr Phe Asp Arg Leu Gln Val Leu Asp
2465                2470                2475                2480

Asp His Tyr Arg Asp Val Leu Lys Glu Met Lys Ala Lys Ala Ser Thr
               2485                2490                2495

Val Lys Ala Lys Leu Leu Ser Val Glu Glu Ala Cys Lys Leu Thr Pro
          2500                2505                2510

Pro His Ser Ala Lys Ser Lys Phe Gly Tyr Gly Ala Lys Asp Val Arg
          2515                2520                2525

,

```
Asn Leu Ser Ser Lys Ala Val Asn His Ile His Ser Val Trp Lys Asp
    2530                2535                2540

Leu Leu Glu Asp Thr Val Thr Pro Ile Asp Thr Thr Ile Met Ala Lys
2545                2550                2555                2560

Asn Glu Val Phe Cys Val Gln Pro Glu Lys Gly Gly Arg Lys Pro Ala
            2565                2570                2575

Arg Leu Ile Val Phe Pro Asp Leu Gly Val Arg Val Cys Glu Lys Met
            2580                2585                2590

Ala Leu Tyr Asp Val Val Ser Thr Leu Pro Gln Val Val Met Gly Ser
    2595                2600                2605

Ser Tyr Gly Phe Gln Tyr Ser Pro Gly Gln Arg Val Glu Phe Leu Val
    2610                2615                2620

Asn Thr Trp Lys Ser Lys Lys Asn Pro Met Gly Phe Ser Tyr Asp Thr
2625                2630                2635                2640

Arg Cys Phe Asp Ser Thr Val Thr Glu Asn Asp Ile Arg Val Glu Glu
            2645                2650                2655

Ser Ile Tyr Gln Cys Cys Asp Leu Ala Pro Glu Ala Arg Gln Ala Ile
            2660                2665                2670

Lys Ser Leu Thr Glu Arg Leu Tyr Ile Gly Gly Pro Leu Thr Asn Ser
    2675                2680                2685

Lys Gly Gln Asn Cys Gly Tyr Arg Arg Cys Arg Ala Ser Gly Val Leu
    2690                2695                2700

Thr Thr Ser Cys Gly Asn Thr Leu Thr Cys Tyr Leu Lys Ala Ser Ala
2705                2710                2715                2720

Ala Cys Arg Ala Ala Lys Leu Gln Asp Cys Thr Met Leu Val Asn Gly
            2725                2730                2735

Asp Asp Leu Val Val Ile Cys Glu Ser Ala Gly Thr Gln Glu Asp Ala
            2740                2745                2750

Ala Ser Leu Arg Val Phe Thr Glu Ala Met Thr Arg Tyr Ser Ala Pro
            2755                2760                2765

Pro Gly Asp Pro Pro Gln Pro Glu Tyr Asp Leu Glu Leu Ile Thr Ser
    2770                2775                2780

Cys Ser Ser Asn Val Ser Val Ala His Asp Ala Ser Gly Lys Arg Val
2785                2790                2795                2800
```

Tyr Tyr Leu Thr Arg Asp Pro Thr Thr Pro Leu Ala Arg Ala Ala Trp
2805                2810              2815

Glu Thr Ala Arg His Thr Pro Val Asn Ser Trp Leu Gly Asn Ile Ile
2820              2825              2830

Met Tyr Ala Pro Thr Leu Trp Ala Arg Met Ile Leu Met Thr His Phe
2835              2840              2845

Phe Ser Ile Leu Leu Ala Gln Glu Gln Leu Glu Lys Ala Leu Asp Cys
2850              2855              2860

Gln Ile Tyr Gly Ala Cys Tyr Ser Ile Glu Pro Leu Asp Leu Pro Gln
2865              2870              2875              2880

Ile Ile Glu Arg Leu His Gly Leu Ser Ala Phe Ser Leu His Ser Tyr
2885              2890              2895

Ser Pro Gly Glu Ile Asn Arg Val Ala Ser Cys Leu Arg Lys Leu Gly
2900              2905              2910

Val Pro Pro Leu Arg Val Trp Arg His Arg Ala Arg Ser Val Arg Ala
2915              2920              2925

Arg Leu Leu Ser Gln Gly Gly Arg Ala Ala Thr Cys Gly Lys Tyr Leu
2930              2935              2940

Phe Asn Trp Ala Val Lys Thr Lys Leu Lys Leu Thr Pro Ile Pro Ala
2945              2950              2955              2960

Ala Ser Arg Leu Asp Leu Ser Gly Trp Phe Val Ala Gly Tyr Ser Gly
2965              2970              2975

Gly Asp Ile Tyr His Ser Leu Ser Arg Ala Arg Pro Arg Trp Phe Met
2980              2985              2990

Leu Cys Leu Leu Leu Leu Ser Val Gly Val Gly Ile Tyr Leu Leu Pro
2995              3000              3005

Asn Arg
3010

64

**Claims**

1. An isolated non-A, non-B hepatitis virus particle comprising at least one antigen selected from the group consisting of a core antigen, a matrix antigen and an envelope antigen of the non-A, non-B hepatitis virus.

2. The non-A, non-B hepatitis virus particle according to claim 1, wherein said core antigen, matrix antigen and envelope antigen are, respectively, coded for by a nucleotide sequence of the 333rd to 677th nucleotides, a nucleotide sequence of the 678th to 905th nucleotides and a nucleotide sequence of the 906th to 1499th nucleotides of the non-A, non-B hepatitis virus entire nucleotide sequence from the 1st to 9416th nucleotides shown in Fig. 2(1) through Fig. 2 (16) hereof.

3. The non-A, non-B hepatitis virus particle according to claim 1 or 2, which has a ribonucleic acid corresponding to at least part of the nucleotide sequence shown in Fig. 2(1) through Fig. 2(16) hereof.

4. A method for producing an isolated non-A, non-B hepatitis virus particle, which comprises:
   (a) providing not more than ten different cDNA clones each comprising at least 1000 nucleotides and prepared from a non-A, non-B hepatitis virus genomic RNA fragment of at least 1000 nucleotides, said not more than ten different cDNA clones containing their respective cloned cDNA fragments which, on the whole, cover a region of at least the 333rd to 5177th nucleotides of the non-A, non-B hepatitis virus entire nucleotide sequence from the 1st to 9416th nucleotides shown in Fig. 2(1) through Fig. 2(16) hereof;
   (b) taking out said cDNA fragments from said cDNA clones by cutting so as to respectively have predetermined nucleotide sequences such that when the predetermined nucleotide sequences are arranged in sequence, the resultant nucleotide sequence has at least a region which coincides with the region of the 333rd to 5177th nucleotides;
   (c) ligating said taken-out cDNA fragments respectively having said predetermined nucleotide sequences in sequence to thereby construct a first deoxyribonucleic acid comprising a nucleotide sequence comprising at least the 333rd to 5177th nucleotides of the non-A, non-B hepatitis virus entire nucleotide sequence from the 1st to 9416th nucleotides shown in Fig. 2(1) through Fig. 2(16) hereof;
   (d) introducing at least one deoxyribonucleic acid selected from said first deoxyribonucleic acid and a second deoxyribonucleic acid obtained by substituting at least one nucleotide of the nucleotide sequence of said first deoxyribonucleic acid in accordance with the degeneracy of the genetic code into a replicable expression vector selected from a plasmid and an animal virus gene to obtain a replicable recombinant DNA comprising said plasmid and said at least one deoxyribonucleic acid introduced therein when said replicable expression vector is a plasmid or obtain a recombinant virus comprising said animal virus and said at least one deoxyribonucleic acid introduced therein when said replicable expression vector is an animal virus gene;
   (e) transfecting prokaryotic or eukaryotic cells with said recombinant DNA when said replicable expression vector used in step (d) is a plasmid, to thereby form a transformant, followed by selection of said transformant from parent cells of the prokaryotic or eukaryotic cell culture;
   (f) culturing said transformant obtained in step (e) in prokaryotic or eukaryotic cells to thereby produce a non-A, non-B hepatitis virus particle, or culturing said recombinant virus obtained in step (d,) in eukaryotic cells to thereby produce a non-A, non-B hepatitis virus particle together with an animal virus; and
   (g) isolating said non-A, non-B hepatitis virus particle.

5. The method according to claim 4, wherein said first deoxyribonucleic acid comprises a nucleotide sequence of the 333rd to 5918th nucleotides.

6. The method according to claim 4, wherein said first deoxyribonucleic acid comprises a nucleotide sequence of the 333rd to 6371st nucleotides.

7. The method according to claim 4, wherein said first deoxyribonucleic acid comprises a nucleotide sequence of the 333rd to 9362nd nucleotides.

8. The method according to claim 4, wherein said first deoxyribonucleic acid comprises a nucleotide sequence of the 1st to 9416th nucleotides.

9. A recombinant comprising a replicable expression vector selected from a plasmid and an animal virus gene and a deoxyribonucleic acid comprising at least one nucleotide sequence selected from the group consisting of a first nucleotide sequence of the 333rd to 5177th nucleotides of the non-A, non-B hepatitis virus entire nucleotide sequence from the 1st to 9416th nucleotides shown in Fig. 2(1) through Fig. 2(16) hereof and a second nucleotide sequence obtained by substituting at least one nucleotide of said first nucleotide sequence in accordance with the degeneracy of the genetic code.

10. The recombinant according to claim 9, wherein said first nucleotide sequence comprises a nucleotide sequence of the 333rd to 5918th nucleotides.

11. The recombinant according to claim 9, wherein said first nucleotide sequence comprises a nucleotide sequence of the 333rd to 6371st nucleotides.

12. The recombinant according to claim 9, wherein said first nucleotide sequence comprises a nucleotide sequence of the 333rd to 9362nd nucleotides.

13. The recombinant according to claim 9, wherein said first nucleotide sequence comprises a nucleotide sequence of the 1st to 9416th nucleotides.

14. A diagnostic agent for the detection of non-A, non-B hepatitis by an antigen-antibody reaction, comprising an effective amount, for the antigen-antibody reaction, of the non-A, non-B hepatitis virus particle according to claim 1 or 2.

15. A vaccine for non-A, non-B hepatitis, comprising an effective immunogenic amount of a non-A, non-B hepatitis virus particle according to claim 1 or 2, and at least one pharmaceutically acceptable carrier, diluent or excipient.

16. Escherichia coli strain BK102 carrying a non-A, non-B hepatitis virus genomic cDNA, deposited at Fermentation Research Institute, Japan under the accession number FERM BP-3384.

17. Escherichia coli strain BK106 carrying a non-A, non-B hepatitis virus genomic cDNA, deposited at Fermentation Research Institute, Japan under the accession number FERM BP-3385.

18. Escherichia coli strain BK112 carrying a non-A, non-B hepatitis virus genomic cDNA, deposited at Fermentation Research Institute, Japan under the accession number FERM BP-3386.

19. Escherichia coli strain BK146 carrying a non-A, non-B hepatitis virus genomic cDNA, deposited at Fermentation Research Institute, Japan under the accession number FERM BP-3387.

20. Escherichia coli strain BK147 carrying a non-A, non-B hepatitis virus genomic cDNA, deposited at Fermentation Research Institute, Japan under the accession number FERM BP-3388.

21. Escherichia coli strain BK157 carrying a non-A, non-B hepatitis virus genomic cDNA, deposited at Fermentation Research Institute, Japan under the accession number FERM BP-3243.

## FIG. 1(1)

## FIG. 1(2)

## FIG. 2(1)

EP 0 463 848 A2

```
  1  CGATTGGGGGCGACACTCCACCATAGATCACTCCCCTGTGAGGAACTACTGTCTTCACGCAGAAAGCGTCTAGCCATGGCGTTAGTATGAGTGTCGTGCAGCCTCCAGGACCCCCCCTCC
     GCTAACCCCCGCTGTGAGGTGGTATCTAGTGAGGGGACACTCCTTGATGACAGAAGTGCGTCTTTCGCAGATCGGTACCGCAATCATACTCACAGCACGTCGGAGGTCCTGGGGGGGAGG

121  CGGGAGAGCCATAGTGGTCTGCGGAACCGGTGAGTACACCGGAATTGCCAGGACGACCGGGTCCTTTCTTGGATCAACCCGCTCAATGCCTGGAGATTTGGGCGTGCCCCCGCGAGACTG
     GCCCTCTCGGTATCACCAGACGCCTTGGCCACTCATGTGGCCTTAACGGTCCTGCTGGCCCAGGAAAGAACCTAGTTGGGCGAGTTACGGACCTCTAAACCCGCACGGGGGCGCTCTGAC

                                                                                          MetSerThrAsnProLysProGlnArgLys
241  CTAGCCGAGTAGTGTTGGGTCGCGAAAGGCCTTGTGGTACTGCCTGATAGGGTGCTTGCGAGTGCCCCGGGAGGTCTCGTAGACCGTGCACCATGAGCACGAATCCTAAACCTCAAAGAA
     GATCGGCTCATCACAACCCAGCGCTTTCCGGAACACCATGACGGACTATCCCACGAACGCTCACGGGGCCCTCCAGAGCATCTGGCACGTGGTACTCGTGCTTAGGATTTGGAGTTTCTT

     ThrLysArgAsnThrAsnArgArgProGlnAspValLysPheProGlyGlyGlyGlnIleValGlyGlyValTyrLeuLeuProArgArgGlyProArgLeuGlyValArgAlaProArg
361  AAACCAAACGTAACACCAACCGCCGCCCACAGGACGTCAAGTTCCCGGGCGGTGGTCAGATCGTTGGTGGAGTTTACCTGTTGCCGCGCAGGGGCCCCAGGTTGGGTGTGCGCGCGCCCA
     TTTGGTTTGCATTGTGGTTGGCGGCGGGTGTCCTGCAGTTCAAGGGCCCGCCACCAGTCTAGCAACCACCTCAAATGGACAACGGCGCGTCCCCGGGGTCCAACCCACACGCGCGCGGGT

     LysThrSerGluArgSerGlnProArgGlyArgArgGlnProIleProLysAlaArgArgProGluGlyArgThrTrpAlaGlnProGlyTyrProTrpProLeuTyrGlyAsnGluGly
481  GGAAGACTTCCGAGCGGTCGCAACCTCGTGGAAGGCGACAACCTATCCCCAAGGCTCGCCGGCCCGAGGGCAGGACCTGGGCTCAGCCCGGGTACCCTTGGCCTCTCTATGGCAATGAGG
     CCTTCTGAAGGCTCGCCAGCGTTGGAGCACCTTCCGCTGTTGGATAGGGGTTCCGAGCGGCCGGGCTCCCGTCCTGGACCCGAGTCGGGCCCATGGGAACCGGAGAGATACCGTTACTCC
```

FIG. 2(2)

LeuGlyTrpAlaGlyTrpLeuLeuSerProArgGlySerArgProSerTrpGlyProThrAspProArgArgArgSerArgAsnLeuGlyLysValIleAspThrLeuThrCysGlyPhe
GCTTAGGGTGGGCAGGATGGCTCCTGTCACCCCGCGGCTCCCGGCCTAGTTGGGGCCCCACGGACCCCGGCGTAGGTCGCGTAATTTGGGTAAGGTCATCGATACCCTCACATGCGGCT
CGAATCCCACCCGTCCTACCGAGGACAGTGGGGCGCCGAGGGCCGGATCAACCCCGGGGTGCCTGGGGGCCGCATCCAGCGCATTAAACCCATTCCAGTAGCTATGGGAGTGTACGCCGA

AlaAspLeuMetGlyTyrIleProLeuValGlyAlaProLeuGlyGlyAlaAlaArgAlaLeuAlaHisGlyValArgValLeuGluAspGlyValAsnTyrAlaThrGlyAsnLeuPro
TCGCCGATCTCATGGGGTACATTCCGCTCGTCGGCGCCCCCCTGGGGGGCGCTGCCAGGGCCCTGGCACATGGTGTCCGGGTTCTGGAGGACGGCGTGAACTATGCAACAGGGAATCTGC
AGCGGCTAGAGTACCCCATGTAAGGCGAGCAGCCGCGGGGGGGACCCCCCGCGACGGTCCCGGGACCGTGTACCACAGGCCCAAGACCTCCTGCCGCACTTGATACGTTGTCCCTTAGACG

GlyCysSerPheSerIlePheLeuLeuAlaLeuLeuSerCysLeuThrThrProAlaSerAlaTyrGluValHisAsnValSerGlyIleTyrHisValThrAsnAspCysSerAsnAla
CCGGTTGCTCTTTTTCTATCTTCCTCTTGGCTCTGCTGTCCTGCCTGACCACCCCAGCTTCCGCTTACGAAGTGCACAACGTGTCCGGGATATATCATGTCACGAACGACTGCTCCAACG
GGCCAACGAGAAAAAGATAGAAGGAGAACCGAGACGACAGGACGGACTGGTGGGGTCGAAGGCGAATGCTTCACGTGTTGCACAGGCCCTATATAGTACAGTGCTTGCTGACGAGGTTGC

SerIleValTyrGluAlaAlaAspLeuIleMetHisThrProGlyCysValProCysValArgGluGlyAsnSerSerArgCysTrpValAlaLeuThrProThrLeuAlaAlaArgAsn
CAAGCATTGTGTATGAGGCAGCGGACTTGATCATGCATACTCCTGGGTGCGTGCCCTGCGTTCGGGAAGGCAACTCCTCCCGCTGCTGGGTAGCGCTCACTCCCACGCTCGCAGCCAGGA
GTTCGTAACACATACTCCGTCGCCTGAACTAGTACGTATGAGGACCCACGCACGGGACGCAAGCCCTTCCGTTGAGGAGGGCGACGACCCATCGCGAGTGAGGGTGCGAGCGTCGGTCCT

ValThrIleProThrThrThrIleArgArgHisValAspLeuLeuValGlyAlaAlaAlaPheCysSerAlaMetTyrValGlyAspLeuCysGlySerValPheLeuValSerGlnLeu
ACGTCACCATCCCCACCACGACGATACGACGCCACGTCGATCTGCTCGTTGGGGCGGCTGCTTTCTGTTCCGCTATGTACGTGGGGGACCTCTGCGGATCTGTTTTCCTCGTCTCTCAGC
TGCAGTGGTAGGGGTGGTGCTGCTATGCTGCGGTGCAGCTAGACGAGCAACCCCGCCGACGAAAGACAAGGCGATACATGCACCCCCTGGAGACGCCTAGACAAAAGGAGCAGAGAGTCG

EP 0 463 848 A2

**FIG. 2(3)**

PheThrPheSerProArgArgHisValThrLeuGlnAspCysAsnCysSerIleTyrProGlyHisValSerGlyHisArgMetAlaTrpAspMetMetMetAsnTrpSerProThrThr
TGTTCACCTTCTCGCCTCGCCGGCATGTGACATTACAGGACTGTAACTGCTCAATTTATCCCGGCCATGTGTCGGGTCACCGTATGGCTTGGGACATGATGATGAACTGGTCGCCCACAA
ACAAGTGGAAGAGCGGAGCGGCCGTACACTGTAATGTCCTGACATTGACGAGTTAAATAGGGCCGGTACACAGCCCAGTGGCATACCGAACCCTGTACTACTACTTGACCAGCGGGTGTT

AlaLeuValValSerGlnLeuLeuArgIleProGlnAlaValValAspMetValAlaGlyAlaHisTrpGlyValLeuAlaGlyLeuAlaTyrTyrSerMetAlaGlyAsnTrpAlaLys
CAGCCCTAGTGGTGTCGCAGTTACTCCGGATCCCACAAGCCGTCGTGGACATGGTGGCGGGGGCCCACTGGGGAGTCCTGGCGGGCCTTGCCTACTATTCCATGGCGGGGAACTGGGCTA
GTCGGGATCACCACAGCGTCAATGAGGCCTAGGGTGTTCGGCAGCACCTGTACCACCGCCCCCGGGTGACCCCTCAGGACCGCCCGGAACGGATGATAAGGTACCGCCCCTTGACCCGAT

ValLeuIleValMetLeuLeuPheAlaGlyValAspGlyAspThrHisValThrGlyGlyAlaGlnAlaLysThrThrAsnArgLeuValSerMetPheAlaSerGlyProSerGlnLys
AGGTTCTGATTGTGATGCTACTTTTTGCTGGCGTTGACGGGGATACCCACGTGACAGGGGGGGCGCAAGCCAAAACCACCAACAGGCTCGTGTCCATGTTCGCAAGTGGGCCGTCTCAGA
TCCAAGACTAACACTACGATGAAAAACGACCGCAACTGCCCCTATGGGTGCACTGTCCCCCCCGCGTTCGGTTTTGGTGGTTGTCCGAGCACAGGTACAAGCGTTCACCCGGCAGAGTCT

IleGlnLeuIleAsnThrAsnGlySerTrpHisIleAsnArgThrAlaLeuAsnCysAsnAspSerLeuGlnThrGlyPheLeuAlaAlaLeuPheTyrThrHisSerPheAsnSerSer
AAATCCAGCTTATAAACACCAATGGGAGTTGGCACATCAACAGGACTGCCCTGAACTGCAATGACTCTCTCCAGACTGGGTTTCTTGCCGCGCTGTTCTACACACATAGTTTCAACTCGT
TTTAGGTCGAATATTTGTGGTTACCCTCAACCGTGTAGTTGTCCTGACGGGACTTGACGTTACTGAGAGAGGTCTGACCCAAAGAACGGCGCGACAAGATGTGTGTATCAAAGTTGAGCA

GlyCysProGluArgMetAlaGlnCysArgThrIleAspLysPheAspGlnGlyTrpGlyProIleThrTyrAlaGluSerSerArgSerAspGlnArgProTyrCysTrpHisTyrPro
CCGGGTGCCCAGAGCGCATGGCCCAGTGCCGCACCATTGACAAGTTCGACCAGGGATGGGGTCCCATTACTTATGCTGAGTCTAGCAGATCAGACCAGAGGCCATATTGCTGGCACTACC
GGCCCACGGGTCTCGCGTACCGGGTCACGGCGTGGTAACTGTTCAAGCTGGTCCCTACCCCAGGGTAATGAATACGACTCAGATCGTCTAGTCTGGTCTCCGGTATAACGACCGTGATGG

FIG. 2(4)

ProProGlnCysThrIleValProAlaSerGluValCysGlyProValTyrCysPheThrProSerProValValValGlyThrThrAspArgPheGlyValProThrTyrArgTrpGly
CACCTCCACAATGTACCATCGTACCTGCGTCGGAGGTGTGCGGCCCAGTGTACTGCTTCACCCCAAGCCCTGTCGTCGTGGGGACGACCGATCGTTTCGGTGTCCCTACGTATAGATGGG
GTGGAGGTGTTACATGGTAGCATGGACGCAGCCTCCACACGCCGGGTCACATGACGAAGTGGGGTTCGGGACAGCAGCACCCCTGCTGGCTAGCAAAGCCACAGGGATGCATATCTACCC

GluAsnGluThrAspValLeuLeuLeuAsnAsnThrArgProProGlnGlyAsnTrpPheGlyCysThrTrpMetAsnSerThrGlyPheThrLysThrCysGlyGlyProProCysAsn
GGGAGAACGAGACTGACGTGCTGCTGCTCAACAACACGCGGCCGCCGCAAGGCAACTGGTTCGGCTGCACATGGATGAATAGCACCGGGTTCACCAAGACATGTGGGGGGCCCCCGTGTA
CCCTCTTGCTCTGACTGCACGACGACGAGTTGTTGTGCGCCGGCGGCGTTCCGTTGACCAAGCCGACGTGTACCTACTTATCGTGGCCCAAGTGGTTCTGTACACCCCCCGGGGGCACAT

IleGlyGlyValGlyAsnAsnThrLeuThrCysProThrAspCysPheArgLysHisProGluAlaThrTyrThrLysCysGlySerGlyProTrpLeuThrProArgCysMetValAsp
ACATCGGGGGGGTCGGCAACAACACCCTGACCTGCCCCACGGACTGCTTCCGGAAGCACCCCGAGGCTACCTACACAAAATGTGGTTCGGGGCCTTGGCTGACACCTAGGTGCATGGTTG
TGTAGCCCCCCCAGCCGTTGTTGTGGGACTGGACGGGGTGCCTGACGAAGGCCTTCGTGGGGCTCGATGGATGTGTTTTACACCAAGCCCCGGAACCGACTGTGGATCCACGTACCAAC

TyrProTyrArgLeuTrpHisTyrProCysThrValAsnPheThrIlePheLysValArgMetTyrValGlyGlyValGluHisArgLeuAsnAlaAlaCysAsnTrpThrArgGlyGlu
ACTATCCATACAGGCTCTGGCATTACCCCTGCACTGTTAACTTTACCATCTTCAAGGTTAGGATGTATGTGGGGGGGGTGGAGCACAGGCTCAATGCTGCATGCAATTGGACCCGAGGAG
TGATAGGTATGTCCGAGACCGTAATGGGGACGTGACAATTGAAATGGTAGAAGTTCCAATCCTACATACACCCCCCCCACCTCGTGTCCGAGTTACGACGTACGTTAACCTGGGCTCCTC

ArgCysAspLeuGluAspArgAspArgProGluLeuSerProLeuLeuLeuSerThrThrGluTrpGlnValLeuProCysSerPheThrThrLeuProAlaLeuSerThrGlyLeuIle
AGCGTTGTGACTTGGAGGACAGGGATAGGCCGGAGCTCAGCCCGCTGCTGCTGTCTACAACAGAGTGGCAGGTACTGCCCTGTTCCTTCACCACCCTACCAGCTCTGTCCACTGGCTTGA
TCGCAACACTGAACCTCCTGTCCCTATCCGGCCTCGAGTCGGGCGACGACGACAGATGTTGTCTCACCGTCCATGACGGGACAAGGAAGTGGTGGGATGGTCGAGACAGGTGACCGAACT

EP 0 463 848 A2

FIG. 2(5)

HisLeuHisGlnAsnIleValAspValGlnTyrLeuTyrGlyIleGlySerAlaValValSerPheAlaIleLysTrpGluTyrValLeuLeuLeuPheLeuLeuLeuAlaAspAlaArg
TTCACCTCCATCAGAACATCGTGGACGTGCAATACCTATACGGTATAGGGTCAGCGGTTGTCTCCTTTGCAATCAAATGGGAGTATGTCCTGTTGCTTTTCCTTCTCCTAGCGGACGCAC
AAGTGGAGGTAGTCTTGTAGCACCTGCACGTTATGGATATGCCATATCCCAGTCGCCAACAGAGGAAACGTTAGTTTACCCTCATACAGGACAACGAAAAGGAAGAGGATCGCCTGCGTG

ValCysAlaCysLeuTrpMetMetLeuLeuIleAlaGlnAlaGluAlaAlaLeuGluAsnLeuValValLeuAsnSerAlaSerValAlaGlyAlaHisGlyIleLeuSerPheLeuVal
GTGTCTGTGCCTGCTTGTGGATGATGCTGCTGATAGCCCAGGCCGAGGCCGCCTTGGAGAACCTGGTGGTCCTCAATTCGGCGTCTGTGGCCGGCGCACATGGCATCCTCTCCTTCCTTG
CACAGACACGGACGAACACCTACTACGACGACTATCGGGTCCGGCTCCGGCGGAACCTCTTGGACCACCAGGAGTTAAGCCGCAGACACCGGCCGCGTGTACCGTAGGAGAGGAAGGAAC

PhePheCysAlaAlaTrpTyrIleLysGlyArgLeuValProGlyAlaThrTyrAlaLeuTyrGlyValTrpProLeuLeuLeuLeuLeuLeuAlaLeuProProArgAlaTyrAlaMet
TGTTCTTCTGTGCCGCCTGGTACATCAAAGGCAGGCTGGTCCCTGGGGCGACATATGCTCTTTATGGCGTGTGGCCGCTGCTCCTGCTCTTGCTGGCATTACCACCGCGAGCTTACGCCA
ACAAGAAGACACGGCGGACCATGTAGTTTCCGTCCGACCAGGGACCCCGCTGTATACGAGAAATACCGCACACCGGCGACGAGGACGAGAACGACCGTAATGGTGGCGCTCGAATGCGGT

AspArgGluMetAlaAlaSerCysGlyGlyAlaValPheValGlyLeuValLeuLeuThrLeuSerProTyrTyrLysValPheLeuAlaArgLeuIleTrpTrpLeuGlnTyrPheThr
TGGACCGGGAGATGGCTGCATCGTGCGGAGGCGCGGTTTTTGTGGGTCTGGTACTCCTGACTTTGTCACCATACTACAAGGTGTTCCTCGCTAGGCTCATATGGTGGTTACAATATTTTA
ACCTGGCCCTCTACCGACGTAGCACGCCTCCGCGCCAAAAACACCCAGACCATGAGGACTGAAACAGTGGTATGATGTTCCACAAGGAGCGATCCGAGTATACCACCAATGTTATAAAAT

ThrArgAlaGluAlaAspLeuHisValTrpIleProProLeuAsnAlaArgGlyGlyArgAspAlaIleIleLeuLeuMetCysAlaValHisProGluLeuIlePheAspIleThrLys
CCACCAGAGCCGAGGCGGACTTACATGTGTGGATCCCCCCCCTCAACGCTCGGGGAGGCCGCGATGCCATCATCCTCCTCATGTGCGCAGTCCATCCAGAGCTAATCTTTGACATCACCA
GGTGGTCTCGGCTCCGCCTGAATGTACACACCTAGGGGGGGGGAGTTGCGAGCCCCTCCGGCGCTACGGTAGTAGGAGGAGTACACGCGTCAGGTAGGTCTCGATTAGAAACTGTAGTGGT

FIG. 2(6)

LeuLeuIleAlaIleLeuGlyProLeuMetValLeuGlnAlaGlyIleThrArgValProTyrPheValArgAlaGlnGlyLeuIleHisAlaCysMetLeuValArgLysValAlaGly
AACTTCTAATTGCCATACTCGGTCCGCTCATGGTGCTCCAAGCTGGCATAACCAGAGTGCCGTACTTCGTGCGCGCTCAAGGGCTCATTCATGCATGCATGTTAGTGCGGAAGGTCGCTG
TTGAAGATTAACGGTATGAGCCAGGCGAGTACCACGAGGTTCGACCGTATTGGTCTCACGGCATGAAGCACGCGCGAGTTCCCGAGTAAGTACGTACGTACAATCACGCCTTCCAGCGAC

GlyHisTyrValGlnMetAlaPheMetLysLeuGlyAlaLeuThrGlyThrTyrIleTyrAsnHisLeuThrProLeuArgAspTrpProArgAlaGlyLeuArgAspLeuAlaValAla
GGGGTCATTATGTCCAAATGGCCTTCATGAAGCTGGGCGCGCTGACAGGCACGTACATTTACAACCATCTTACCCCGCTACGGGATTGGCCACGCGCGGGGCCTACGAGACCTTGCGGTGG
CCCCAGTAATACAGGTTTACCGGAAGTACTTCGACCCGCGCGACTGTCCGTGCATGTAAATGTTGGTAGAATGGGGCGATGCCCTAACCGGTGCGCGCCCGGATGCTCTGGAACGCCACC

ValGluProValValPheSerAspMetGluThrLysIleIleThrTrpGlyAlaAspThrAlaAlaCysGlyAspIleIleLeuGlyLeuProValSerAlaArgArgGlyLysGluIle
CAGTGGAGCCCGTCGTCTTCTCCGACATGGAGACCAAGATCATCACCTGGGGAGCAGACACCGCGGCGTGTGGGGACATCATCTTGGGTCTGCCCGTCTCCGCCCGAAGGGGAAAGGAGA
GTCACCTCGGGCAGCAGAAGAGGCTGTACCTCTGGTTCTAGTAGTGGACCCCTCGTCTGTGGCGCCGCACACCCCTGTAGTAGAACCCAGACGGGCAGAGGCGGGCTTCCCCTTTCCTCT

LeuLeuGlyProAlaAspSerLeuGluGlyArgGlyLeuArgLeuLeuAlaProIleThrAlaTyrSerGlnGlnThrArgGlyLeuLeuGlyCysIleIleThrSerLeuThrGlyArg
TACTCCTGGGCCCGGCCGATAGTCTTGAAGGGCGGGGGGTTGCGACTCCTCGCGCCCATCACGGCCTACTCCCAACAGACGCGGGGCCTACTTGGTTGCATCATCACTAGCCTTACAGGCC
ATGAGGACCCGGGCCGGCTATCAGAACTTCCCGCCCCCAACGCTGAGGAGCGCGGGTAGTGCCGGATGAGGGTTGTCTGCGCCCCGGATGAACCAACGTAGTAGTGATCGGAATGTCCGG

AspLysAsnGlnValGluGlyGluValGlnValValSerThrAlaThrGlnSerPheLeuAlaThrCysValAsnGlyValCysTrpThrValTyrHisGlyAlaGlySerLysThrLeu
GGGACAAGAACCAGGTCGAGGGAGAGGTTCAGGTGGTTTCCACCGCAACACAATCCTTCCTGGCGACCTGCGTCAACGGCGTGTGTTGGACCGTTTACCATGGTGCTGGCTCAAAGACCT
CCCTGTTCTTGGTCCAGCTCCCTCTCCAAGTCCACCAAAGGTGGCGTTGTGTTAGGAAGGACCGCTGGACGCAGTTGCCGCACACAACCTGGCAAATGGTACCACGACCGAGTTTCTGGA

EP 0 463 848 A2

FIG. 2(7)

AlaAlaProLysGlyProIleThrGlnMetTyrThrAsnValAspGlnAspLeuValGlyTrpProLysProProGlyAlaArgSerLeuThrProCysThrCysGlySerSerAspLeu
TAGCCGCGCCAAAGGGGCCAATCACCCAGATGTACACTAATGTGGACCAGGACCTCGTCGGCTGGCCCAAGCCCCCCGGGGCGCGTTCCTTGACACCATGCACCTGTGGCAGCTCAGACC
ATCGGCGCGGTTTCCCCGGTTAGTGGGTCTACATGTGATTACACCTGGTCCTGGAGCAGCCGACCGGGTTCGGGGGGCCCCGCGCAAGGAACTGTGGTACGTGGACACCGTCGAGTCTGG

TyrLeuValThrArgHisAlaAspValIleProValArgArgArgGlyAspSerArgGlySerLeuLeuSerProArgProValSerTyrLeuLysGlySerSerGlyGlyProLeuLeu
TTTACTTGGTCACGAGACATGCTGACGTCATTCCCGGTGCGCCGGCGGGGCGACAGTAGGGGGAGCCTGCTCTCCCCCAGGCCTGTCTCCTACTTGAAGGGGCTCTTCGGGTGGTCCACTGC
AAATGAACCAGTGCTCTGTACGACTGCAGTAAGGCCACGCGGCCGCCCCGCTGTCATCCCCCTCGGACGAGAGGGGGTCCGGACAGAGGATGAACTTCCCGAGAAGCCCACCAGGTGACG

CysProPheGlyHisAlaValGlyIlePheArgAlaAlaValCysThrArgGlyValAlaLysAlaValAspPheValProValGluSerMetGluThrThrMetArgSerProValPhe
TCTGCCCCTTCGGGCACGCTGTGGGCATCTTCCGGGCTGCCGTATGCACCCGGGGGGGTTGCGAAGGCGGTGGACTTTGTGCCCGTAGAGTCCATGGAAACTACTATGCGGTCTCCGGTCT
AGACGGGGAAGCCCGTGCGACACCCGTAGAAGGCCCGACGGCATACGTGGGCCCCCCAACGCTTCCGCCACCTGAAACACGGGCATCTCAGGTACCTTTGATGATACGCCAGAGGCCAGA

ThrAspAsnSerSerProProAlaValProGlnSerPheGlnValAlaHisLeuHisAlaProThrGlySerGlyLysSerThrLysValProAlaAlaTyrAlaAlaGlnGlyTyrLys
TCACGGACAACTCATCCCCCCCGGCCGTACCGCAGTCATTTCAAGTGGCCCACCTACACGCTCCCACTGGCAGCGGCAAGAGTACTAAAGTGCCGGCTGCATATGCAGCCCAAGGGTACA
AGTGCCTGTTGAGTAGGGGGGGCCGGCATGGCGTCAGTAAAGTTCACCGGGTGGATGTGCGAGGGTGACCGTCGCCGTTCTCATGATTTCACGGCCGACGTATACGTCGGGTTCCCATGT

ValLeuValLeuAsnProSerValAlaAlaThrLeuGlyPheGlyAlaTyrMetSerLysAlaHisGlyIleAspProAsnIleArgThrGlyValArgThrIleThrThrGlyAlaPro
AGGTGCTCGTCCTCAATCCGTCCGTTGCCGCTACCTTAGGGTTTGGGGCGTATATGTCTAAGGCACACCGTATTGACCCCAACATCAGAACTGGGGTAAGGACCATTACCACAGGCGCCC
TCCACGAGCAGGAGTTAGGCAGGCAACGGCGATGGAATCCCAAACCCCGCATATACAGATTCCGTGTGCCATAACTGGGGTTGTAGTCTTGACCCCATTCCTGGTAATGGTGTCCGCGGG

EP 0 463 848 A2

FIG. 2(8)

ValThrTyrSerThrTyrGlyLysPheLeuAlaAspGlyGlyCysSerGlyGlyAlaTyrAspIleIleIleCysAspGluCysHisSerThrAspSerThrThrIleLeuGlyIleGly

CCGTCACATACTCTACCTATGGCAAGTTTCTTGCCGATGGTGGTTGCTCTGGGGGCGCTTATGACATCATAATATGTGATGAGTGCCATTCAACTGACTCGACTACAATCTTGGGCATCG

GGCAGTGTATGAGATGGATACCGTTCAAAGAACGGCTACCACCAACGAGACCCCCGCGAATACTGTAGTATTATACACTACTCACGGTAAGTTGACTGAGCTGATGTTAGAACCCGTAGC


ThrValLeuAspGlnAlaGluThrAlaGlyAlaArgLeuValValLeuAlaThrAlaThrProProGlySerValThrValProHisProAsnIleGluGluValAlaLeuSerAsnThr

GCACAGTCCTGGACCAAGCGGAGACGGCTGGAGCGCGGCTTGTCGTGCTCGCCACCGCTACGCCTCCGGGATCGGTCACCGTGCCACACCCAAACATCGAGGAGGTGGCCCTGTCTAATA

CGTGTCAGGACCTGGTTCGCCTCTGCCGACCTCGCGCCGAACAGCACGAGCGGTGGCGATGCGGAGGCCCTAGCCAGTGGCACGGTGTGGGTTTCTAGCTCCTCCACCGGGACAGATTAT


GlyGluIleProPheTyrGlyLysAlaIleProIleGluAlaIleArgGlyGlyArgHisLeuIlePheCysHisSerLysLysLysCysAspGluLeuAlaAlaLysLeuSerGlyLeu

CTGGAGAGATCCCCTTCTATGGCAAAGCCATCCCCATTGAAGCCATCAGGGGGGGGAAGGCATCTCATTTTCTGTCATTCCAAGAAGAAGTGCGACGAGCTCGCCGCAAAGCTGTCAGGCC

GACCTCTCTAGGGGAAGATACCGTTTCGGTAGGGGTAACTTCGGTAGTCCCCCCCTTCCGTAGAGTAAAAGACAGTAAGGTTCTTCTTCACGCTGCTCGAGCGGCGTTTCGACAGTCCGG


GlyIleAsnAlaValAlaTyrTyrArgGlyLeuAspValSerValIleProThrIleGlyAspValValValValAlaThrAspAlaLeuMetThrGlyTyrThrGlyAspPheAspSer

TCGGAATCAACGCTGTGGCGTATTACCGGGGGGCTCGATGTGTCCGTCATACCAACTATCGGAGACGTCGTTGTCGTGGCAACAGACGCTCTGATGACGGGCTATACGGGCGACTTTGACT

AGCCTTAGTTGCGACACCGCATAATGGCCCCCGAGCTACACAGGCAGTATGGTTGATAGCCTCTGCAGCAACAGCACCGTTGTCTGCGAGACTACTGCCCGATATGCCCGCTGAAACTGA


ValIleAspCysAsnThrCysValThrGlnThrValAspPheSerLeuAspProThrPheThrIleGluThrThrThrValProGlnAspAlaValSerArgSerGlnArgArgGlyArg

CAGTGATCGACTGTAACACATGTGTCACCCAGACAGTCGACTTCAGCTTGGATCCCACCTTCACCATTGAGACGACGACCGTGCCTCAAGACGCAGTGTCGCGCTCGCAGCGGCGGGGTA

GTCACTAGCTGACATTGTGTACACAGTGGGTCTGTCAGCTGAAGTCGAACCTAGGGTGGAAGTGGTAACTCTGCTGCTGGCACGGAGTTCTGCGTCACAGCGCGAGCGTCGCCGCCCCAT


EP 0 463 848 A2

FIG. 2(9)

ThrGlyArgGlyArgArgGlyIleTyrArgPheValThrProGlyGluArgProSerGlyMetPheAspSerSerValLeuCysGluCysTyrAspAlaGlyCysAlaTrpTyrGluLeu
GGACTGGCAGGGGTAGGAGAGGCATCTACAGGTTTGTGACTCCGGGAGAACGGCCCTCGGGCATGTTCGATTCCTCGGTCCTGTGTGAGTGCTATGACGCGGGCTGTGCTTGGTACGAGC
CCTGACCGTCCCCATCCTCTCCGTAGATGTCCAAACACTGAGGCCCTCTTGCCGGGAGCCCGTACAAGCTAAGGAGCCAGGACACACTCACGATACTGCGCCCGACACGAACCATGCTCG

ThrProAlaGluThrSerValArgLeuArgAlaTyrLeuAsnThrProGlyLeuProValCysGlnAspHisLeuGluPheTrpGluSerValPheThrGlyLeuThrHisIleAspAla
TCACCCCGGCCGAGACCTCGGTTAGGTTGCGGGCCTACCTGAACACACCAGGGTTGCCCGTTTGCCAGGACCACCTGGAGTTCTGGGAGAGTGTCTTCACAGGCCTCACCCATATAGATG
AGTGGGGCCGGCTCTGGAGCCAATCCAACGCCCGGATGGACTTGTGTGGTCCCAACGGGCAAACGGTCCTGGTGGACCTCAAGACCCTCTCACAGAAGTGTCCGGAGTGGGTATATCTAC

HisPheLeuSerGlnThrLysGlnAlaGlyAspAsnPheProTyrLeuValAlaTyrGlnAlaThrValCysAlaArgAlaGlnAlaProProProSerTrpAspGlnMetTrpLysCys
CACACTTCTTGTCCCAGACCAAGCAGGCAGGAGACAACTTCCCCTACCTGGTAGCATACCAAGCCACGGTGTGCGCCAGGGCTCAGGCCCCACCTCCATCATGGGATCAAATGTGGAAGT
GTGTGAAGAACAGGGTCTGGTTCGTCCGTCCTCTGTTGAAGGGGATGGACCATCGTATGGTTCGGTGCCACACGCGGTCCCGAGTCCGGGGTGGAGGTAGTACCCTAGTTTACACCTTCA

LeuIleArgLeuLysProThrLeuHisGlyProThrProLeuLeuTyrArgLeuGlyAlaValGlnAsnGluValThrLeuThrHisProIleThrLysTyrIleMetAlaCysMetSer
GTCTCATACGGCTGAAACCTACGCTGCACGGGCCAACACCCTTGCTGTACAGGCTGGGAGCCGTCCAGAATGAGGTCACCCTCACCCACCCCATAACCAAATACATCATGGCATGCATGT
CAGAGTATGCCGACTTTGGATGCGACGTGCCCGGTTGTGGGAACGACATGTCCGACCCTCGGCAGGTCTTACTCCAGTGGGAGTGGGTGGGGTATTGGTTTATGTAGTACCGTACGTACA

AlaAspLeuGluValValThrSerThrTrpValLeuValGlyGlyValLeuAlaAlaLeuAlaAlaTyrCysLeuThrThrGlySerValValIleValGlyArgIleIleLeuSerGly
CGGCTGACCTGGAGGTCGTCACTAGCACCTGGGTGCTGGTGGGCGGAGTCCTTGCAGCTCTGGCCGCGTATTGCCTGACAACAGGCAGTGTGGTCATTGTGGGTAGGATTATCTTGTCCG
GCCGACTGGACCTCCAGCAGTGATCGTGGACCCACGACCACCCGCCTCAGGAACGTCGAGACCGGCGCATAACGGACTGTTGTCCGTCACACCAGTAACACCCATCCTAATAGAACAGGC

EP 0 463 848 A2

FIG. 2(10)

ArgProAlaIleValProAspArgGluLeuLeuTyrGlnGluPheAspGluMetGluGluCysAlaSerHisLeuProTyrIleGluGlnGlyMetGlnLeuAlaGluGlnPheLysGln
GGAGGCCGGCCATTGTTCCCGACAGGGAGCTTCTCTACCAGGAGTTCGATGAAATGGAAGAGTGCGCCTCGCACCTCCCTTACATCGAGCAGGGAATGCAGCTCGCCGAGCAATTCAAGC
CCTCCGGCCGGTAACAAGGGCTGTCCCTCGAAGAGATGGTCCTCAAGCTACTTTACCTTCTCACGCGGAGCGTGGAGGGAATGTAGCTCGTCCCTTACGTCGAGCGGCTCGTTAAGTTCG

LysAlaLeuGlyLeuLeuGlnThrAlaThrLysGlnAlaGluAlaAlaAlaProValValGluSerLysTrpArgAlaLeuGluThrPheTrpAlaLysHisMetTrpAsnPheIleSer
AGAAAGCGCTCGGGTTACTGCAAACAGCCACCAAACAAGCGGAGGCTGCTGCTCCCGTGGTGGAGTCCAAGTGGCGAGCCCTTGAGACATTCTGGGCGAAGCACATGTGGAATTTCATCA
TCTTTCGCGAGCCCAATGACGTTTGTCGGTGGTTTGTTCGCCTCCGACGACGAGGGCACCACCTCAGGTTCACCGCTCGGGAACTCTGTAAGACCCGCTTCGTGTACACCTTAAAGTAGT

GlyIleGlnTyrLeuAlaGlyLeuSerThrLeuProGlyAsnProAlaIleAlaSerLeuMetAlaPheThrAlaSerIleThrSerProLeuThrThrGlnSerThrLeuLeuPheAsn
GCGGGATACAGTACTTAGCAGGCTTATCCACTCTGCCTGGGAACCCCGCAATAGCATCATTGATGGCATTCACAGCCTCTATCACCAGCCCGCTCACCACCCAAAGTACCCTCCTGTTTA
CGCCCTATGTCATGAATCGTCCGAATAGGTGAGACGGACCCTTGGGGCGTTATCGTAGTAACTACCGTAAGTGTCGGAGATAGTGGTCGGGCGAGTGGTGGGTTTCATGGGAGGACAAAT

IleLeuGlyGlyTrpValAlaAlaGlnLeuAlaProProSerAlaAlaSerAlaPheValGlyAlaGlyIleAlaGlyAlaAlaValGlySerIleGlyLeuGlyLysValLeuValAsp
ACATCTTGGGGGGGTGGGTGGCTGCCCAACTCGCCCCCCCCAGCGCCGCTTCGGCTTTCGTGGGCGCCGGCATCGCCGGTGCGGCTGTTGGCAGCATAGGCCTTGGGAAGGTGCTTGTGG
TGTAGAACCCCCCCACCCACCGACGGGTTGAGCGGGGGGGGGTCGCGGCGAAGCCGAAAGCACCCGCGGCCGTAGCGGCCACGCCGACAACCGTCGTATCCGGAACCCTTCCACGAACACC

IleLeuAlaGlyTyrGlyAlaGlyValAlaGlyAlaLeuValAlaPheLysValMetSerGlyGluMetProSerThrGluAspLeuValAsnLeuLeuProAlaIleLeuSerProGly
ACATTCTGGCGGGTTATGGAGCAGGAGTGGCCGGCGCGCTCGTGGCCTTTAAGGTCATGAGCGGCGAGATGCCCTCCACCGAGGACCTGGTCAATCTACTTCCTGCCATCCTCTCTCCTG
TGTAAGACCGCCCAATACCTCGTCCTCACCGGCCGCGCGAGCACCGGAAATTCCAGTACTCGCCGCTCTACGGGAGGTGGCTCCTGGACCAGTTAGATGAAGGACGGTAGGAGAGAGGAC

EP 0 463 848 A2

FIG. 2(11)

AlaLeuValValGlyValValCysAlaAlaIleLeuArgArgHisValGlyProGlyGluGlyAlaValGlnTrpMetAsnArgLeuIleAlaPheAlaSerArgGlyAsnHisValSer
GCGCCCTGGTCGTCGGGGTCGTGTGTGCAGCAATACTGCGTCGACACGTGGGTCCGGGAGAGGGGGCTGTGCAGTGGATGAACCGGCTGATAGCGTTCGCCTCGCGGGGTAATCATGTTT
CGCGGGACCAGCAGCCCCAGCACACACGTCGTTATGACGCAGCTGTGCACCCAGGCCCTCTCCCCGACACGTCACCTACTTGGCCGACTATCGCAAGCGGAGCGCCCCATTAGTACAAA

ProThrHisTyrValProGluSerAspAlaAlaAlaArgValThrGlnIleLeuSerSerLeuThrIleThrGlnLeuLeuLysArgLeuHisGlnTrpIleAsnGluAspCysSerThr
CCCCCACGCACTATGTGCCTGAGAGCGACGCCGCAGCGCGTGTTACTCAGATCCTCTCCAGCCTTACCATCACTCAGCTGCTGAAAAGGCTCCACCAGTGGATTAATGAAGACTGCTCCA
GGCGGGTGCGTGATACACGGACTCTCGCTGCGGCGTCGCGCACAATGAGTCTAGGAGAGGTCGGAATGGTAGTGAGTCGACGACTTTTCCGAGGTGGTCACCTAATTACTTCTGACGAGGT

ProCysSerGlySerTrpLeuArgAspValTrpAspTrpIleCysThrValLeuThrAspPheLysThrTrpLeuGlnSerLysLeuLeuProGlnLeuProGlyValProPhePheSer
CACCGTGTTCCGGCTCGTGGCTAAGGGATGTTTGGGACTGGATATGCACGGTGTTGACTGACTTCAAGACCTGGCTCCAGTCCAAGCTCCTGCCGCAGCTACCTGGAGTCCCTTTTTTCT
GTGGCACAAGGCCGAGCACCGATTCCCTACAAACCCTGACCTATACGTGCCACAACTGACTGAAGTTCTGGACCGAGGTCAGGTTCGAGGACGGCGTCGATGGACCTCAGGGAAAAAAGA

CysGlnArgGlyTyrLysGlyValTrpArgGlyAspGlyIleMetGlnThrThrCysProCysGlyAlaGlnIleThrGlyHisValLysAsnGlySerMetArgIleValGlyProLys
CGTGCCAACGCGGGTACAAGGGAGTCTGGCGGGGGAGACGGCATCATGCAAACCACCTGCCCATGTGGAGCACAGATCACCGGACATGTCAAAAACGGTTCCATGAGGATCGTCGGGCCTA
GCACGGTTGCGCCCATGTTCCCTCAGACCGCCCCTCTGCCGTAGTACGTTTGGTGGACGGGTACACCTCGTGTCTAGTGGCCTGTACAGTTTTTGCCAAGGTACTCCTAGCAGCCCGGAT

ThrCysSerAsnThrTrpHisGlyThrPheProIleAsnAlaTyrThrThrGlyProCysThrProSerProAlaProAsnTyrSerArgAlaLeuTrpArgValAlaAlaGluGluTyr
AGACCTGCAGCAACACGTGGCATGGAACATTCCCCATCAACGCATACACCACGGGCCCCTGCACACCCTCTCCAGCGCCAAACTATTCTAGGGCGCTGTGGCGGGTGGCCGCTGAGGAGT
TCTGGACGTCGTTGTGCACCGTACCTTGTAAGGGGTAGTTGCGTATGTGGTGCCCGGGGACGTGTGGGAGAGGTCGCGGTTTGATAAGATCCCGCGACACCGCCCACCGGCGACTCCTCA

FIG. 2(12)

ValGluValThrArgValGlyAspPheHisTyrValThrGlyMetThrThrAspAsnValLysCysProCysGlnValProAlaProGluPhePheSerGluValAspGlyValArgLeu
ACGTGGAGGTCACGCGGGTGGGGGATTTCCACTACGTGACGGGCATGACCACTGACAACGTAAAGTGCCCATGCCAGGTTCCGGCTCCTGAATTCTTCTCGGAGGTGGACGGAGTGCGGT
TGCACCTCCAGTGCGCCCACCCCCTAAAGGTGATGCACTGCCCGTACTGGTGACTGTTGCATTTCACGGGTACGGTCCAAGGCCGAGGACTTAAGAAGAGCCTCCACCTGCCTCACGCCA

HisArgTyrAlaProAlaCysArgProLeuLeuArgGluGluValThrPheGlnValGlyLeuAsnGlnTyrLeuValGlySerGlnLeuProCysGluProGluProAspValAlaVal
TGCACAGGTACGCTCCGGCGTGCAGGCCTCTCCTACGGGAGGAGGTTACATTCCAGGTCGGGCTCAACCAATACCTGGTTGGGTCACAGCTACCATGCGAGCCCGAACCGGATGTAGCAG
ACGTGTCCATGCGAGGCCGCACGTCCGGAGAGGATGCCCTCCTCCAATGTAAGGTCCAGCCCGAGTTGGTTATGGACCAACCCAGTGTCGATGGTACGCTCGGGCTTGGCCTACATCGTC

LeuThrSerMetLeuThrAspProSerHisIleThrAlaGluThrAlaLysArgArgLeuAlaArgGlySerProProSerLeuAlaSerSerSerAlaSerGlnLeuSerAlaProSer
TGCTCACTTCCATGCTCACCGACCCCTCCCACATCACAGCAGAAACGGCTAAGCGTAGGTTGGCCAGGGGGTCTCCCCCCTCCTTGGCCAGCTCTTCAGCTAGCCAGTTGTCTGCGCCTT
ACGAGTGAAGGTACGAGTGGCTGGGGAGGGTGTAGTGTCGTCTTTGCCGATTCGCATCCAACCGGTCCCCCAGAGGGGGGAGGAACCGGTCGAGAAGTCGATCGGTCAACAGACGCGGAA

LeuLysAlaThrCysThrThrHisHisValSerProAspAlaAspLeuIleGluAlaAsnLeuLeuTrpArgGlnGluMetGlyGlyAsnIleThrArgValGluSerGluAsnLysVal
CCTTGAAGGCGACATGCACTACCCACCATGTCTCTCCGGACGCTGACCTCATCGAGGCCAACCTCCTGTGGCGGCAGGAGATGGGCGGGAACATCACCCGCGTGGAGTCGGAGAACAAGG
GGAACTTCCGCTGTACGTGATGGGTGGTACAGAGAGGCCTGCGACTGGAGTAGCTCCGGTTGGAGGACACCGCCGTCCTCTACCCGCCCTTGTAGTGGGCGCACCTCAGCCTCTTGTTCC

ValValLeuAspSerPheAspProLeuArgAlaGluGluAspGluArgGluValSerValProAlaGluIleLeuArgLysSerLysLysPheProAlaAlaMetProIleTrpAlaArg
TGGTAGTCCTGGACTCTTTCGACCCGCTTCGAGCGGAGGAGGATGAGAGGGAAGTATCCGTTCCGGCGGAGATCCTGCGGAAATCCAAGAAGTTCCCCGCAGCGATGCCCATCTGGGCGC
ACCATCAGGACCTGAGAAAGCTGGGCGAAGCTCGCCTCCTCCTACTCTCCCTTCATAGGCAAGGCCGCCTCTAGGACGCCTTTAGGTTCTTCAAGGGGCGTCGCTACGGGTAGACCCGCG

EP 0 463 848 A2

FIG. 2(13)

ProAspTyrAsnProProLeuLeuGluSerTrpLysAspProAspTyrValProProValValHisGlyCysProLeuProProIleLysAlaProProIleProProProArgArgLys
GCCCGGATTACAACCCTCCACTGTTAGAGTCCTGGAAGGACCCGGACTACGTCCCTCCGGTGGTGCACGGGTGCCCGTTGCCACCTATCAAGGCCCCTCCAATACCACCTCCACGGAGAA
CGGGCCTAATGTTGGGAGGTGACAATCTCAGGACCTTCCTGGGCCTGATGCAGGGAGGCCACCACGTGCCCACGGGCAACGGTGGATAGTTCCGGGGAGGTTATGGTGGAGGTGCCTCTT

ArgThrValValLeuThrGluSerSerValSerSerAlaLeuAlaGluLeuAlaThrLysThrPheGlySerSerGluSerSerAlaValAspSerGlyThrAlaThrAlaLeuProAsp
AGAGGACGGTTGTCCTAACAGAGTCCTCCGTGTCTTCTGCCTTAGCGGAGCTCGCTACTAAGACCTTCGGCAGCTCCGAATCATCGGCCGTCGACAGCGGCACGGCGACCGCCCTTCCTG
TCTCCTGCCAACAGGATTGTCTCAGGAGGCACAGAAGACGGAATCGCCTCGAGCGATGATTCTGGAAGCCGTCGAGGCTTAGTAGCCGGCAGCTGTCGCCGTGCCGCTGGCGGGAAGGAC

GlnAlaSerAspAspGlyAspLysGlySerAspValGluSerTyrSerSerMetProProLeuGluGlyGluProGlyAspProAspLeuSerAspGlySerTrpSerThrValSerGlu
ACCAGGCCTCCGACGACGGTGACAAAGGATCCGACGTTGAGTCGTACTCCTCCATGCCCCCCCTTGAGGGGGAACCGGGGGACCCCGATCTCAGTGACGGGTCTTGGTCTACCGTGAGCG
TGGTCCGGAGGCTGCTGCCACTGTTTCCTAGGCTGCAACTCAGCATGAGGAGGTACGGGGGGGAACTCCCCCTTGGCCCCCTGGGCTAGAGTCACTGCCCAGAACCAGATGGCACTCGC

GluAlaSerGluAspValValCysCysSerMetSerTyrThrTrpThrGlyAlaLeuIleThrProCysAlaAlaGluGluSerLysLeuProIleAsnAlaLeuSerAsnSerLeuLeu
AGGAAGCTAGTGAGGATGTCGTCTGCTGCTCAATGTCCTACACATGGACAGGCGCCTTGATCACGCCATGCGCTGCGGAGGAAAGCAAGCTGCCCATCAACGCGTTGAGCAACTCTTTGC
TCCTTCGATCACTCCTACAGCAGACGACGAGTTACAGGATGTGTACCTGTCCGCGGAACTAGTGCGGTACGCGACGCCTCCTTTCGTTCGACGGGTAGTTGCGCAACTCGTTGAGAAACG

ArgHisHisAsnMetValTyrAlaThrThrSerArgSerAlaGlyLeuArgGlnLysLysValThrPheAspArgLeuGlnValLeuAspAspHisTyrArgAspValLeuLysGluMet
TGCGCCACCATAACATGGTTTATGCCACAACATCTCGCAGCGCAGGCCTGCGGCAGAAGAAGGTCACCTTTGACAGACTGCAAGTCCTGGACGACCACTACCGGGACGTGCTCAAGGAGA
ACGCGGTGGTATTGTACCAAATACGGTGTTGTAGAGCGTCGCGTCCGGACGCCGTCTTCTTCCAGTGGAAACTGTCTGACGTTCAGGACCTGCTGGTGATGGCCCTGCACGAGTTCCTCT

EP 0 463 848 A2

FIG. 2(14)

LysAlaLysAlaSerThrValLysAlaLysLeuLeuSerValGluGluAlaCysLysLeuThrProProHisSerAlaLysSerLysPheGlyTyrGlyAlaLysAspValArgAsnLeu
TGAAGGCGAAGGCGTCCACAGTTAAGGCTAAACTCCTATCCGTAGAGGAAGCCTGCAAGCTGACGCCCCCACATTCGGCCAAATCCAAGTTTGGCTATGGGGCAAAGGACGTCCGGAACC
ACTTCCGCTTCCGCAGGTGTCAATTCCGATTTGAGGATAGGCATCTCCTTCGGACGTTCGACTGCGGGGGTGTAAGCCGGTTTAGGTTCAAACCGATACCCCGTTTCCTGCAGGCCTTGG

SerSerLysAlaValAsnHisIleHisSerValTrpLysAspLeuLeuGluAspThrValThrProIleAspThrThrIleMetAlaLysAsnGluValPheCysValGlnProGluLys
TATCCAGCAAGGCCGTTAACCACATCCACTCCGTGTGGAAGGACTTGCTGGAAGACACTGTGACACCAATTGACACCACCATCATGGCAAAAAATGAGGTTTTCTGTGTCCAACCAGAGA
ATAGGTCGTTCCGGCAATTGGTGTAGGTGAGGCACACCTTCCTGAACGACCTTCTGTGACACTGTGGTTAACTGTGGTGGTAGTACCGTTTTTTACTCCAAAAGACACAGGTTGGTCTCT

GlyGlyArgLysProAlaArgLeuIleValPheProAspLeuGlyValArgValCysGluLysMetAlaLeuTyrAspValValSerThrLeuProGlnValValMetGlySerSerTyr
AAGGAGGCCGTAAGCCAGCCCGCCTTATCGTATTCCCAGATCTGGGAGTCCGTGTATGCGAGAAGATGGCCCTCTATGATGTGGTCTCCACCCTTCCTCAGGTCGTGATGGGCTCCTCAT
TTCCTCCGGCATTCGGTCGGGCGGAATAGCATAAGGGTCTAGACCCTCAGGCACATACGCTCTTCTACCGGGAGATACTACACCAGAGGTGGGAAGGAGTCCAGCACTACCCGAGGAGTA

GlyPheGlnTyrSerProGlyGlnArgValGluPheLeuValAsnThrTrpLysSerLysLysAsnProMetGlyPheSerTyrAspThrArgCysPheAspSerThrValThrGluAsn
ACGGATTCCAGTACTCTCCTGGGCAGCGAGTCGAGTTCCTGGTGAATACCTGGAAATCAAAGAAAAACCCCATGGGCTTTTCATATGACACTCGCTGTTTCGACTCAACGGTCACCGAGA
TGCCTAAGGTCATGAGAGGACCCGTCGCTCAGCTCAAGGACCACTTATGGACCTTTAGTTTCTTTTTGGGGTACCCGAAAAGTATACTGTGAGCGACAAAGCTGAGTTGCCAGTGGCTCT

AspIleArgValGluGluSerIleTyrGlnCysCysAspLeuAlaProGluAlaArgGlnAlaIleLysSerLeuThrGluArgLeuTyrIleGlyGlyProLeuThrAsnSerLysGly
ACGACATCCGTGTTGAGGAGTCAATTTACCAATGTTGTGACTTGGCCCCCGAAGCCAGACAGGCCATAAAATCGCTCACAGAGCGGCTTTATATCGGGGGGTCCTCTGACTAATTCAAAAG
TGCTGTAGGCACAACTCCTCAGTTAAATGGTTACAACACTGAACCGGGGGCTTCGGTCTGTCGGTATTTTAGCGAGTGTCTCGCCGAAATATAGCCCCCAGGAGACTGATTAAGTTTTC

FIG. 2(15)

GlnAsnCysGlyTyrArgArgCysArgAlaSerGlyValLeuThrThrSerCysGlyAsnThrLeuThrCysTyrLeuLysAlaSerAlaAlaCysArgAlaAlaLysLeuGlnAspCys
GGCAGAACTGCCGGTTATCGCCGGTGCCGCGCGAGCGGCGTGCTGACGACTAGCTGCGGTAACACCCTCACATGTTACTTGAAGGCCTCTGCAGCCTGTCGAGCTGCGAAGCTCCAGGACT
CCGTCTTGACGCCAATAGCGGCCACGGCGCGCTCGCCGCACGACTGCTGATCGACGCCATTGTGGGAGTGTACAATGAACTTCCGGAGACGTCGGACAGCTCGACGCTTCGAGGTCCTGA

ThrMetLeuValAsnGlyAspAspLeuValValIleCysGluSerAlaGlyThrGlnGluAspAlaAlaSerLeuArgValPheThrGluAlaMetThrArgTyrSerAlaProProGly
GCACGATGCTCGTGAACGGAGACGACCTCGTCGTTATCTGTGAAAGCGCGGGAACCCAAGAGGACCCGGCGAGCCTACGAGTCTTCACGGAGGCTATGACTAGGTACTCCGCCCCCCCG
CGTGCTACGAGCACTTGCCTCTGCTGGAGCAGCAATAGACACTTTCGCGCCCTTGGGTTCTCCTGCGCCGCTCGGATGCTCAGAAGTGCCTCCGATACTGATCCATGAGGCGGGGGGGGC

AspProProGlnProGluTyrAspLeuGluLeuIleThrSerCysSerSerAsnValSerValAlaHisAspAlaSerGlyLysArgValTyrTyrLeuThrArgAspProThrThrPro
GGGACCCGCCCCAACCAGAATACGACTTGGAGCTGATAACATCATGTTCCTCCAATGTGTCGGTCGCCCACGATGCATCAGGCAAAAGGGTGTACTACCTCACCCGTGATCCCACCACCC
CCCTGGGCGGGGTTGGTCTTATGCTGAACCTCGACTATTGTAGTACAAGGAGGTTACACAGCCAGCGGGTGCTACGTAGTCCGTTTTCCCACATGATGGAGTGGGCACTAGGGTGGTGGG

LeuAlaArgAlaAlaTrpGluThrAlaArgHisThrProValAsnSerTrpLeuGlyAsnIleIleMetTyrAlaProThrLeuTrpAlaArgMetIleLeuMetThrHisPhePheSer
CCCTAGCACGGGCTGCGTGGGAGACAGCTAGACACACTCCAGTTAACTCCTGGCTAGGCAACATTATTATGTATGCGCCCACTTTGTGGGCAAGGATGATTCTGATGACTCACTTCTTCT
GGGATCGTGCCCGACGCACCCTCTGTCGATCTGTGTGAGGTCAATTGAGGACCGATCCGTTGTAATAATACATACGCGGGTGAAACACCCGTTCCTACTAAGACTACTGAGTGAAGAAGA

IleLeuLeuAlaGlnGluGlnLeuGluLysAlaLeuAspCysGlnIleTyrGlyAlaCysTyrSerIleGluProLeuAspLeuProGlnIleIleGluArgLeuHisGlyLeuSerAla
CCATCCTTCTAGCGCAGGAGCAACTTGAAAAAGCCCTGGACTGCCAGATCTACGGGGCCTGTTACTCCATTGAGCCACTTGACCTACCTCAGATCATTGAACGACTCCATGGCCTTAGCG
GGTAGGAAGATCGCGTCCTCGTTGAACTTTTTCGGGACCTGACGGTCTAGATGCCCCGGACAATGAGGTAACTCGGTGAACTGGATGGAGTCTAGTAACTTGCTGAGGTACCGGAATCGC

EP 0 463 848 A2

## FIG. 2(16)

PheSerLeuHisSerTyrSerProGlyGluIleAsnArgValAlaSerCysLeuArgLysLeuGlyValProProLeuArgValTrpArgHisArgAlaArgSerValArgAlaArgLeu
CATTTTCACTCCATAGTTACTCTCCAGGTGAGATCAATAGGGTGGCTTCATGCCTCAGGAAACTTGGGGTACCACCCTTGCGAGTCTGGAGACATCGGGCCAGGAGCGTCCGCGCTAGGC
GTAAAAGTGAGGTATCAATGAGAGGTCCACTCTAGTTATCCCACCGAAGTACGGAGTCCTTTGAACCCCATGGTGGGAACGCTCAGACCTCTGTAGCCCGGTCCTCGCAGGCGCGATCCG

LeuSerGlnGlyGlyArgAlaAlaThrCysGlyLysTyrLeuPheAsnTrpAlaValLysThrLysLeuLysLeuThrProIleProAlaAlaSerArgLeuAspLeuSerGlyTrpPhe
TACTGTCCCAGGGAGGGAGGGCCGCCACTTGTGGCAAATACCTCTTCAACTGGGCAGTAAAAACCAAACTTAAACTCACTCCAATCCCGGCTGCGTCCCGGCTGGACTTGTCCGGCTGGT
ATGACAGGGTCCCTCCCTCCCGGCGGTGAACACCGTTTATGGAGAAGTTGACCCGTCATTTTTGGTTTGAATTTGAGTGAGGTTAGGGCCGACGCAGGGCCGACCTGAACAGGCCGACCA

ValAlaGlyTyrSerGlyGlyAspAleTyrHisSerLeuSerArgAlaArgProArgTrpPheMetLeuCysLeuLeuLeuLeuSerValGlyValGlyIleTyrLeuLeuProAsnArg
TCGTTGCTGGTTACAGCGGGGGAGACATATATCACAGCCTGTCTCGTCCCCGACCCCGTTGGTTCATGCTGTGCCTACTCCTACTTTCTGTAGGGGTAGGCATCTACCTGCTCCCCAACC
AGCAACGACCAATGTCGCCCCCTCTGTATATAGTGTCGGACAGAGCACGGGCTGGGGCAACCAAGTACGACACGGATGAGGATGAAAGACATCCCCATCCGTAGATGGACGAGGGGTTGG

GATGAACGGGGAGATAAACACTCCAGGCCAATAGGCCATCCCCCTTTTTTTTTTTTT
CTACTTGCCCCTCTATTTGTGAGGTCCGGTTATCCGGTAGGGGGAAAAAAAAAAAAA

FIG. 3

# FIG. 4

# FIG. 5

# FIG. 6

# FIG. 7

FIG. 8